# EUROPEAN PATENT APPLICATION

(11) **EP 4 733 306 A1**
(43) Date of publication of application: **29.04.2026**
(21) Application number: 24832425.3
(22) Date of filing: 26.06.2024
(51) Int. Cl.: C07H 21/02, A61K 39/00, C12N 15/113

(54) **NOVEL MODIFIED TRINUCLEOTIDE CAPPING DERIVATIVE, AND MRNA INTO WHICH CAPPING DERIVATIVE IS INTRODUCED**

(30) Priority: 26.06.2023 KR 20230081577; 14.09.2023 KR 20230122297; 18.09.2023 KR 20230123789
(71) Applicant: Elonova Co., Ltd., Jeonju-si, Jeonbuk-do 54896 (KR)
(72) Inventor: SEO, Young Jun, Jeonju-si, Jeonbuk-do 54969 (KR); SALEH, Salahi, Jeonju-si, Jeonbuk-do 54896 (KR); YUN, Hyeonsu, Jeonju-si, Jeonbuk-do 54896 (KR); KAZI, Morshed Alom, Jeonju-si, Jeonbuk-do 54896 (KR); THOKCHOM, Simander Singh, Jeonju-si, Jeonbuk-do 54896 (KR); CHABUNGBAM, Dhurbachandra Singh, Jeonju-si, Jeonbuk-do 54896 (KR); MIRAY, Cebeci, Jeonju-si, Jeonbuk-do 54896 (KR); APURBA, Gouri, Jeonju-si, Jeonbuk-do 54896 (KR)
(74) Representative: M. Zardi & Co S.A.
(86) International application number: PCT/KR2024/008876
(87) International publication number: WO 2025/005647

(57) **Abstract**

The present invention relates to a novel modified trinucleotide capping derivative, and mRNA into which the capping derivative is introduced. The derivatives of the present invention in which a methylene bridge heterocyclic compound and arylmethyl are individually introduced into the N7-position of the 5'- terminal guanosine base have higher efficiency than capping materials used in existing mRNA vaccines, and thus can be efficiently used in the development of mRNA vaccines and the development of various mRNA-based therapeutic agents.

## Description

The present invention relates to novel modified trinucleotide capping derivatives and mRNA
introduced with the capping derivatives, and more specifically, to mRNA constructs introduced with trinucleotide-based capping substances in which the N7 position of a 5'-terminal guanosine base is modified with a methylene-bridged heterocyclic compound or
arylmethyl compound instead of a conventional methyl group.

### BACKGROUND ART

mRNA vaccines have played a key role in treating the COVID-19 pandemic that has spread worldwide. Capping substances serve to protect mRNA vaccines from degradation. Specifically, in order to express a specific protein that is functionally important based on the DNA genetic information, a transcription process is required during which an mRNA having a complementary sequence is synthesized based on the DNA template strand in the nucleus. The synthesized mRNA is transported out of the nucleus and undergoes a translation process in the cytoplasm through ribosomes to synthesize a specific protein.

In particular, N7-methylation in the CAP structure refers to the addition of a methyl group to the seventh nitrogen atom of the guanosine at the 5' terminus of the mRNA, and this methylation performs several important functions that significantly enhance the biological effects of the mRNA. The N7-methylated 5' cap structure improves the stability of the mRNA molecule, promotes binding with ribosomes, and facilitates initiation of translation by promoting binding with translation initiation factors eIF4E and eIF4G. Furthermore, the N7-methylated cap structure plays an important role in efficient transport of mRNA from the nucleus to the cytoplasm and functions as an intracellular marker of mRNA to avoid recognition and degradation by the innate immune system. Overall, the N7-methylated 5' cap is an essential feature for the stability and function of mRNA in eukaryotic cells, affecting the life cycle of RNA and the ability to efficiently produce proteins.

Two methods have been used for the capping process. The first is a post-transcriptional 5' capping method involving processing of the 5' terminus of the RNA product after DNA transcription. After transcription, the 5' terminus of the RNA transcript contains a free triphosphate group because it is the first nucleotide incorporated into the chain. The capping process replaces this triphosphate group with another structure called a "cap." The cap is added by the guanylyl transferase enzyme, which catalyzes the reaction between the 5' terminus of the RNA transcript and a guanine triphosphate (GTP) molecule. The second capping process used in mRNA vaccines is performed by in vitro transcription (IVT), which enables capping simultaneously with transcription of mRNA. In this method, the cap is added during mRNA synthesis rather than as a separate step after transcription. This approach can simplify the capping process, improve capping efficiency, and enable simple processing and high-yield mRNA production. The 5' capping structure, which serves as the translation initiation factor for protein expression, plays an important role by forming a translation initiation complex through eukaryotic translation initiation factor 4E (eIF4E). In addition, the cap structure functions as an intracellular marker of mRNA to avoid recognition and degradation by the innate immune system.

For about 20 years, dinucleotide-based ARCA cap compounds have been used as chemical capping substances through IVT; however, after the COVID-19 outbreak, a trinucleotide-based CleanCap, which exhibits a much higher antibody expression efficiency, has been used as the main component of the Pfizer mRNA vaccine.

CleanCap, developed by TriLink, has a chemically modified trinucleotide structure and has been reported to be the most efficient compound to date for expression of antigen proteins in mRNA vaccines. TriLink has secured exclusive patent rights to this capping substance.

However, in Korea, a technology capable of improving antigen protein expression efficiency in mRNA vaccines, such as CleanCap, has not yet been developed, and thus domestic acquisition of mRNA vaccine technology has not been achieved. Therefore, the inventors of the present invention synthesized mRNA by employing a capping technology that enables capping to occur simultaneously with transcription through IVT, due to its superior efficiency.

### DETAILED DESCRIPTION OF THE INVENTION

### TECHNICAL PROBLEM

Accordingly, the inventors of the present invention have completed the present invention by developing novel capping substances substituted with a new N7-methylene-bridged heterocyclic compound and capping substances substituted with a new N7-arylmethyl (benzyl (BzCapl)) and derivatives thereof, which are different from CleanCap-modified nucleotides, and which can increase antigen protein expression efficiency of mRNA vaccines, similar to CleanCap.

### TECHNICAL SOLUTION

In order to solve the above problem, the present invention provides a trinucleotide cap analog comprising a compound of Chemical Formula 1:

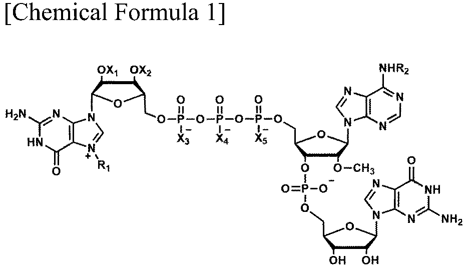

In the compound of Chemical Formula 1, R₁ is a methylene-bridged heterocyclic compound linked via a methylene bridge to the N7 position of a guanosine base,
X₁ and X₂ are each H or CH₃, X₃, X₄, and X₅ are each selected from the group consisting of O, S, and Se,
R₂ is a derivative comprising benzyl,
the heterocyclic compound is a substituent selected from heteroaromatic ring compounds having 1 to 2 heteroatoms selected from O, N, and S, and being a saturated or partially saturated 5- to 10-membered heteroaromatic ring compound, and
the heteroaromatic ring compound is a substituent selected from the group consisting of furan, pyrole, thiophene, imidazole, pyrazole, oxazole, isoxazole, thiazole, pyridine, pyrazine, primidine, pyridazine, and triazine.

The heterocyclic compound may be a substituent selected from aromatic ring groups having 1 to 2 heteroatoms selected from O, N, and S and being saturated or partially saturated 5- to 10-membered rings. Specific examples of the heterocyclic compound may include thiophene, pyridyl, imidazole, tetrahydrofuran-yl, 2,3-dihydrofuran-yl, 2,5-dihydrofuran-yl, pyrrolidin-yl, 2,3-dihydropyrrolidin-yl, 2,5-dihydropyrrolidin-yl, tetrahydro-2H-pyranyl, 3,4-dihydro-2H-pyranyl, 4H-pyranyl, piperidin-yl, 1,2,3,4-tetrahydropyridin-yl, 1,4-dihydropyridin-yl, piperazin-yl, N-protected piperazinyl, and morpholino. The N-protecting group of piperazinyl may typically include alkyl, alkylcarbonyl, or alkylsulfonyl groups.

In the present invention, the trinucleotide cap analog may be the compound of Chemical Formula 2 or Chemical Formula 3:

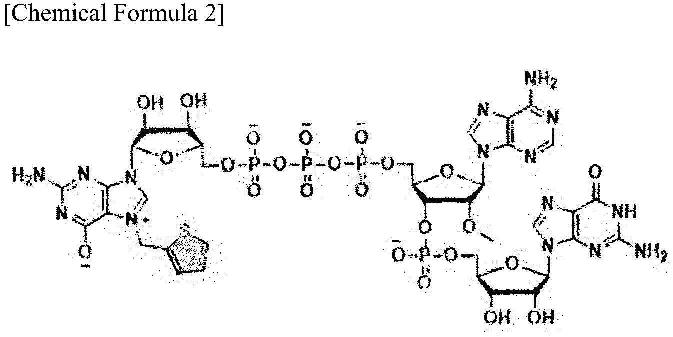

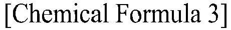

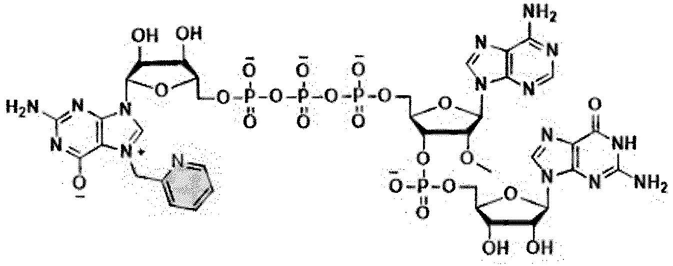

A trinucleotide cap analog comprising a compound of Chemical Formula [4-1], [4-2], or [4-3]:

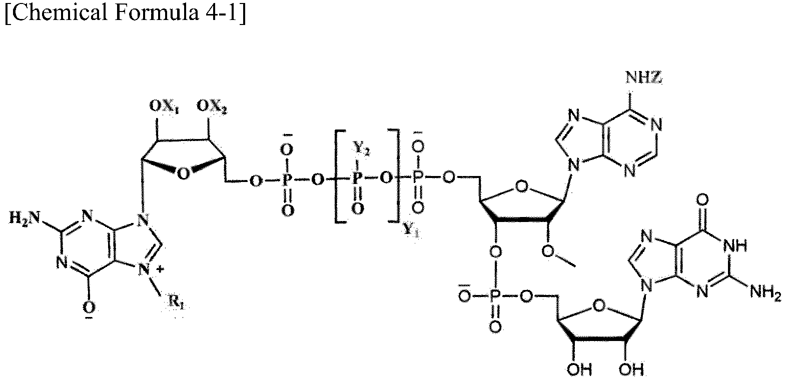

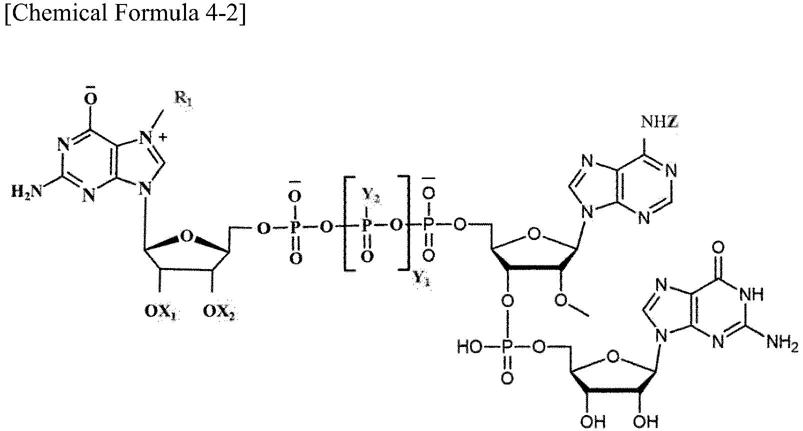

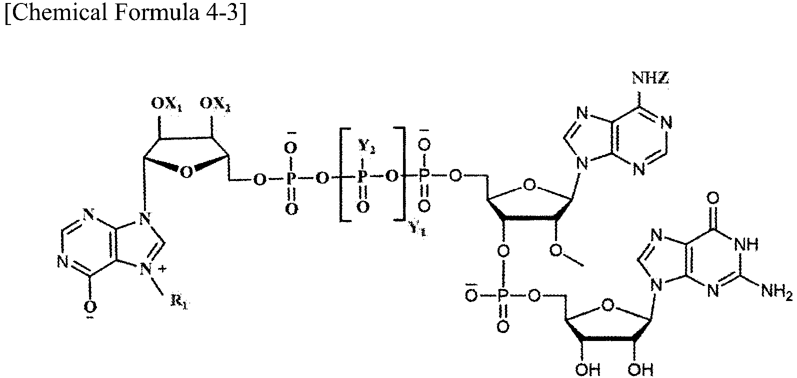

In the compound of Chemical Formula [4-1], [4-2], or [4-3],
R₁ is a compound comprising a compound of Chemical Formula 5 linked via a methyl (CH₂) group to the N7 position,
X₁ and X₂ are each H or CH₃,
Y₁ is 0 or 1,
Y₂ is O or S, and
Z is H or C₁-C₃ alkylbenzyl,

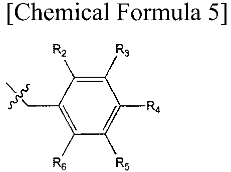

In Chemical Formula 5, R₂, R₃, R₄, R₅, and R₆ are each independently a substituent selected from the group consisting of H, OH, halo, methyl, alkyl, alkoxy, nitro, carboxylic, azide, amino, and cyano.

The present invention also provides an mRNA capped at the 5' end with the trinucleotide cap analog.

The present invention also provides a composition or kit for preparing an mRNA capped at the 5' end comprising the trinucleotide cap analog.

The present invention also provides a pharmaceutical composition for expression of a desired peptide or protein, comprising an mRNA capped at the 5' end with the trinucleotide cap analog and a pharmaceutically acceptable carrier.

### ADVANTAGEOUS EFFECTS

The derivatives introduced at the N7 position of the 5'-terminal guanosine base in the present invention, namely methylene-bridged heterocyclic compounds and arylmethyl compounds, introduce methylene-bridged heterocyclic groups and arylmethyl groups at the N7 position of the 5'-terminal guanosine base, and thus exhibit higher efficiency than the capping substances used in conventional mRNA vaccines, and can therefore be usefully applied in the development of mRNA vaccines and various mRNA-based therapeutic agents.

### DESCRIPTION OF THE DRAWINGS

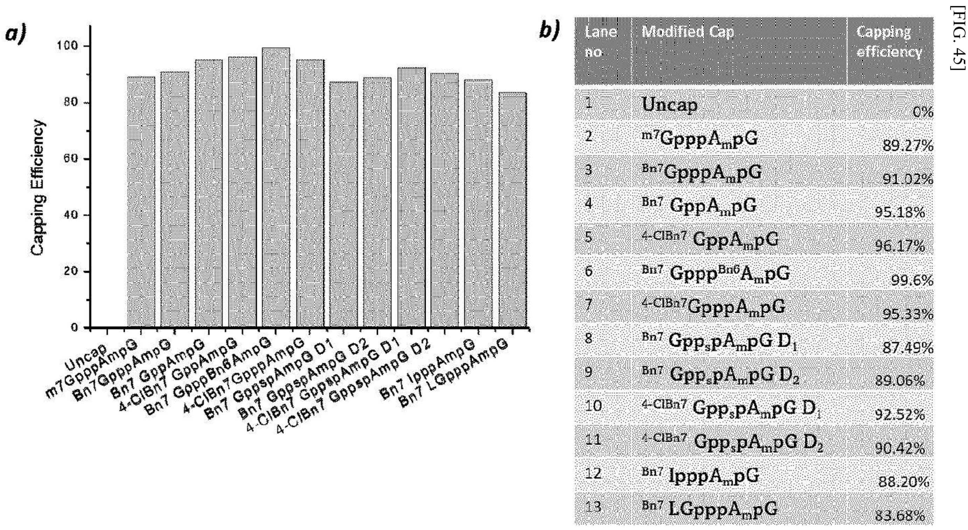

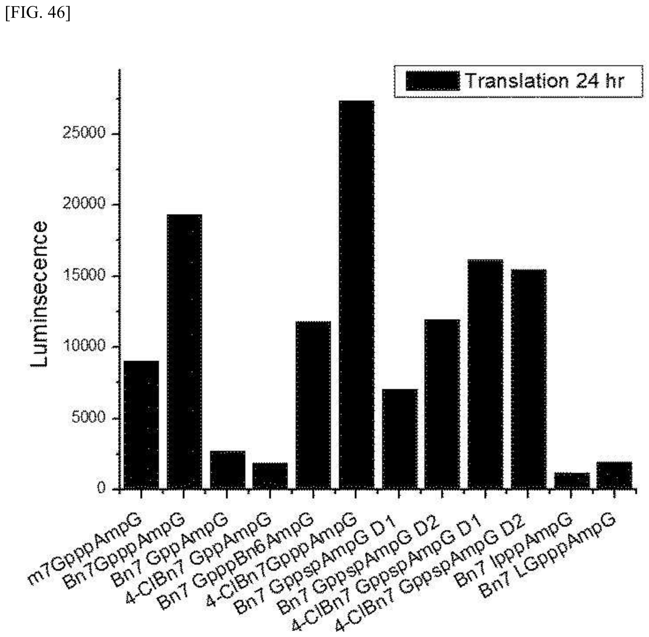

FIG. 1 illustrates trinucleotide-based capping substances, ThioCap1 and PyCap1, in which methylene-bridged thiophene and methylene-bridged pyridine groups, respectively, are introduced at the N7 position (R1) of the 5'-terminal guanosine.

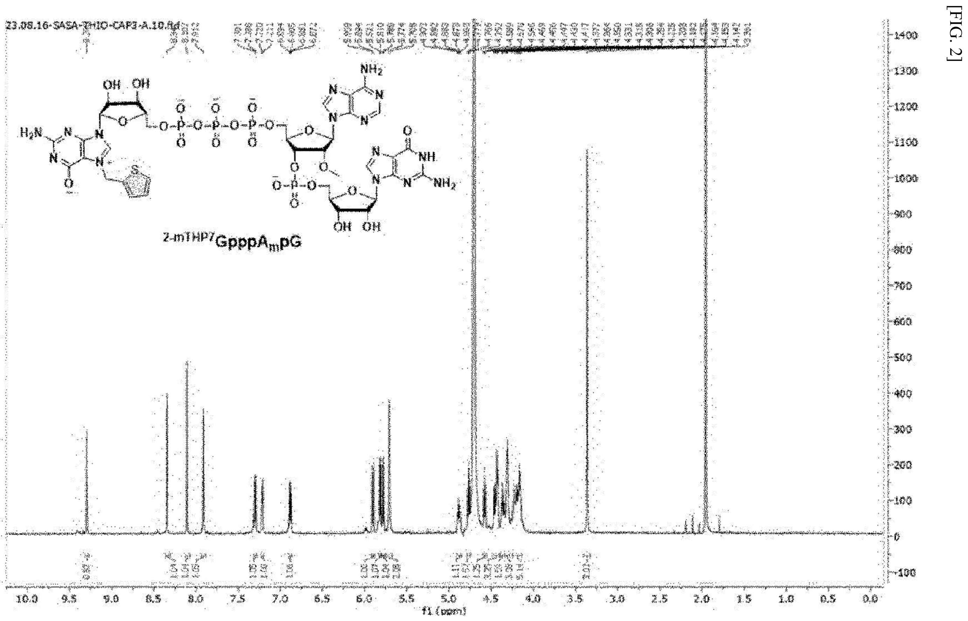

FIG. 2 is a graph showing the ¹H NMR spectrum of the ThioCapl substance.

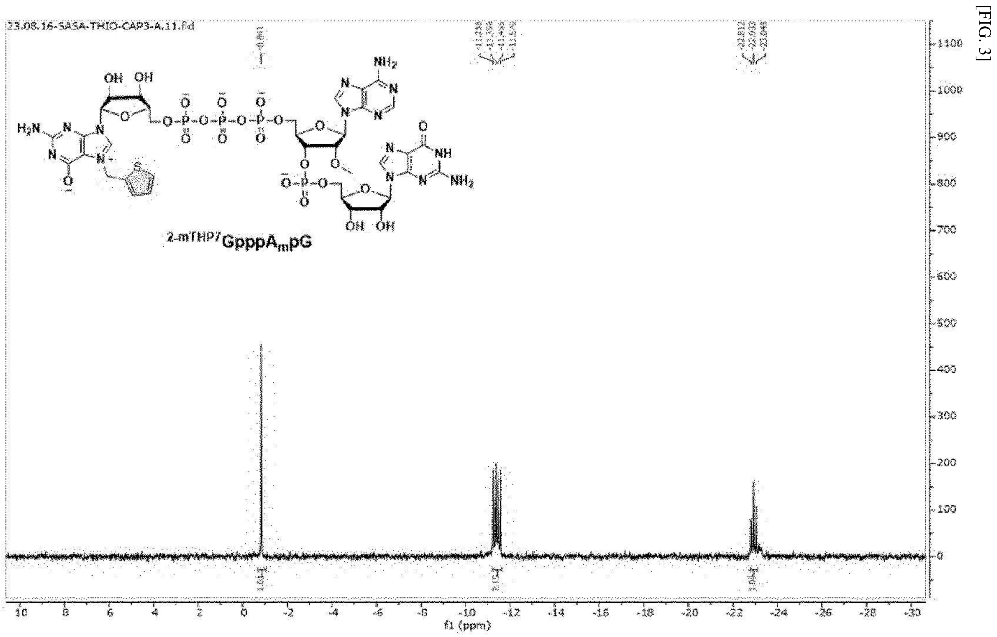

FIG. 3 is a graph showing the ³¹P NMR spectrum of the ThioCap1 substance.
FIG. 4 is a graph showing the ¹H NMR spectrum of the PyCapl substance.

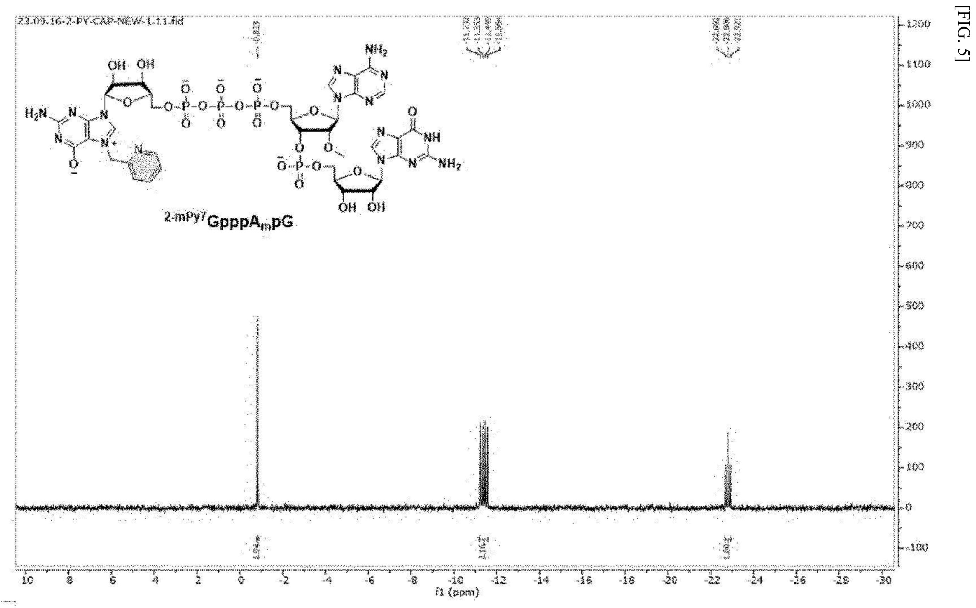

FIG. 5 is a graph showing the ³¹P NMR spectrum of the PyCapl substance.

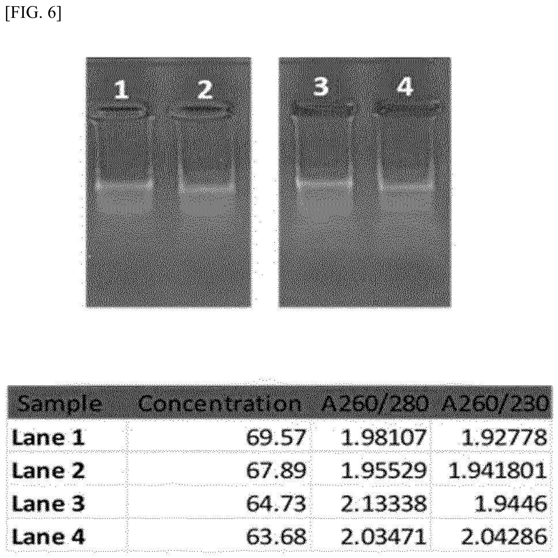

FIG. 6 shows verification of transcription of the capping substance (ThioCapl) of the present invention using T7 RNA polymerase (Lane1: Luciferase Template + T7RNAPol + rNTPs + CleanCap (Commercial Capl); Lane2: Luciferase Template + T7RNAPol + rATP + rGTP + rCTP + PseudoUTP + CleanCap (Commercial Cap1); Lane3: Luciferase Template + T7RNAPol + rNTPs + ThioCapl; Lane4: Luciferase Template + T7RNAPol + rATP + rGTP + rCTP + PseudoUTP + ThioCapl).
FIG. 7 shows verification of the capping efficiency of the capping substance (ThioCapl) of the present invention using T7 RNA polymerase and treatment with RNase H (Lane1: Luciferase Template + T7RNAPol + rNTPs → gDNA + RNase H; Lane2: Luciferase Template + T7RNAPol + rNTPs + ARCA → gDNA + RNase H; Lane3: Luciferase Template + T7RNAPol + rNTPs + CleanCap → gDNA + RNase H; Lane4: Luciferase Template + T7RNAPol + rNTPs + ThioCapl → gDNA + RNase H).
FIG. 8 is a graph comparing the expression of the antigen protein (Luciferase) between Luciferase mRNA (Luc mRNA ThioCapl) introduced with the capping substance (ThioCapl) of the present invention and Luciferase mRNA (Luc mRNA Cap1) introduced with conventional CleanCap.

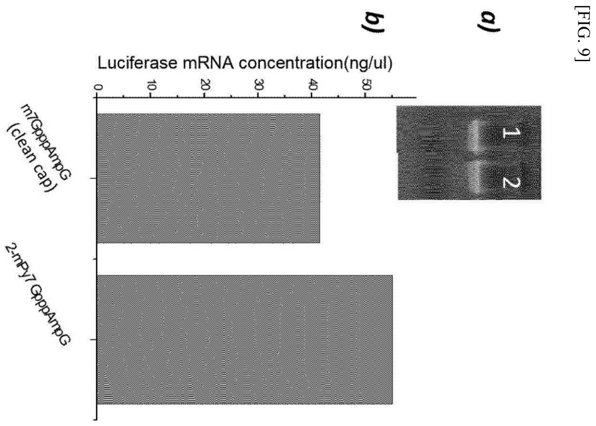

FIG. 9 shows verification of transcription of the capping substance (PyCap1) of the present invention using T7 RNA polymerase (Lane1: Luciferase Template + T7RNAPol + rNTPs + CleanCap (Commercial Capl); Lane2: Luciferase Template + T7RNAPol + rNTPs + PyCapl).
FIG. 10 shows verification of the capping efficiency of the capping substance (PyCapl) of the present invention using RNA polymerase and treatment with RNase H.
FIG. 11 is a graph comparing the expression of the antigen protein (Luciferase) between Luciferase mRNA (Luc mRNA PyCapl) introduced with the capping substance (PyCapl) of the present invention and Luciferase mRNA (Luc mRNA Cap1) introduced with conventional CleanCap.
FIG. 12 shows the chemical structure of a trinucleotide-based capping substance (BzCapl) in which an arylmethyl (benzyl) group is introduced at the N7 position (R1) of the 5'-terminal guanosine.

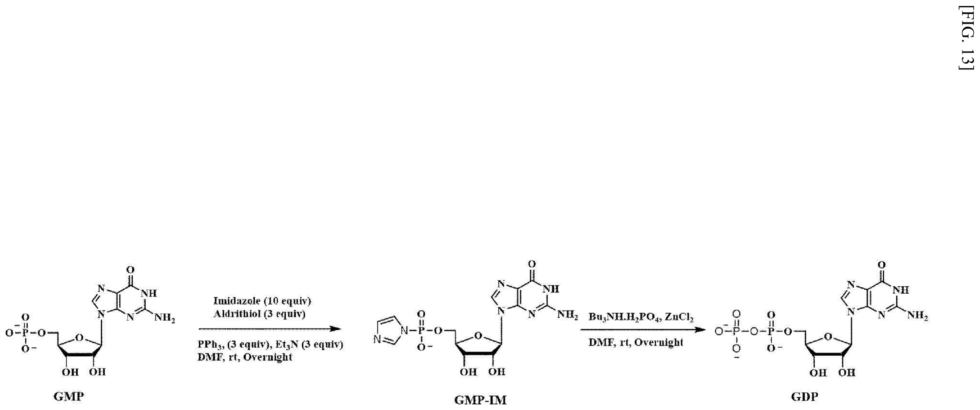

FIG. 13 is a schematic diagram illustrating a synthetic procedure of an N7-(arylmethyl)guanosine 5'-diphosphoimidazolide analog.
FIG. 14 is a schematic diagram illustrating a synthetic procedure of the dinucleotide pAmpG.

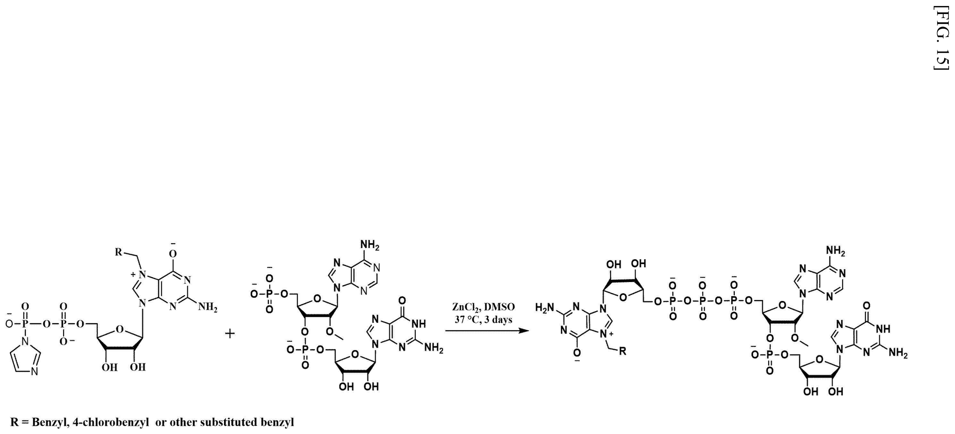

FIG. 15 is a schematic diagram illustrating a synthetic procedure of a guanosine N7-(aryl-2-ylmethyl) trinucleotide mRNA cap1 analog.

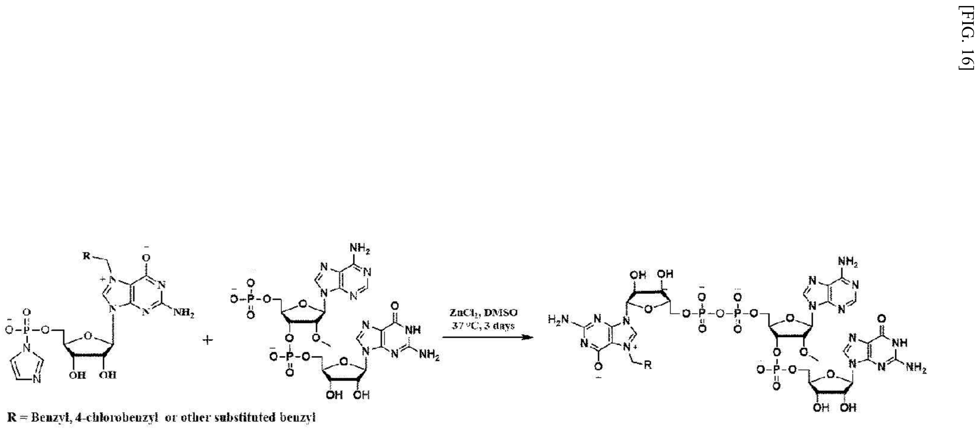

FIG. 16 is a schematic diagram illustrating a synthetic procedure of an N7-(aryl-2-ylmethyl) trinucleotide diphosphate cap1 analog.

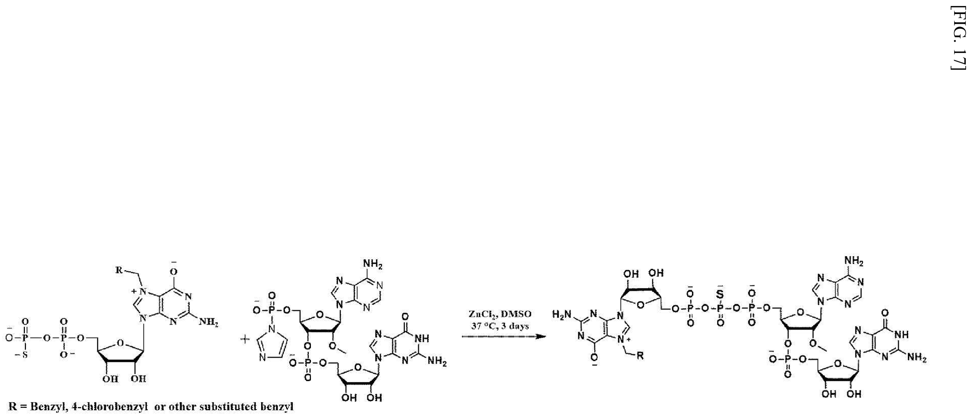

FIG. 17 is a schematic diagram illustrating a synthetic procedure of an N7-(aryl-2-ylmethyl) β-phosphorothioate trinucleotide cap1 analog.

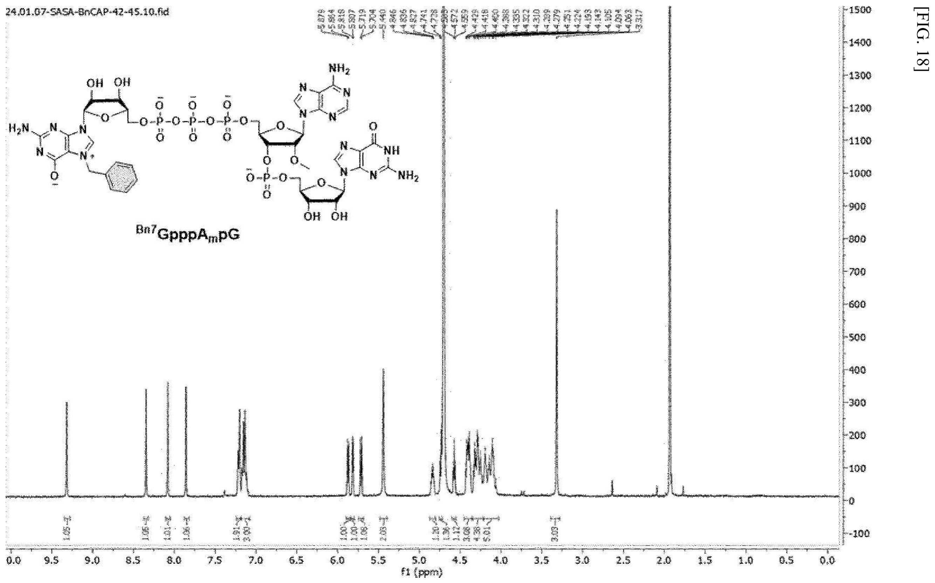

FIG. 18 is a ¹H NMR spectrum of a guanosine N7-benzyl trinucleotide cap analog.

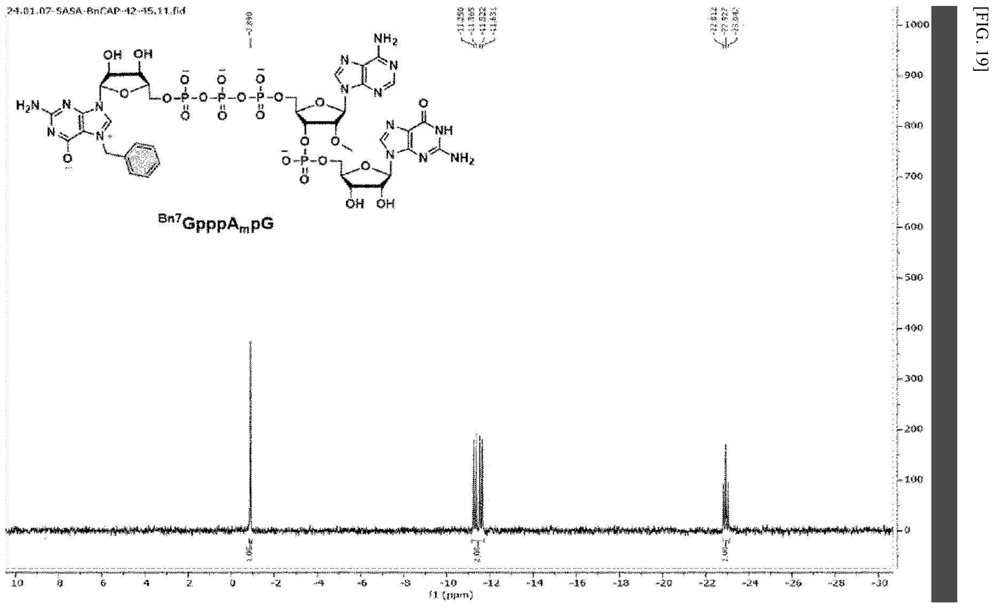

FIG. 19 is a ³¹P NMR spectrum of a guanosine N7-benzyl trinucleotide cap analog.

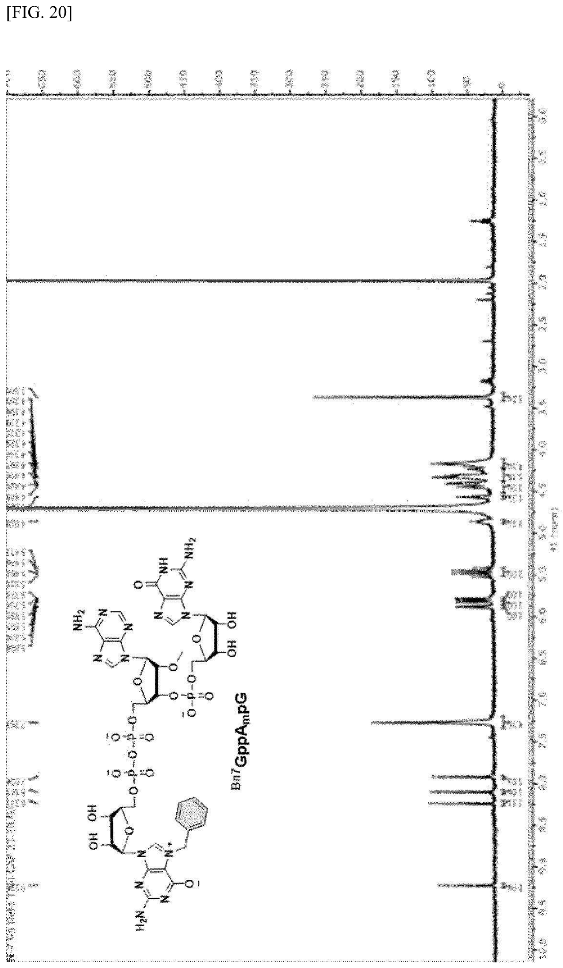

FIG. 20 is a ¹H NMR spectrum of a guanosine N7-benzyl trinucleotide diphosphate cap analog.

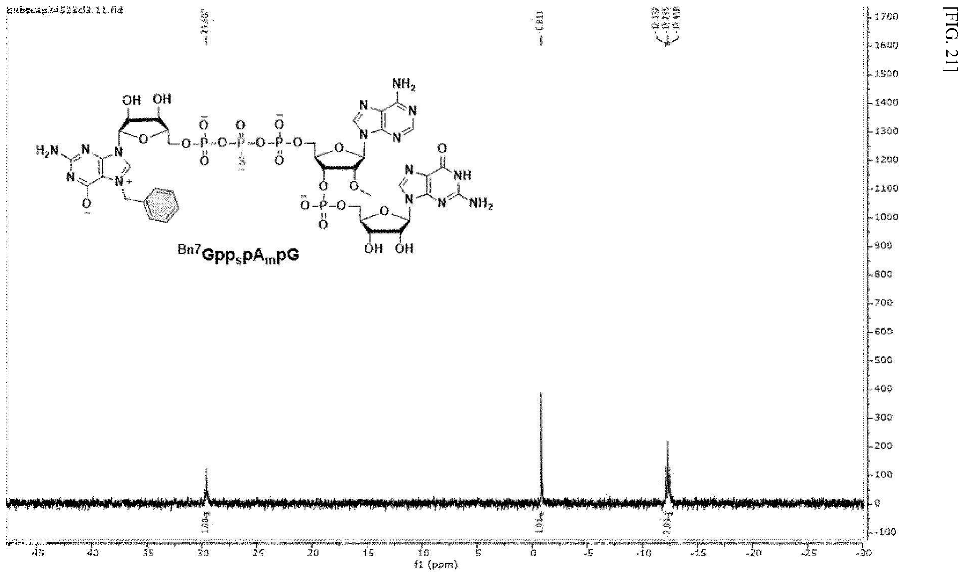

FIG. 21 is a ³¹P NMR spectrum of a guanosine N7-benzyl trinucleotide diphosphate cap analog.

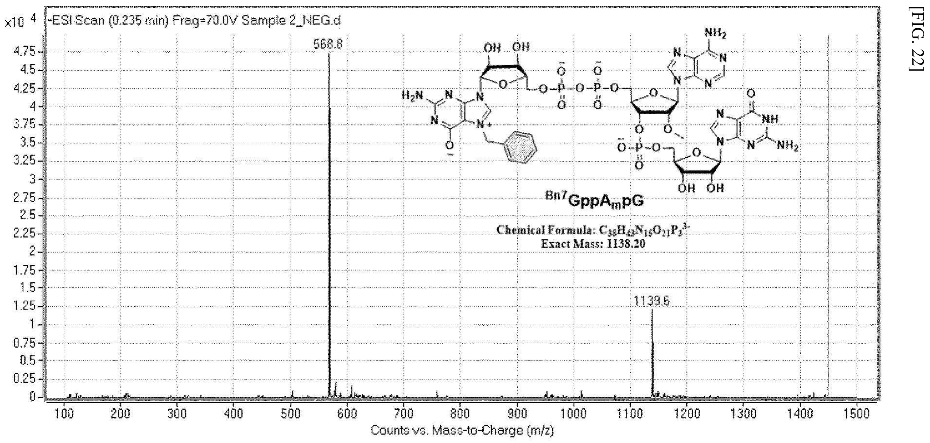

FIG. 22 is an ESI-MS spectrum of a guanosine N7-benzyl trinucleotide diphosphate cap analog.

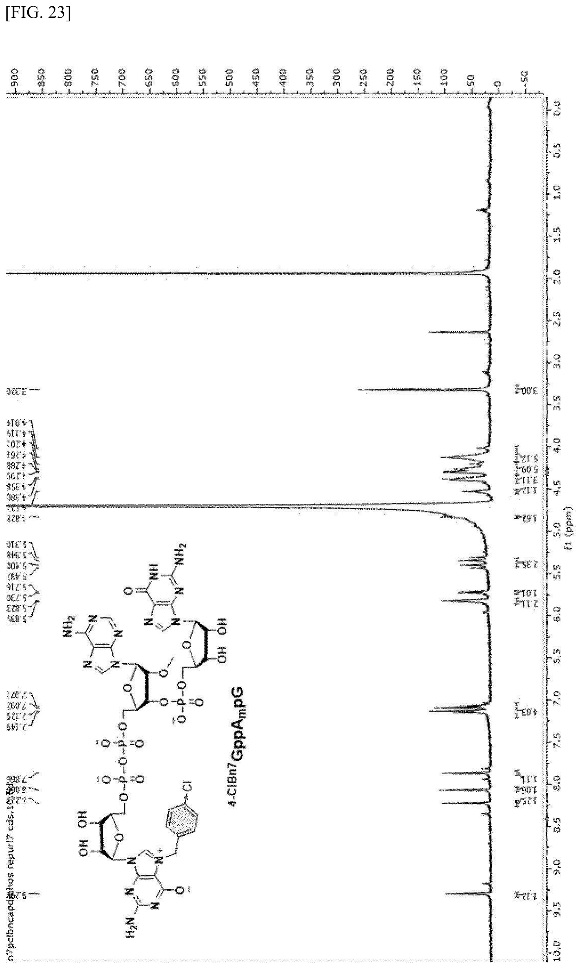

FIG. 23 is a ¹H NMR spectrum of a guanosine N7-4-Cl-benzyl trinucleotide diphosphate cap analog.

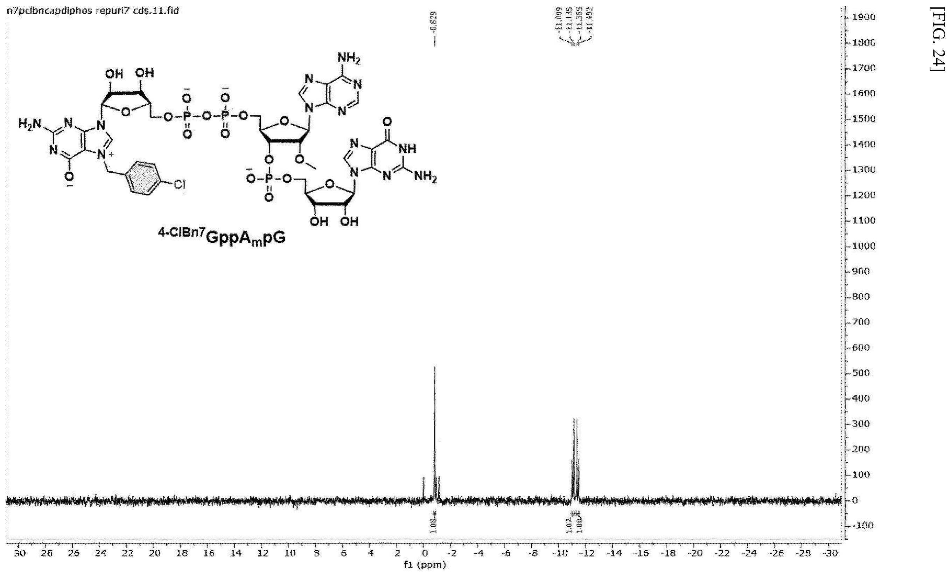

FIG. 24 is a ³¹P NMR spectrum of a guanosine N7-4-Cl-benzyl trinucleotide diphosphate cap analog.

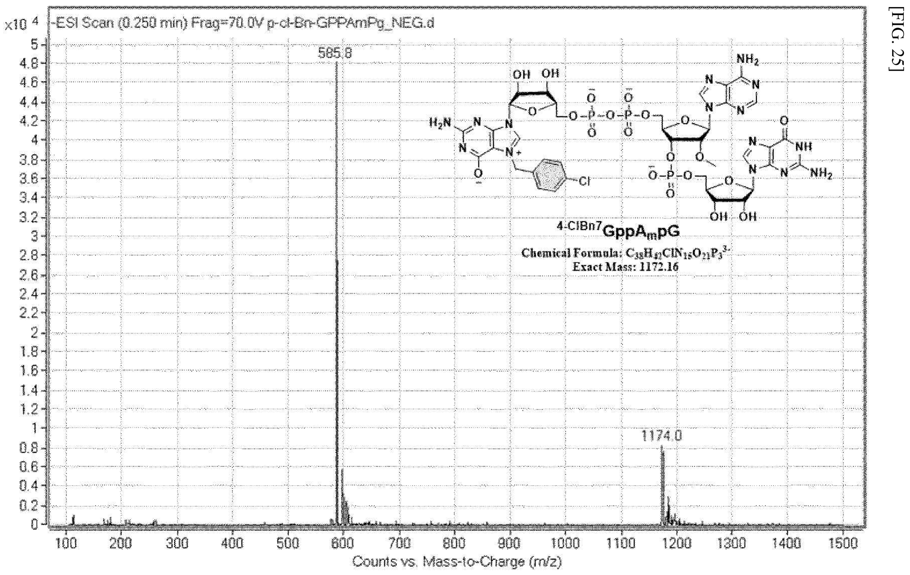

FIG. 25 is an ESI-MS spectrum of a guanosine N7-4-Cl-benzyl trinucleotide diphosphate cap analog.

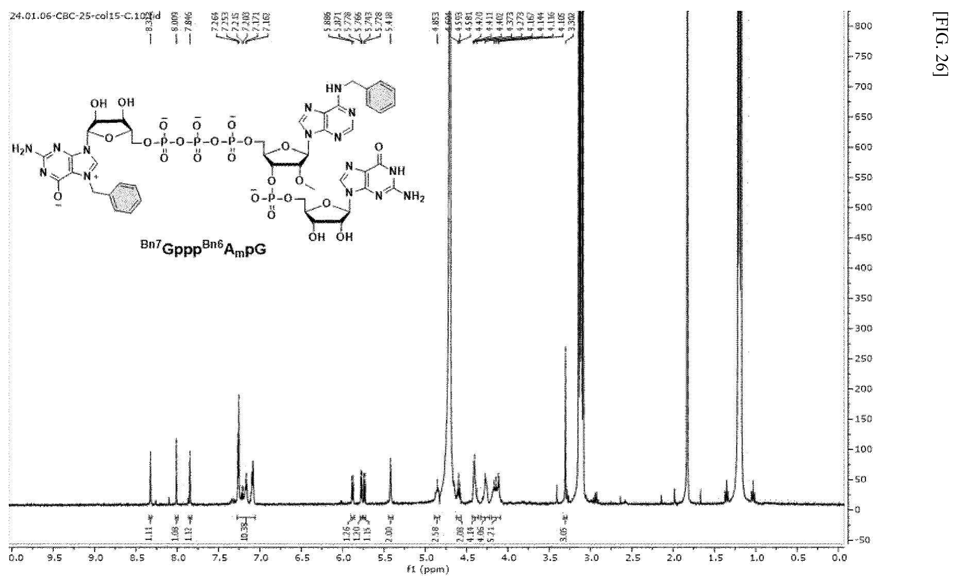

FIG. 26 is a ¹H NMR spectrum of a guanosine N7, adenosine N6-dibenzyl trinucleotide cap analog.

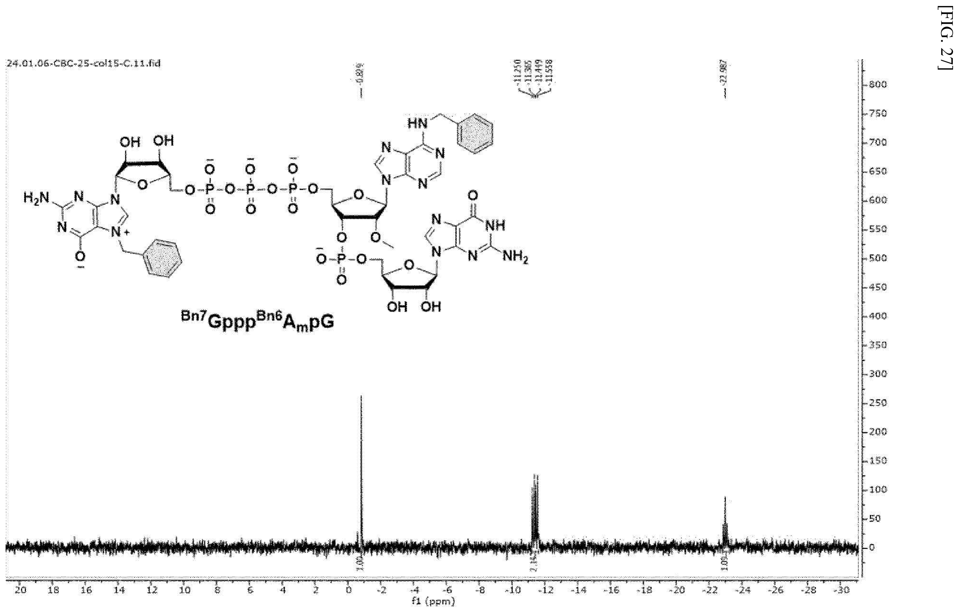

FIG. 27 is a ³¹P NMR spectrum of a guanosine N7, adenosine N6-dibenzyl trinucleotide cap analog.

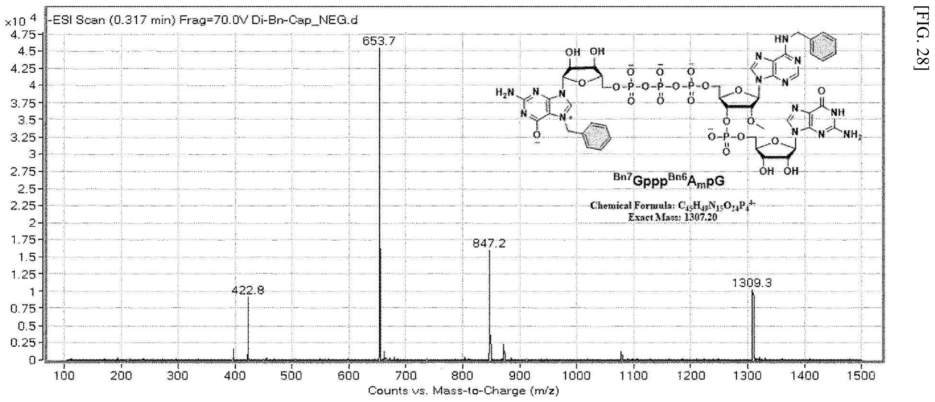

FIG. 28 is an ESI-MS spectrum of a guanosine N7, adenosine N6-dibenzyl trinucleotide cap analog.

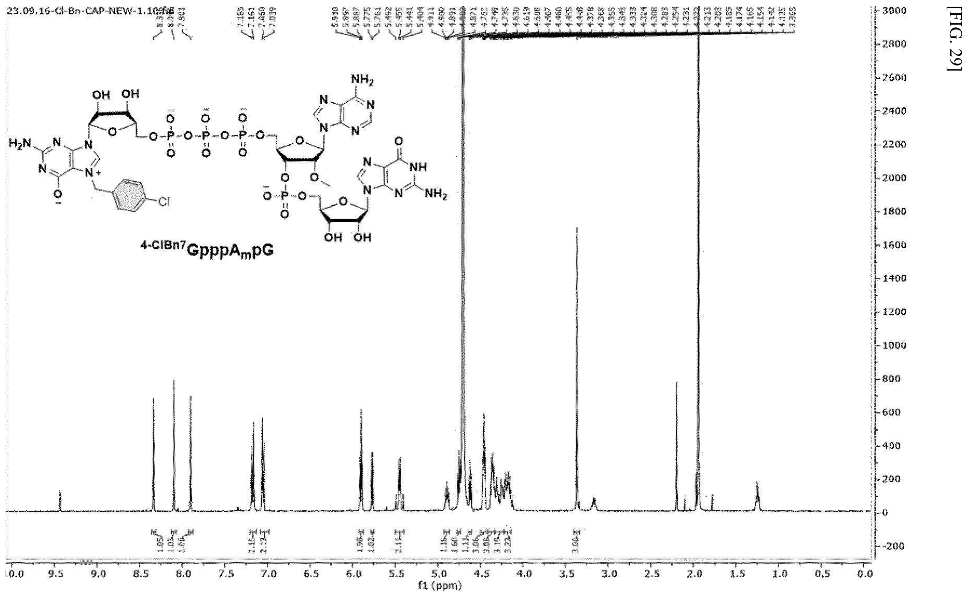

FIG. 29 is a ¹H NMR spectrum of a guanosine N7-4-Cl-benzyl trinucleotide cap analog.

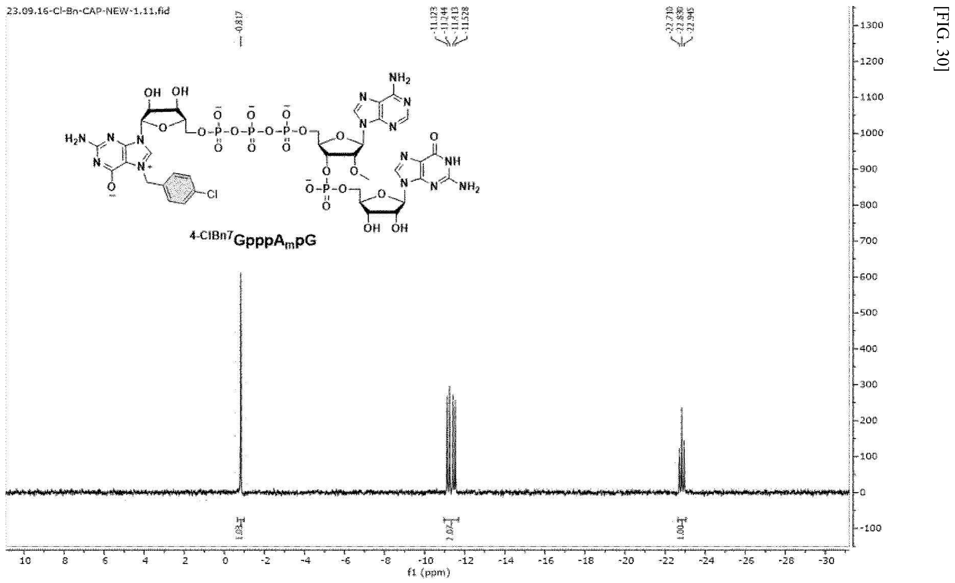

FIG. 30 is a ³¹P NMR spectrum of a guanosine N7-4-Cl-benzyl trinucleotide cap analog.

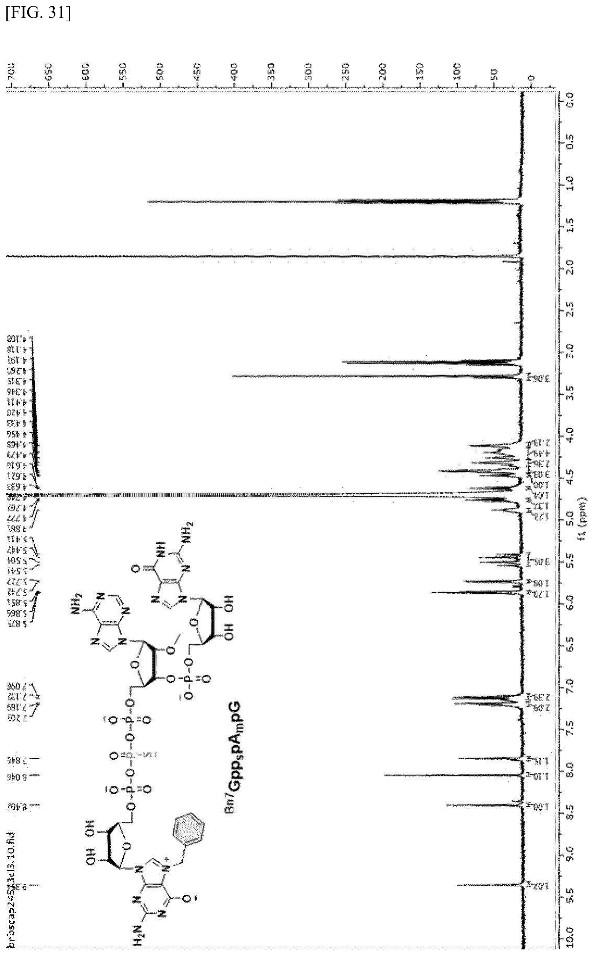

FIG. 31 is a ¹H NMR spectrum of a guanosine N7-benzyl β-phosphorothioate trinucleotide cap analog (D1).

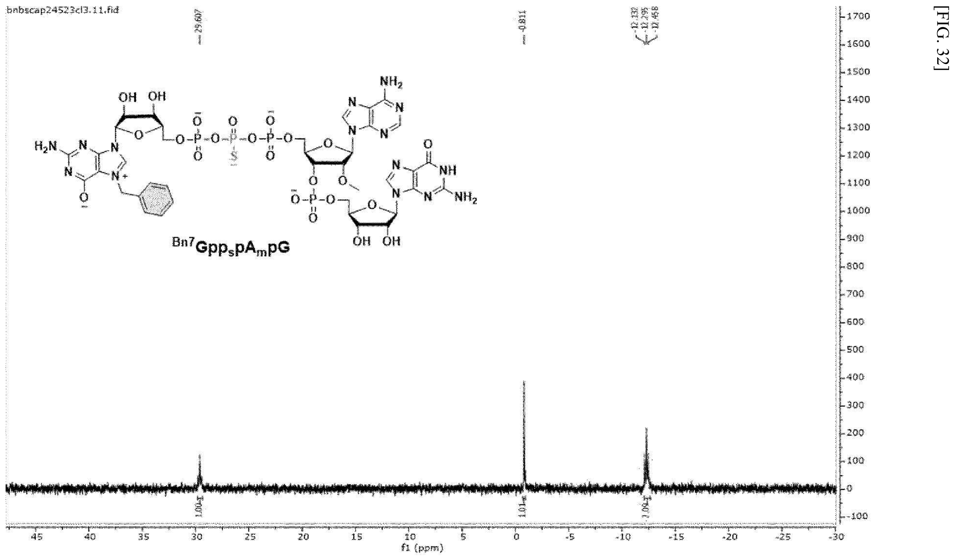

FIG. 32 is a ³¹P NMR spectrum of a guanosine N7-benzyl β-phosphorothioate trinucleotide cap analog (D1).

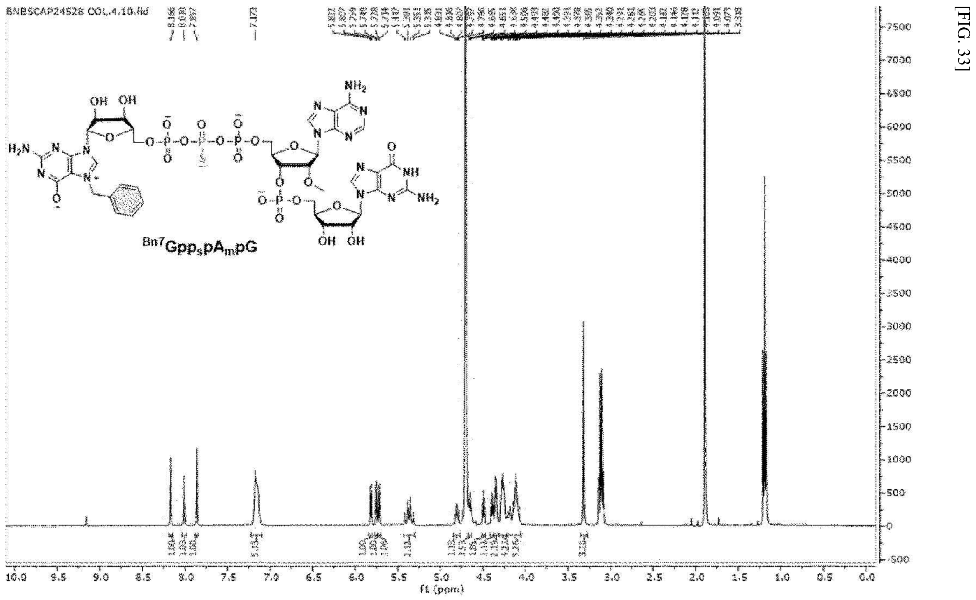

FIG. 33 is a ¹H NMR spectrum of a guanosine N7-benzyl β-phosphorothioate trinucleotide cap analog (D2).

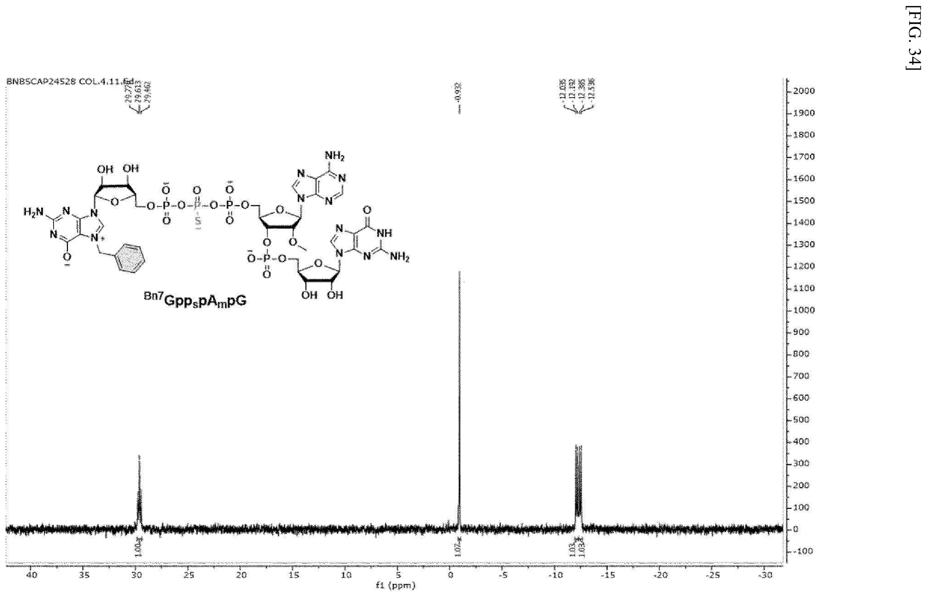

FIG. 34 is a ³¹P NMR spectrum of a guanosine N7-benzyl β-phosphorothioate trinucleotide cap analog (D2).

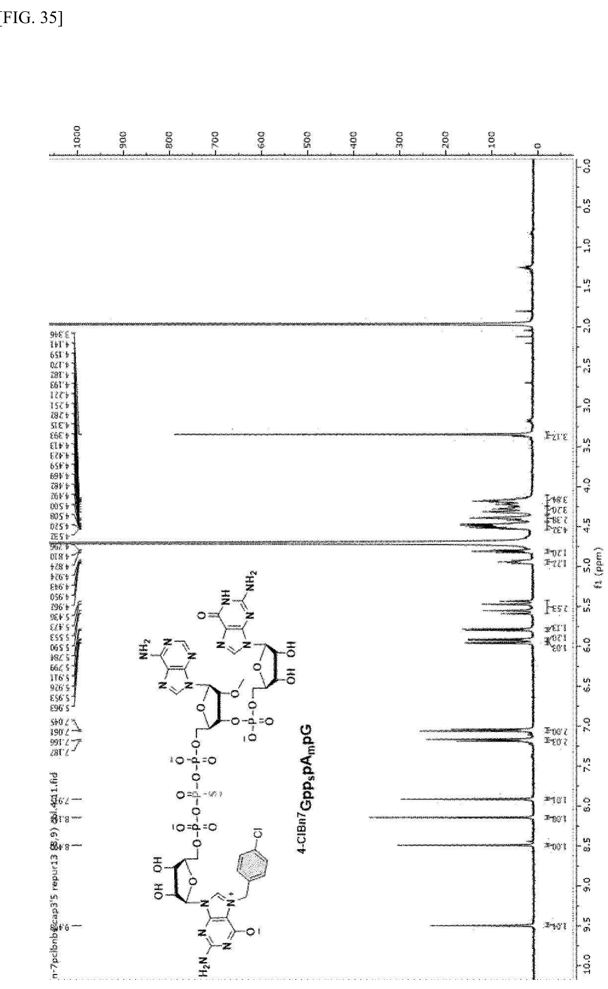

FIG. 35 is a ¹H NMR spectrum of a guanosine N7-4-Cl-benzyl β-phosphorothioate trinucleotide cap analog (D1).

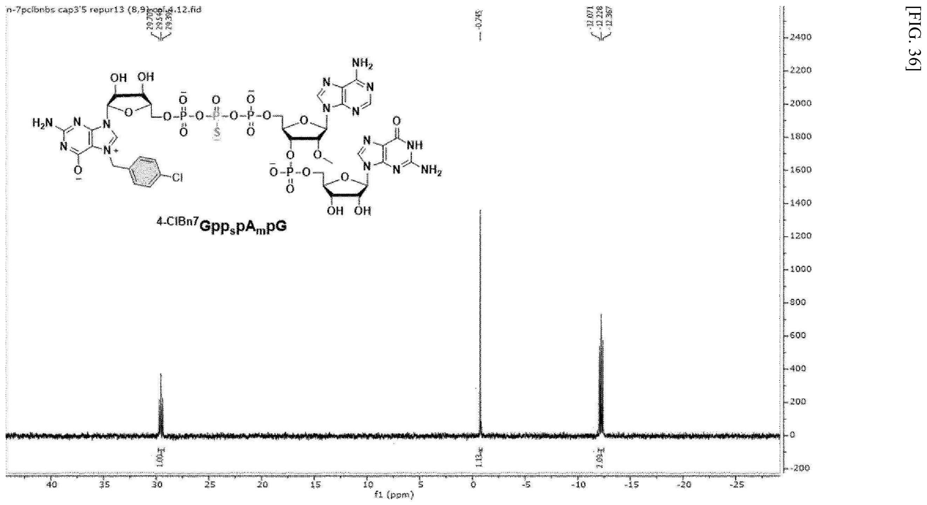

FIG. 36 is a ³¹P NMR spectrum of a guanosine N7-4-Cl-benzyl β-phosphorothioate trinucleotide cap analog (D1).

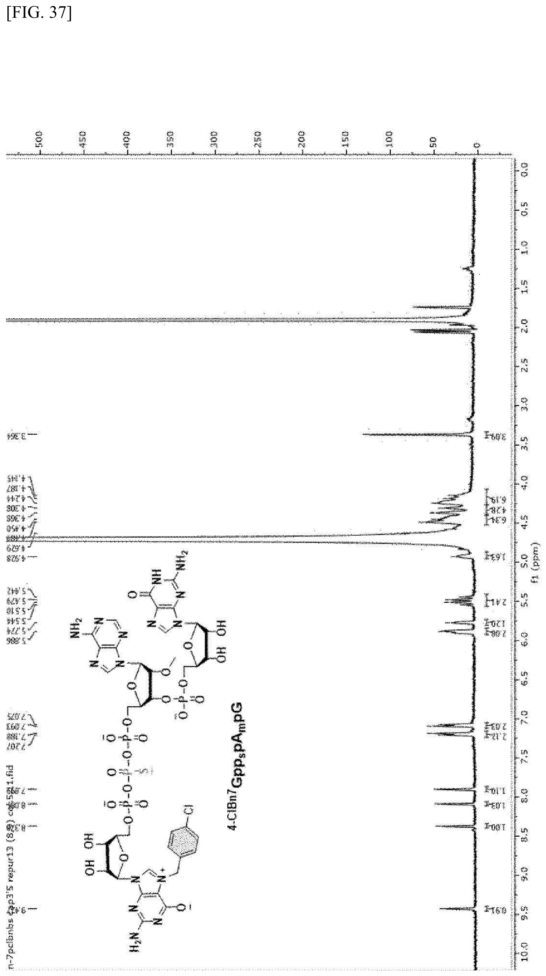

FIG. 37 is a ¹H NMR spectrum of a guanosine N7-4-Cl-benzyl β-phosphorothioate trinucleotide cap analog (D2).

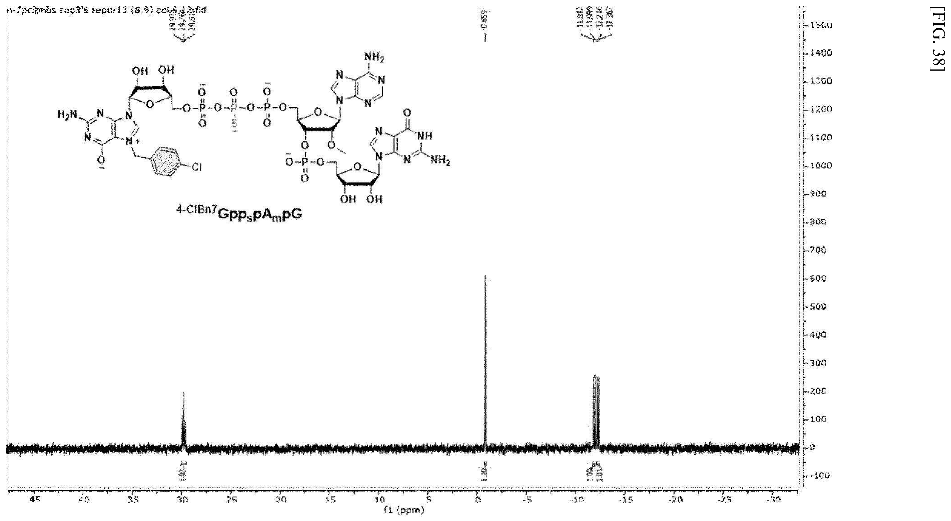

FIG. 38 is a ³¹P NMR spectrum of a guanosine N7-4-Cl-benzyl β-phosphorothioate trinucleotide cap analog (D2).

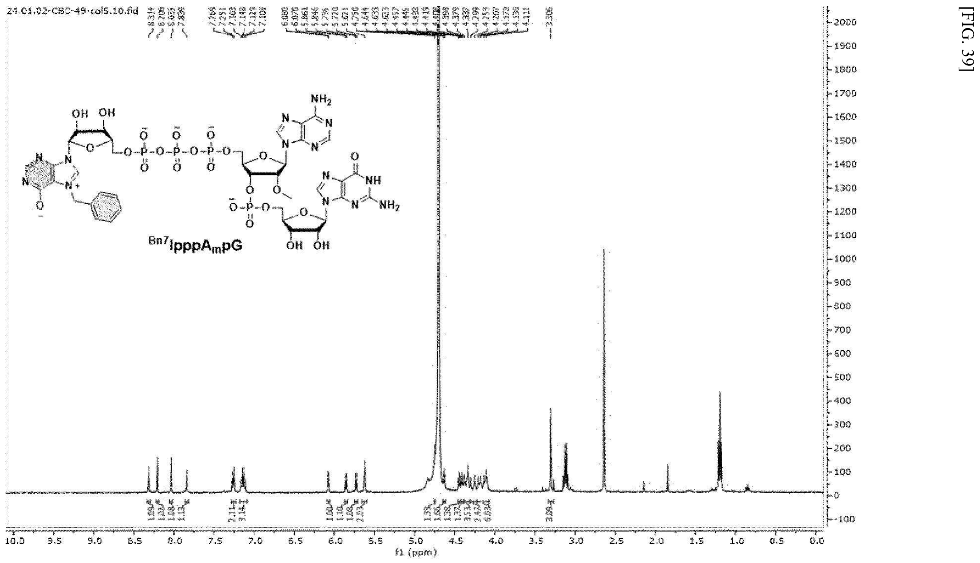

FIG. 39 is a ¹H NMR spectrum of an inosine N7-benzyl trinucleotide cap analog.

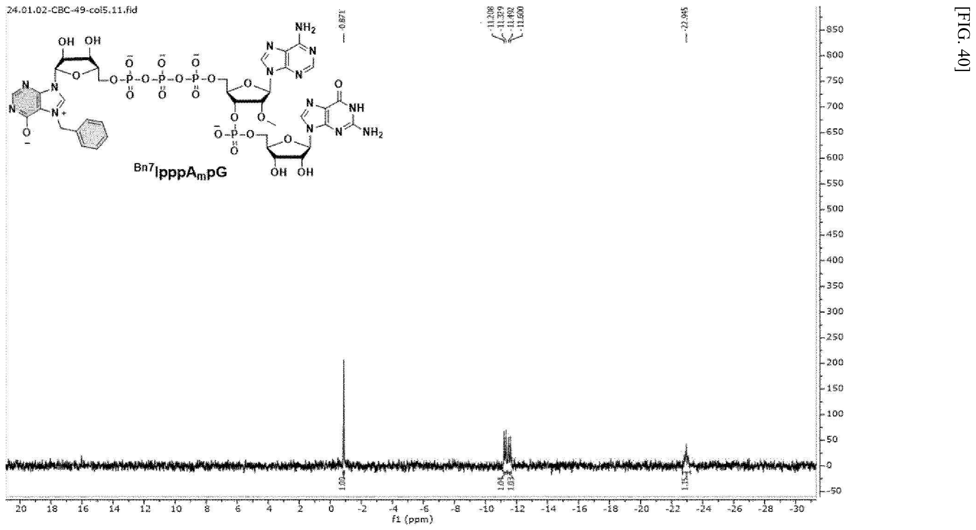

FIG. 40 is a ³¹P NMR spectrum of an inosine N7-benzyl trinucleotide cap analog.

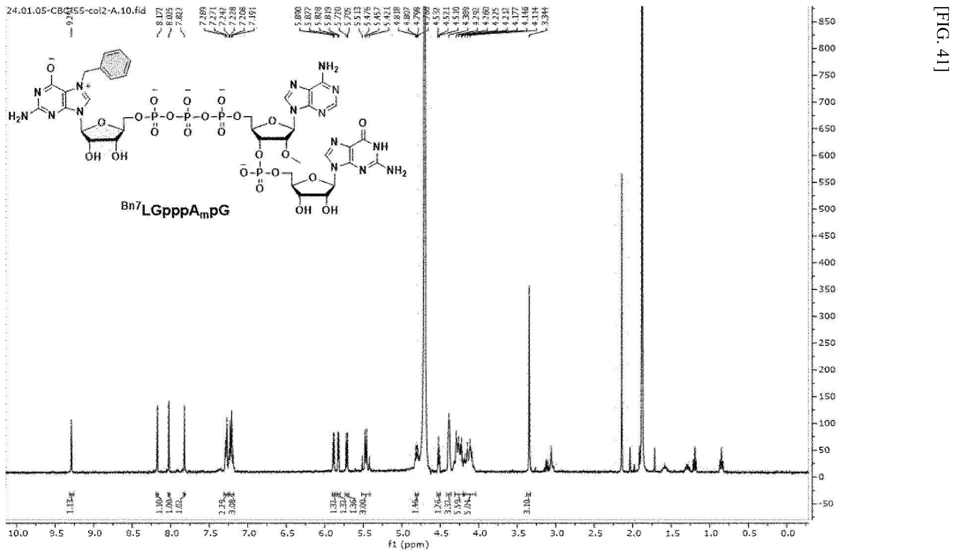

FIG. 41 is a ¹H NMR spectrum of an L-guanosine N7-benzyl trinucleotide cap analog.

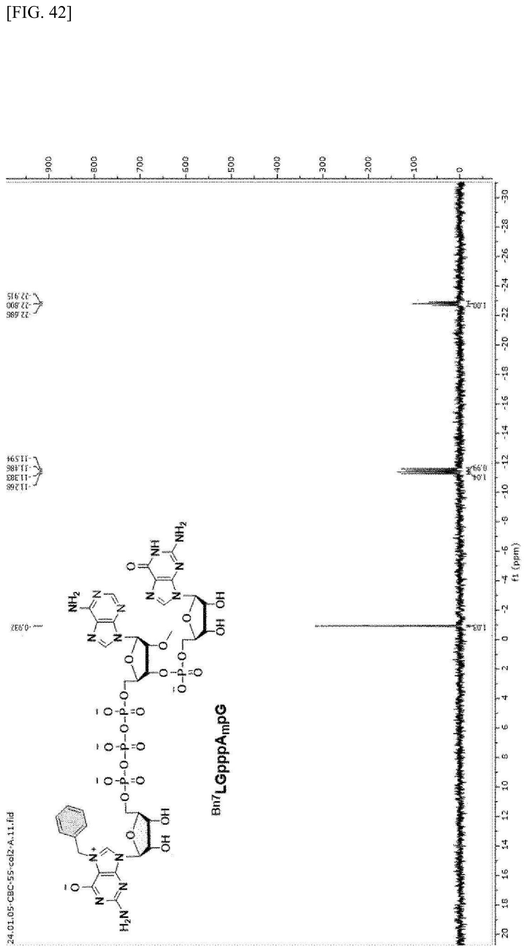

FIG. 42 is a ³¹P NMR spectrum of an L-guanosine N7-benzyl trinucleotide cap analog.

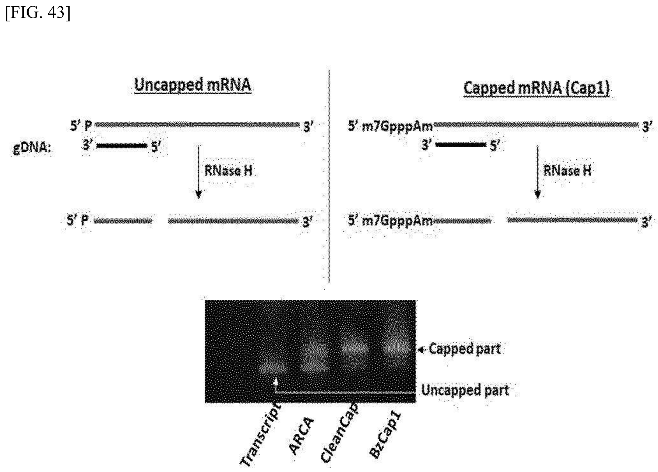

FIG. 43 is a gel image showing transcription of the capping substance (BzCAP1) using T7 RNA polymerase and verifying capping efficiency by RNase H treatment in vitro; capping efficiency was compared with conventional ARCA and CleanCap.

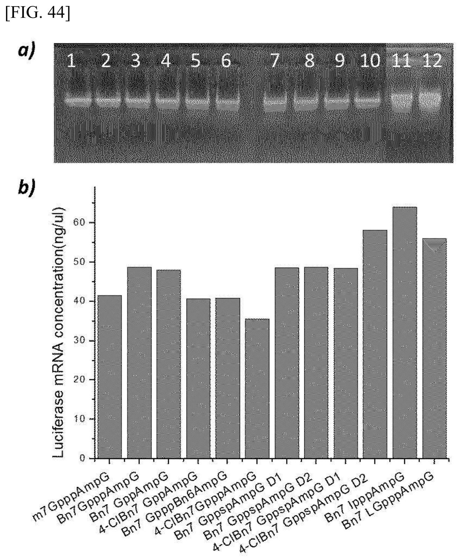

FIG. 44 shows transcription results of various arylmethyl-modified trinucleotide-based capping substances according to the present invention using T7 RNA polymerase.
   Lane 1: Luciferase mRNA + ^{m7}GpppAₘpG
   Lane 2: Luciferase mRNA + ^{Bn7}GpppAₘpG
   Lane 3: Luciferase mRNA + ^{Bn7}GppAₘpG
   Lane 4: Luciferase mRNA + ^{4-ClBn7}GppAₘpG
   Lane 5: Luciferase mRNA + ^{Bn7}GpppBn6AₘpG
   Lane 6: Luciferase mRNA + ^{4-ClBn7}GpppAₘpG
   Lane 7: Luciferase mRNA + ^{Bn7}GppspAₘpG (D1)
   Lane 8: Luciferase mRNA + ^{Bn7}GppspAₘpG (D2)
   Lane 9: Luciferase mRNA + ^{4-ClBn7}GppspAₘpG (D1)
   Lane 10: Luciferase mRNA + ^{4-ClBn7}GppspAₘpG (D2)
   Lane 11: Luciferase mRNA + ^{Bn7}IpppAₘpG
   Lane 12: Luciferase mRNA + ^{Bn7}LGpppAₘpG
FIG. 45 shows verification of capping efficiency of various arylmethyl-modified trinucleotide-based capping substances according to the present invention using RNA polymerase and RNase H treatment.
   Lane 1: Luciferase mRNA + ^{m7}GpppAₘpG
   Lane 2: Luciferase mRNA + ^{Bn7}GpppAₘpG
   Lane 3: Luciferase mRNA + ^{Bn7}GppAₘpG
   Lane 4: Luciferase mRNA + ^{4-ClBn7}GppAₘpG
   Lane 5: Luciferase mRNA + ^{Bn7}GpppBn6AₘpG
   Lane 6: Luciferase mRNA + ^{4-ClBn7}GpppAₘpG
   Lane 7: Luciferase mRNA + ^{Bn7}GppspAₘpG (D1)
   Lane 8: Luciferase mRNA + ^{Bn7}GppspAₘpG (D2)
   Lane 9: Luciferase mRNA + ^{4-ClBn7}GppspAₘpG (D1)
   Lane 10: Luciferase mRNA + ^{4-ClBn7}GppspAₘpG (D2)
   Lane 11: Luciferase mRNA + ^{Bn7}IpppAₘpG
   Lane 12: Luciferase mRNA + ^{Bn7}LGpppAₘpG
FIG. 46 is a graph showing the expression of an antigen protein (Luciferase) using trinucleotide-based capping substances in which the N7 position of the 5'-terminal guanosine has been modified with various arylmethyl groups.
   Lane 1: Luciferase mRNA + ^{m7}GpppAₘpG
   Lane 2: Luciferase mRNA + ^{Bn7}GpppAₘpG
   Lane 3: Luciferase mRNA + ^{Bn7}GppAₘpG
   Lane 4: Luciferase mRNA + ^{4-ClBn7}GppAₘpG
   Lane 5: Luciferase mRNA + ^{Bn7}GpppBn6AₘpG
   Lane 6: Luciferase mRNA + ^{4-ClBn7}GpppAₘpG
   Lane 7: Luciferase mRNA + ^{Bn7}GppspAₘpG (D1)
   Lane 8: Luciferase mRNA + ^{Bn7}GppspAₘpG (D2)
   Lane 9: Luciferase mRNA + ^{4-ClBn7}GppspAₘpG (D1)
   Lane 10: Luciferase mRNA + ^{4-ClBn7}GppspAₘpG (D2)
   Lane 11: Luciferase mRNA + ^{Bn7}IpppAₘpG
   Lane 12: Luciferase mRNA + ^{Bn7}LGpppAₘpG

### BEST MODE

Hereinafter, the present invention will be described in detail.

The inventors of the present invention prepared novel modified trinucleotide capping derivatives and mRNA introduced with the capping derivatives. Specifically, they developed various modified capping compounds in which a methylene-bridged heterocyclic group and an arylmethyl group were introduced, respectively, at the N7 position of the 5'-terminal guanosine base, so as to increase the antigen protein expression efficiency of mRNA vaccines similar to CleanCap and to be more efficient than CleanCap-modified nucleotides. They confirmed that mRNA constructs introduced with various methylene-bridged heterocyclic groups and/or arylmethyl groups exhibited superior antigen protein expression efficiency compared with mRNA capped with conventional capping substances used in mRNA vaccines, thereby completing the present invention.

A feature of the present invention lies in designing and synthesizing novel modified trinucleotide capping substances and verifying their potential use as mRNA vaccines. Among the trinucleotide structures of CleanCap, new compounds were synthesized through organic chemical reactions by modifying the N7 position of the 5'-terminal guanosine base from a conventional methyl group to a methylene-bridged thiophene compound, a methylene-bridged pyridine compound, and an arylmethyl compound, and the synthesis processes and results are shown.

It was confirmed that the capping substances were recognized by T7 RNA polymerase during the transcription process with similar or higher efficiency compared with ARCA or CleanCap and could be introduced into RNA. Specifically, ARCA exhibited approximately 50% capping efficiency, CleanCap approximately 86%, ThioCap1 approximately 92% or higher, BzCap1 and its analogs approximately 90% or higher, and the PyCap1 capping substance also showed 88% or higher capping efficiency.

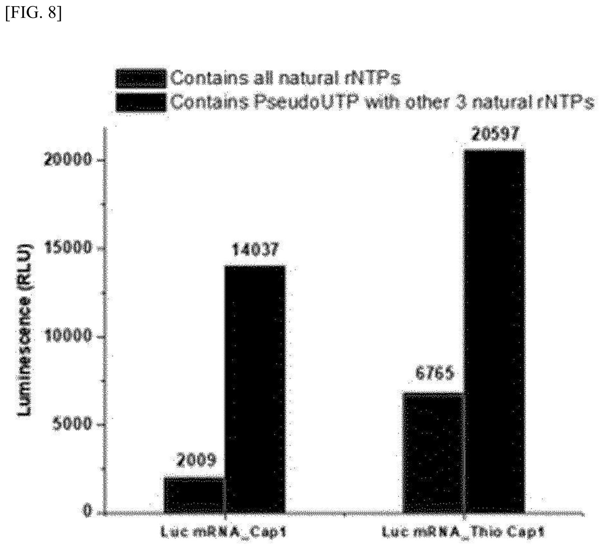

Furthermore, it was confirmed that Luciferase mRNA (Luc mRNA ThioCap1), Luciferase mRNA (Luc mRNA PyCapl), Luciferase mRNA (Luc mRNA BzCapl), and Luciferase mRNA introduced with BzCap1 analogs developed in the present invention exhibited higher antigen protein expression efficiency than Luciferase mRNA (Luc mRNA Cap1) introduced with CleanCap (see FIGS. 8, 11, and 46).

**It** was also confirmed that Luciferase mRNA introduced with BzCap1 and BzCap1 analogs developed in the present invention exhibited higher antigen protein expression efficiency than mRNA introduced with ARCA cap (Cap0) and CleanCap (Cap1 commercial).

Accordingly, through the above results, it was confirmed that the trinucleotide cap analogs according to the present invention, which introduce methylene-bridged heterocyclic compounds and arylmethyl groups at the N7 position of the 5'-terminal guanosine base, are more efficient than conventional capping substances used in mRNA vaccines, and thus can be usefully applied in the development of new mRNA vaccines and various mRNA-based therapeutics.

A trinucleotide cap analog comprising a compound of Chemical Formula 1:

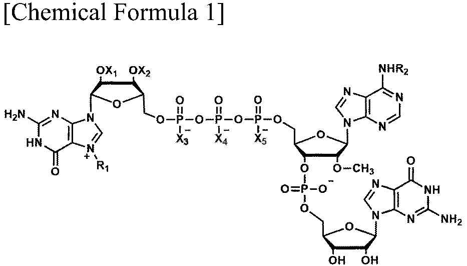

In the compound of Chemical Formula 1, R₁ is a methylene bridged heterocyclic compound linked via a methylene bridge to the N7 position of a guanosine base,
in the compound of Chemical Formula 1, X₁ and X₂ are H or CH₃, X₃, X₄, and X₅ are each selected from the group consisting of O, S, and Se,
R₂ is a derivative comprising benzyl,
the heterocyclic compound is any one substituent selected from heteroaromatic ring compounds including 1 to 2 heteroatoms selected from O, N, and S and being a saturated or partially saturated 5- to 10-membered heteroaromatic ring compound, and
the heteroaromatic ring compound may be any one substituent selected from the group consisting of furan, pyrole, thiophen, imidazole, pyrazole, oxazol, isoxazole, thiazole, pyridine, pyrazine, primidine, pyridazine, and triazine.

In the present invention, the term "heterocyclic compound" means an aromatic ring group including 1 to 2 heteroatoms selected from O, N, and S and being a saturated or partially saturated 5- to 10-membered aromatic ring group.

Specific examples of such heterocyclic compounds may include thiophen, pyridyl, imidazole, tetrahydrofuran-yl, 2,3-dihydrofuran-yl, 2,5-dihydrofuran-yl, pyrrolidin-yl, 2,3-dihydropyrrolidin-yl, 2,5-dihydropyrrolidin-yl, tetrahydro-2H-pyranyl, 3,4-dihydro-2H-pyranyl, 4H-pyranyl, piperidin-yl, 1,2,3,4-tetrahydropyridin-yl, 1,4-dihydropyridin-yl, piperazin-yl, N-protected piperazinyl, and morpholino. The N-protecting group of piperazinyl may typically include alkyl, alkylcarbonyl, or alkylsulfonyl groups.

A trinucleotide cap analog comprising a compound of Chemical Formula [4-1], [4-2], or [4-3]:

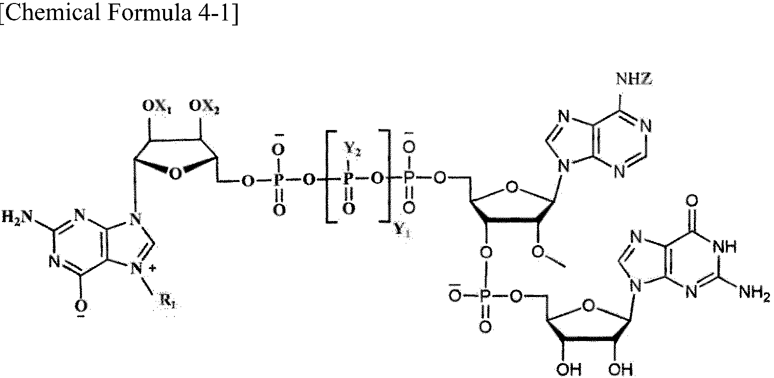

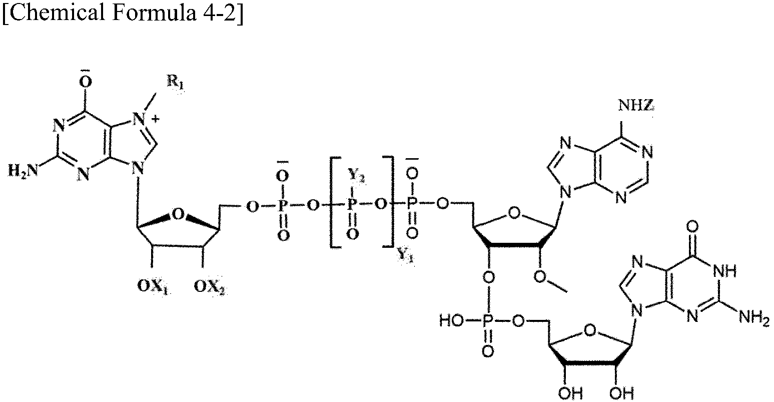

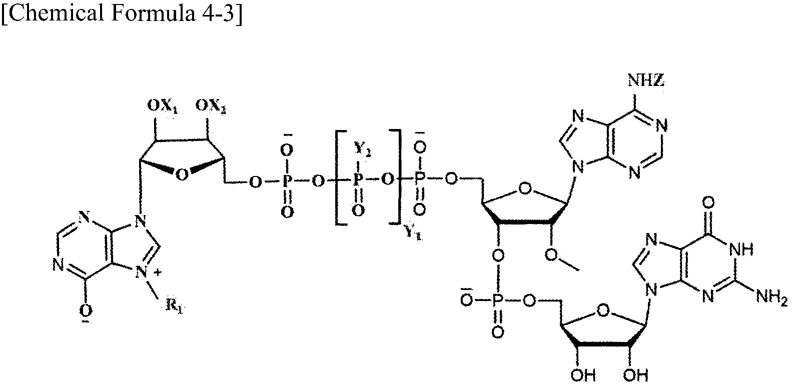

In the compound of Chemical Formula [4-1], [4-2], or [4-3],
R₁ is a compound comprising a compound of Chemical Formula 5 linked via methyl (CH₂) to the N7 position,
X₁ and X₂ are each H or CH₃,
Y is 0 or 1,
Y₂ is O or S, and
Z is H or C₁-C₃ alkylbenzyl,

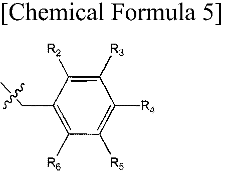

In Chemical Formula 5, R₂, R₃, R₄, R₅, and R₆ are each independently any one substituent selected from the group consisting of H, OH, halo, methyl, alkyl, alkoxy, nitro, carboxylic, azide, amino, and cyano.

R₁ may be benzyl or chlorobenzyl, but is not limited thereto.

In addition, Z may be methylbenzyl.

The substituents used to define the compounds according to the present invention are described in more detail as follows.

In the present invention, the term "halo" or "halogen atom" is used interchangeably and means chloro, fluoro, bromo, or iodo.

In the present invention, the term "alkyl" means an aliphatic saturated hydrocarbon group having 1 to 5 carbon atoms and being straight-chain, branched-chain, or cyclic. Specific examples of such alkyl groups may include methyl, ethyl, n-propyl, isopropyl, and cyclopropyl.

In the present invention, the term "aryl" means a monocyclic aromatic hydrocarbon group having six carbon atoms. A specific example of such an aryl group may include phenyl.

The present invention provides a trinucleotide cap analog comprising a compound of Chemical Formula 1:

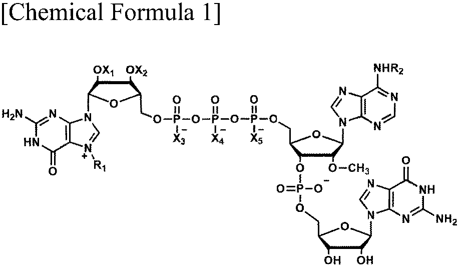

In Chemical Formula 1, R₁ is a heterocyclic group linked via CH₂ (a methylene bridge) to the N7 position, and specifically, R₁ provides a trinucleotide cap analog (ThioCap1) which is methylene bridged thiophene and a trinucleotide cap analog (PyCapl) which is methylene bridged pydidine.

The trinucleotide cap analog according to the present invention may be characterized in that it is the compound of Chemical Formula 2 or Chemical Formula 3.

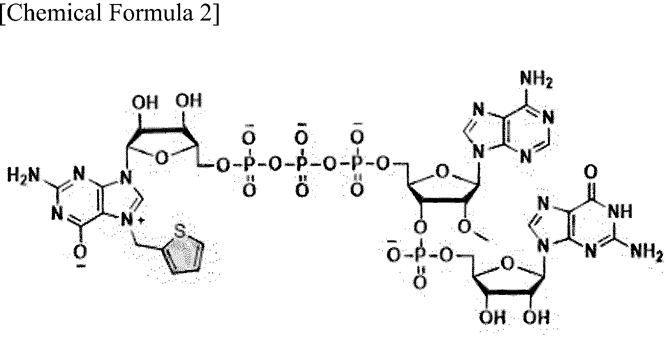

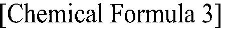

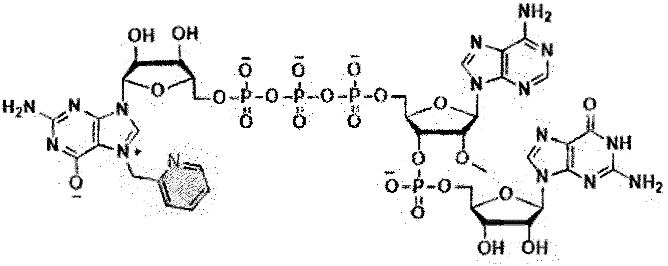

The trinucleotide cap analog according to the present invention may be Thio-Cap1 (Thio-Cap1, ^{2-mTHP7}GpppAₘpG) or Py-Capl (Py-Capl, ^{2-mPy7}GpppAₘpG).

The present invention provides a trinucleotide cap analog comprising a compound of Chemical Formula [4-1], [4-2], or [4-3]:

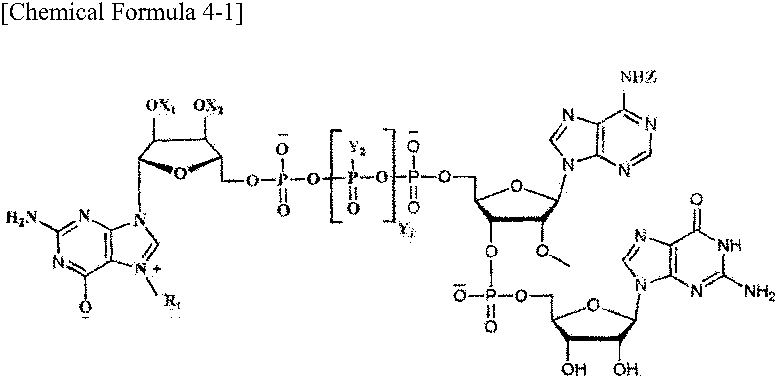

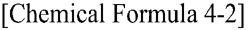

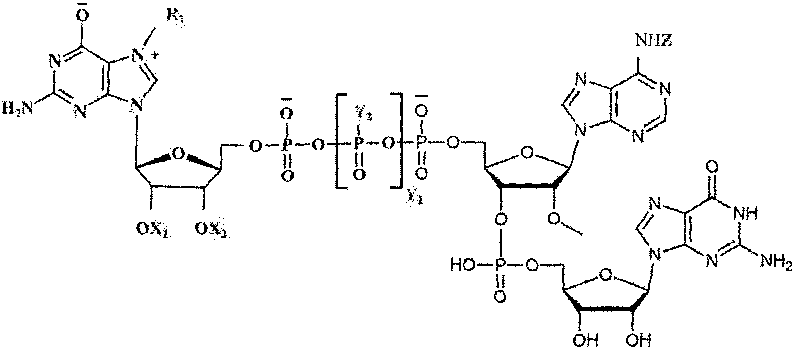

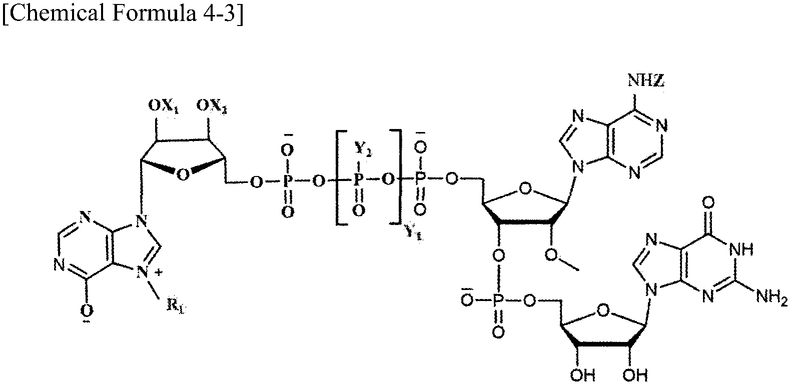

In the above Chemical Formulae, R₁ is Chemical Formula 6, which is linked via CH₂ (methyl) to the N7 position,

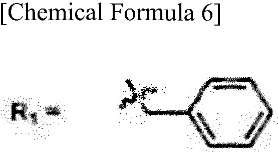

In the above formula,
R1 provides a trinucleotide cap analog which is arylmethyl (specifically benzyl).

In addition, in the above Chemical Formulae, R₁ is Chemical Formula 7, which is linked via CH₂ (methyl) to the N7 position,

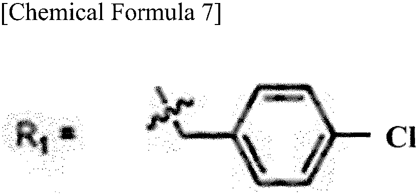

In the above formula,
R1 provides a trinucleotide cap analog which is arylmethyl (specifically Clorobenzyl).

The trinucleotide cap analog according to the present invention may be benzyl-Capl (Bz-Cap1, ^{Bn7}GpppAₘpG), chlorobenzyl-Capl (ClBz-Capl, ^{4-ClBn7}GpppAₘpG), L-benzyl-Cap1 (LBz-Capl, ^{Bn7}LGpppAₘpG), dibenzyl-Capl (DiBz-Cap1, ^{Bn7}Gppp^{Bn6}AₘpG), inosine-Cap1 (Ino-Cap1, ^{Bn7}IpppAₘpG), benzyl diphosphate-Cap1 (Bzdipho-Cap1, ^{Bn7}GppAₘpG), chlorobenzyl diphosphate-Cap1 (ClBzdipho-Cap1, ^{4-ClBn7}GppAₘpG), benzyl thiophosphate1-Cap1 (Bzthiopho1-Cap1, ^{Bn7}GppspAₘpG (D1)), benzyl thiophosphate2-Cap1 (Bzthiopho2-Cap1, ^{Bn7}GppspAₘpG (D2)), chlorobenzyl thiophosphatel-Capl (ClBzthiophol-Capl, ^{4-ClBn7}GppₛpAₘpG (D1)), and chlorobenzyl diphosphate2-Capl (ClBzthiopho2-Cap1, ^{4-ClBn7}GppₛpAₘpG (D2)).

Specifically, the cap analog may be the compounds themselves of Chemical Formula 1 and Chemical Formulae [4-1] to [4-3], and in addition to the compounds of Chemical Formula 1 and Chemical Formulae [4-1] to [4-3], may further include a hybridization sequence that can be complementary to a sequence on a DNA template at an initiation site. The length of the hybridization sequence of the cap analog for use in the methods and compositions provided herein depends on several factors including the nature of the template nucleotide sequence and the temperature at which such a primer is hybridized to a DNA template and used during in vitro transcription. The desired length of the hybridization nucleotide sequence of the cap analog for use in transcription can be readily determined by a person of ordinary skill in the art by routine experimentation. For example, the length of the hybridization nucleotide may be determined based on the desired hybridization specificity or selectivity.

By using the cap analogs described above, 5'-capped mRNA can be prepared.

One aspect of the present invention provides a method for producing a 5'-capped mRNA, comprising including, during synthesis of mRNA, a cap analog comprising the compounds of Chemical Formula 1 and Chemical Formulae [4-1] to [4-3].

In addition, one aspect of the present invention provides a composition for producing a 5'-capped mRNA, comprising a cap analog comprising the compounds of Chemical Formula 1 and Chemical Formulae [4-1] to [4-3].

In addition, one aspect of the present invention provides a use of a cap analog comprising the compounds of Chemical Formula 1 and Chemical Formulae [4-1] to [4-3] for synthesis of mRNA.

**In** addition, one aspect of the present invention provides a 5'-capped mRNA capped with a cap analog comprising the compounds of Chemical Formula 1 and Chemical Formulae [4-1] to [4-3].

In addition, a method for increasing protein expression efficiency using the cap analog, and a method for increasing protein expression efficiency using a 5'-capped mRNA construct prepared using the cap analog, are provided. In this case, the protein may be an antigen protein.

The method of producing a 5'-capped mRNA using a cap analog comprising the compounds of Chemical Formula 1 and Chemical Formulae [4-1] to [4-3] can be carried out according to any method known in the art.

**In** addition, one aspect of the present invention is that the 5'-capped mRNA can be produced by a co-transcriptional capping method, which is an mRNA synthesis method in which a chemically synthesized cap analog is introduced simultaneously during in vitro mRNA synthesis so that the cap analog constitutes the 5' terminus. Specifically, the method may comprise a step of introducing a cap analog, which is the compounds of Chemical Formula 1 and Chemical Formulae [4-1] to [4-3], into a mixture comprising an RNA polymerase under conditions in which transcription of a polynucleotide template by the RNA polymerase occurs, and a step of incubating the mixture for a time sufficient to allow transcription of the template.

The present invention provides a pharmaceutical composition for expression of a desired peptide or protein, comprising an mRNA capped at the 5' terminus with a trinucleotide cap analog and a pharmaceutically acceptable carrier.

Depending on the type of desired peptide or protein, a therapeutic or preventive effect on a desired disease can be obtained in vivo. Therefore, it can be used for treatment or prevention of any disease that can be treated or prevented by expression of a peptide or protein. Diseases that can be treated or prevented by expression of specific types of peptides or proteins are known, and by using the pharmaceutical composition, expression of the peptides or proteins can be induced and used for prevention or treatment of a desired disease.

In addition, one aspect of the present invention provides a medical use of a 5'-capped mRNA capped with the cap analog for prevention or treatment of any disease in which in vivo expression of a peptide or protein is effective.

The pharmaceutical composition and the treatment or prevention method can be used in gene replacement therapy, genome editing, cancer immunotherapy, or treatment or prevention using vaccines. In one specific example, the pharmaceutical composition is an mRNA vaccine.

The pharmaceutical composition may be formulated for administration by injection, or by another appropriate route known to a person skilled in the relevant art for treatment or prevention of a particular pathological condition. An injectable composition includes, as a pharmaceutically acceptable carrier, for example, sterile physiological saline. The injectable composition may also be formulated as a suspension in lipids or phospholipids, a liposome suspension, or an aqueous emulsion. Methods for formulating the pharmaceutical composition are well known to those of ordinary skill in the art.

In one specific example, the pharmaceutical composition may contain mRNA comprising the cap analog as an active ingredient at a concentration of about 0.01% to 1%. The concentration may vary depending on administration frequency, administration dose, administration method, and the like.

In one specific example, the pharmaceutical composition can be administered to mammals, specifically humans, and the dose may vary depending on the health condition of the subject, severity of the disease, body weight, age, race, and the like, and an appropriate dose can be determined by a person skilled in the art. In one specific example, the dose for humans is in the range of 0.0001 to 100 mg/day, and more specifically in the range of about 0.1 to 50 mg/day.

The 5'-capped mRNA capped with the cap analog according to the above aspect can be introduced into cells in vivo or in vitro to express a protein or peptide.

Methods of expressing a protein or peptide in cells in vivo or ex vivo using the mRNA are known in the art, and according to such conventional methods, a protein or peptide can appropriately be expressed in cells.

Hereinafter, examples are presented to aid in understanding of the present invention. The following examples are merely illustrative of the present invention, and it will be apparent to those skilled in the art that various changes and modifications can be made within the scope and spirit of the present invention, and it is of course that such changes and modifications are encompassed within the appended claims.

### MODE FOR CARRYING OUT THE INVENTION

### <Example>

### A. N7-methylene bridged heterocyclic compound-substituted CleanCap analogs

### <Example 1> Synthesis of Trinucleotide Capping Substances

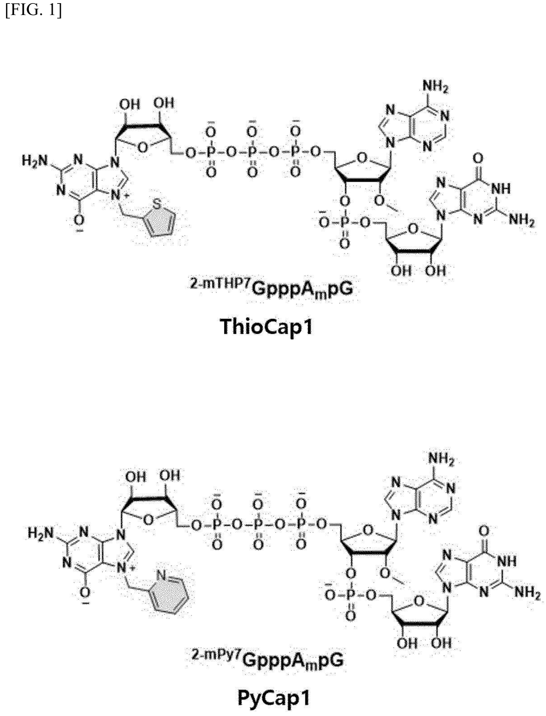

The present example relates to the synthesis of newly modified trinucleotide capping substances and the use thereof as an mRNA vaccine. Specifically, among the trinucleotide structure of CleanCap, new compounds in which the N7 position of the 5'-terminal guanosine base is modified not with a conventional methyl group but with a methylene-bridged heterocyclic compound-more specifically, methylene-bridged thiophene (ThioCapl) or methylene-bridged pyridine (PyCap1)-were synthesized through organic chemical reactions (FIG. 1).

### <1-1> Reagent Information

¹H, ¹³C, and ³¹P NMR spectra were recorded using a Bruker AV-400 spectrometer; the solvents used were D₂O or DMSO-d6, and tetramethylsilane was used as the internal standard. UV-Vis spectra were recorded at room temperature using a Cary series UV-Vis spectrophotometer (Agilent Technologies) and 1cm quartz cuvettes, and absorbance changes were measured immediately after UV irradiation of the sample solution in the cuvette. Fluorescence emission spectra were recorded at room temperature using a PF-65000 spectrofluorometer.

### <1-2> Method for Synthesizing N7-(heteroaryl-2-ylmethyl) Guanosine 5'-diphosphoroimidazolide Analogs

### ① Synthesis of the Triethylammonium Salt of Guanosine 5'-diphosphate

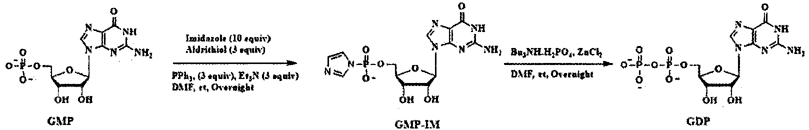

To synthesize the triethylammonium salt of guanosine 5'-diphosphate (GDP·TEA), guanosine 5'-phosphate (GMP·TEA; 930 mg, 2 mmol) was dissolved in anhydrous dimethyl sulfoxide (4 mL) under stirring, followed by addition of triethylamine (1.7 mL, 12 mmol), imidazole (817 mg, 12 mmol), and 2,2'-dithiodipyridine (1.3 g, 6 mmol). The mixture was stirred for 5 minutes, after which triphenylphosphine (1.56 g, 6 mmol) dissolved in anhydrous dimethyl sulfoxide (4 mL) was added, and stirring was continued at room temperature for 5 hours. After completion of the reaction, the mixture was slowly poured into a mixture of acetone (50 mL) and sodium perchlorate (0.5 g). After cooling at 4 °C for 30 minutes, the mixture was centrifuged at 5,000 rpm for 8 minutes and the supernatant was discarded. To remove small amounts of imidazole and triphenylphosphine, the solid was triturated with fresh acetone (20 mL), cooled, and centrifuged again. This process was repeated once more, and the precipitate was dried at room temperature in a vacuum oven.

The resulting guanosine 5'-phosphoroimidazolide (NaGMP-IM) was dissolved in dimethylformamide (10 mL) and added dropwise into a mixture consisting of a 1 M tributylammonium orthophosphate solution in dimethylformamide (5 mL) that had been stirred for 30 minutes. Finally, zinc chloride (200 mg, 1.5 mmol) was added, and the reaction mixture was stirred overnight at room temperature. The reaction mixture was extracted with chloroform (3 × 20 mL) after diluting with water (50 mL). The resulting aqueous layer was concentrated using a rotary evaporator at 37 °C and then purified by applying it to an anion-exchange resin.

Chromatographic purification was performed using DEAE (diethylaminoethyl weak anion exchange) Sepharose fast-flow resin. The desired compound was eluted using a 0-30% gradient of 1 M TEAB (triethylammonium bicarbonate) buffer, pH 7.5, at a flow rate of 3 mL/min for four bed volumes on a CombiFlash EZ Prep. Fractions containing GDP·TEA were pooled, concentrated using a rotary evaporator (bath at 37 °C), and lyophilized to yield a fine white powder (951 mg, 64%). NMR results for GDP·TEA are shown below:

### GDP·TEA (951 mg, 64%)

¹H NMR (400 MHz, D2O) δ = 7.99 (s, 1H), 5.81 (d, J = 6.0 Hz, 1H), 4.63 (t, J = 5.2 Hz, 1H), 4.51 (t, J = 5.6 Hz, 1H), 4.22 (m, 1H), 4.14 (m, 1H), 4.05 (m, 1H). ³¹P NMR (162 MHz, D₂O) δ = -6.47 (d, J = 23.5 Hz, 1P), -10.99 (d, J = 22.5 Hz, 1P).

### ② General Synthetic Process of N7-(heteroaryl-2-ylmethyl) Guanosine 5'-diphosphate Analogs

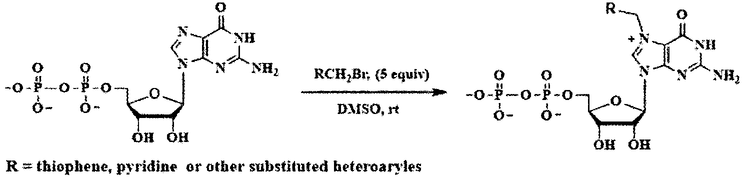

**A** triethylammonium salt of guanosine 5'-diphosphate (0.5 mmol, 1 equiv.) was dispersed in DMSO (3 mL), and an appropriate 2-bromomethyl heteroaryl compound (2.5 mmol, 5 equiv.) was added. The mixture was stirred overnight at room temperature. After completion of the reaction, the mixture was diluted with water (20 mL) and extracted with diethyl ether (3 × 10 mL). After completion of the reaction, the mixture was slowly poured into a mixture prepared by combining acetone (50 mL) and sodium perchlorate (0.5 g). After cooling at 4 °C for 30 minutes, the mixture was centrifuged at 5,000 rpm for 8 minutes and the supernatant was discarded. The solid was triturated with fresh acetone (20 mL), the mixture was cooled, and centrifuged again. This process was repeated once more, and the precipitate was vacuum-dried at room temperature. This process was repeated once more, and the precipitate was vacuum-dried at room temperature. The crude product solution (total volume 15-20 mL) was purified using Sepharose fast-flow resin as described above. Fractions containing the product were combined, concentrated using a rotary evaporator (bath temperature 37 °C), azeotropically evaporated with ethanol three times, and dried using a freeze dryer.

According to the above method, N7-(thiophen-2-ylmethyl) guanosine 5'-diphosphate triethylammonium salt and N7-(pyridin-2-ylmethyl) guanosine 5'-diphosphate triethylammonium salt were synthesized respectively by using 2-bromomethyl thiophene and 2-bromomethyl pyridine, and the NMR results are shown below.

### N7-(thiophen-2-ylmethyl) guanosine 5'-diphosphate triethylammonium salt: N7-2-mTHP-GDP.TEA (155 mg, 42 %).

¹H NMR (400 MHz, D₂O) δ = 7.36 (d, J = 5.2 Hz, 1H), 7.27 (d, J = 3.6 Hz, 1H), 6.96 (t, J = 5.2 Hz,1H), 5.94 (d, J = 3.6 Hz, 1H), 5.80 (dd, J1 = 18.0 Hz, J2 = 15.2 Hz, 2H), 4.62 (t, J = 3.6 Hz,1H), 4.48 (t, J = 5.6 Hz, 1H), 4.32-4.27 (m, 1H), 4.26-4.14 (m, 2H); ³¹P NMR (162 MHz, D₂O) δ = -8.34 (d, J = 22.4 Hz, 1P), -11.10 (d, J = 22.5 Hz, 1P).

### N7-(pyridin-2-ylmethyl) guanosine 5'-diphosphate triethylammonium salt: N7-2mPy-GDP.TEA (213 mg, 58 %)

¹H NMR (400 MHz, D₂O) δ = 8.35 (d, J = 5.6 Hz, 1H), 7.70 (td, J1 = 8 Hz, J2 = 2 Hz, 1H), 7.35-7.29 (m, 2H), 6.04 (d, J = 3.2 Hz, 1H), 5.75 (d, J = 5.2 Hz, 2H), 4.64 (dd, J1 = 4.8 Hz, J2 = 3.2 Hz, 1H), 4.55 (dd, J1 = 6.0 Hz, J2 = 4.8 Hz, 1H), 4.36 (dd, J1 = 6.0 Hz, J2 = 2.4 Hz, 1H), 4.22 (dd, J1 = 5.2 Hz, J2 = 2.4 Hz, 1H); ³¹P NMR (162 MHz, D₂O) δ = -6.34 (d, J = 22.5 Hz, 1P), -10.95 (d, J = 23.5 Hz, 1P).

### ③ General Synthetic Process of N7-(heteroaryl-2-ylmethyl) Guanosine 5'-diphosphoroimidazolide Analogs

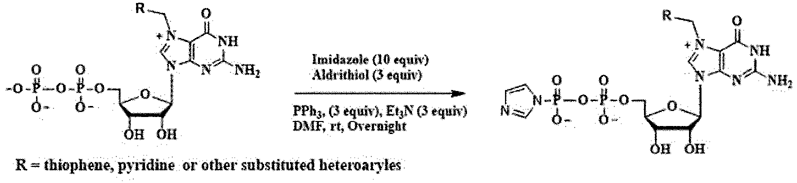

A solution prepared by stirring the triethylammonium salt of N7-(heteroaryl-2-ylmethyl) guanosine 5'-diphosphate (0.3 mmol) in anhydrous dimethyl sulfoxide (3 mL) was added with triethylamine (251 µL, 1.8 mmol), imidazole (123 mg, 1.8 mmol), and 2,2'-dithiopyridine (198 mg, 0.9 mmol), and the mixture was stirred for 5 minutes. Then, triphenylphosphine (236 mg, 0.9 mmol) dissolved in anhydrous dimethyl sulfoxide (2 mL) was added, and stirring was continued for 5 hours at room temperature. After completion of the reaction, the mixture was slowly poured into a mixture of acetone (25 mL) and sodium perchlorate (250 mg). After cooling at 4°C for 30 minutes, the mixture was centrifuged and the supernatant was discarded. In order to remove trace amounts of imidazole and triphenylphosphine, the solid was triturated with fresh acetone (10 mL), the mixture was cooled, and centrifuged again. This process was repeated once more, and the precipitate was dried in a vacuum oven at room temperature using P2O5.

According to the above method, N7-(thiophen-2-ylmethyl) guanosine 5'-diphosphate and N7-(pyridine-2-ylmethyl) guanosine 5'-diphosphate were respectively used to synthesize N⁷-(thiophen-2-ylmethyl) guanosine 5'-diphosphoroimidazolide and N⁷-(pyridin-2-ylmethyl) guanosine 5'-diphosphoroimidazolide, and their NMR results are shown below.

### <N7-(thiophen-2-ylmethyl) guanosine 5'-diphosphoroimidazolide: N7-2-mTHP-GDP-IM>

¹H NMR (400 MHz, D₂O) δ = 7.99 (s, 1H), 7.53 (dd, J1 = 5.8 Hz, J2 = 1.6 Hz, 1H), 7.40 (dd, J1 = 3.2 Hz, J2 = 1.2 Hz, 1H), 7.36 (dd, J = 1.6 Hz, 1H), 7.12 (dd, J1 = 5.2 Hz, J2 = 3.6 Hz, 1H), 7.06 (bs, 1H), 6.03 (d, J = 4.4 Hz, 1H), 5.91 (d, J = 4.4 Hz, 2H), 4.69 (t, J = 4.0 Hz, 1H), 4.45-4.38 (m, 2H), 4.30 (m, 1H), 4.17 (m, 1H); ³¹P NMR (162 MHz, D₂O) δ = - 11.70 (d, J = 24.5 Hz, 1P), -19.90 (d, J = 21.5 Hz, 1P).

### <N7-(pyridin-2-ylmethyl) guanosine 5'-diphosphoroimidazolide: N7-2mPy-GDP-IM>

¹H NMR (400 MHz, D₂O) δ = 8.35 (d, J = 7.6 Hz, 1H), 7.83-7.77 (m, 2H), 7.33 (d, J = 8.0 Hz, 2H), 7.18 (bs, 1H), 6.88 (bs, 1H), 6.02 (d, J = 4.0 Hz, 1H), 5.69 (dd, J1 = 22.0 Hz, J2 = 15.6 Hz, 2H), 4.60 (t, J = 4.8 Hz, 1H), 4.35 (t, J = 5.6 Hz, 1H), 4.32-4.27 (m, 1H), 4.17 (m, 1H), 4.04 (m, 1H); ³¹P NMR (162 MHz, D₂O) δ = -11.84 (d, J = 20.6 Hz, 1P), -19.92 (d, J = 21.5 Hz, 1P).

### <1-3> Method for Synthesizing the Dinucleotide pAmpG

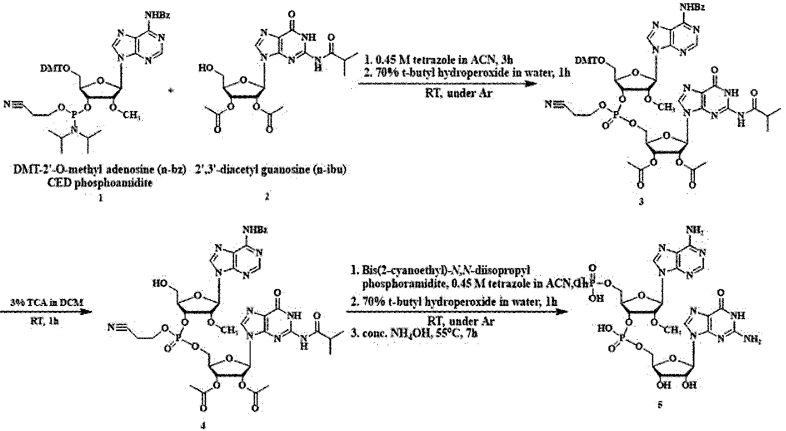

### ① Synthetic Process of 5'-O-DMT-2'-O-methyl Adenosylyl (n-bz)-{3'-OP-[2-cyanoethyl] → 5'}-2',3'-diacetyl Guanosine (n-ibu) 3

DMT-2'-O-methyl adenosine (n-bz) CED phosphoramidite 1 (1.29 g, 1.45 mmol) and 2',3'-diacetyl guanosine (n-ibu) 2 (0.58 g, 1.32 mmol) were dried under high vacuum for 16 hours. To acetonitrile (7.3 mL, 3.30 mmol) were added anhydrous DMF (1.7 mL) and 0.45 M tetrazole, and the mixture was stirred at room temperature for 3 hours under an argon (nitrogen) atmosphere. After confirming consumption of compound 1 by TLC, the mixture was cooled in an ice-salt bath for 30 minutes, after which 70% t-butyl hydroperoxide (0.91 mL, 6.60 mmol) in water was added. The reaction mixture was stirred at room temperature for 1 hour. After confirming conversion of the intermediate by TLC, the mixture was extracted with ethyl acetate (2 × 200 mL) and washed with 5% NaHCO₃ solution (2 × 100 mL). The extracted organic layer was combined, dried over sodium sulfate, evaporated, and crude solid 3 (1.77 g) was obtained.

### ② Synthetic Process of 2'-O-Methyl Adenosylyl (n-bz)-{3'-OP-[2-cyanoethyl] → 5'}-2',3'-diacetyl Guanosine (n-ibu) 4

Crude dinucleotide 3 solid (1.77 g) was dissolved in 3% trichloroacetic acid in dichloromethane (31.2 mL, 5.72 mmol). The mixture was stirred at room temperature for 1 hour. After confirming conversion of compound 3 by TLC, 5% NaHCO₃ solution (30 mL) was added and the mixture was stirred for 10 minutes. The mixture was extracted with dichloromethane and evaporated. The resulting residue was purified by silica gel column chromatography to give product 4 (0.74 g, 0.79 mmol, 60%).

¹H NMR (DMSO, 400MHz): δ 12.12 (s, 1H), 11.59 (d, 1H), 11.26 (d, 1H), 7.93 (d, d, 2H), 8.28 (d, 1H), 8.06 (d, 2H), 7.66 (t, 1H), 7.57 (d, 2H), 6.19(d, 1H), 6.14 (dd, 1H), 5.84 (q, 1H), 5.54 (dd, 1H), 5. 39 (t, 1H), 5.20 (m, 1H), 4.83 (m, 1H), 4.48 (m, 3H), 4.30 (m, 3H), 3.64 (m, 2H), 3.37 (d, 3H), 2.97 (q, 2H), 2.78 (m, 1H), 2.15 (d, 3H), 2.03 (d, 3H), 1.13 (m, 6H); ³¹P NMR (DMSO, 162 MHz): δ -2.43 (d, 1P);

### ③ Synthetic Process of 5'-O-Phosphoryl-2'-O-methyl Adenosylyl-{3'-OP → 5'}-Guanosine (pAmpG) 5

Dinucleotide 4 (0.74 g, 0.7891 mmol) was dried under high vacuum for 16 hours. To the mixture were added 0.45 M tetrazole in acetonitrile (3.51 mL, 1.58 mmol) and bis-(2-cyanoethyl)-N,N-diisopropyl-phosphoramidite (0.41 mL, 1.58 mmol), and the mixture was stirred for 3 hours at room temperature under an argon (nitrogen) atmosphere. After confirming consumption of compound 4, the reaction mixture was cooled in an ice-salt bath for 30 minutes, and 70% t-butyl hydroperoxide in water (17.2 mL, 3.16 mmol) was added. The mixture was stirred at room temperature for 1 hour. After confirming conversion of the intermediate by TLC, the mixture was extracted with ethyl acetate (2 × 200 mL) and washed with 5% NaHCO₃ solution (2 × 100 mL). The extracted organic layers were combined and evaporated. The resulting residue was dissolved in methanol (25 mL) and concentrated ammonia solution (25 mL), and the mixture was stirred at 55 °C for 7 hours. After completion of deprotection, the reaction mixture was evaporated with methanol. The resulting residue was dissolved in water, adjusted to pH 5.5, and loaded onto a DEAE-Sepharose column. Solvent A: 1 M TEAB buffer (pH 7.5-8), Solvent B: water. Fractions containing the product were collected, evaporated, and dried in a freeze dryer to obtain compound 5 as a fine white powder containing a triethylammonium salt (446 mg, 0.4908 mmol, 62%).

### Dinucleotide (pAmpG) TEA salt

¹H NMR (D₂O, 400 MHz): δ 8.53 (s, 1H), 8.10 (s, 1H), 7.89 (s, 1H), 6.06 (d, 1H), 5.77 (d, 1H), 4.88 (m, 1H), 4.50 (t, 1H), 4.42 (m, 2H), 4.27 (m, 1H), 4.12 (t, 2H), 3.92 (m, 2H), 3.37 (s, 3H); ³¹P NMR (D₂O, 162 MHz): δ 3.69 (s, 1P), -0.84 (s, 1P)

### <1-4> General Synthetic Procedure of Trinucleotide-Based Capping Materials Containing an N7-methylene Bridged Heterocyclic Group (ThioCap1 and PyCap1)

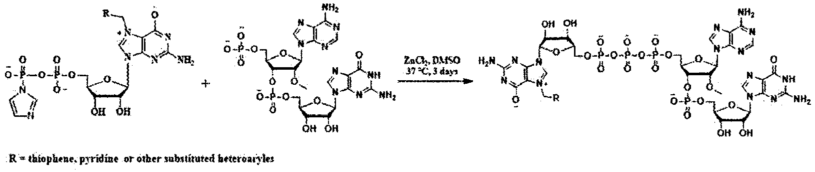

**A** solution containing pAmpG triethylammonium salt (0.07 mmol, 63 mg) in DMSO (50 mM) was added with N7-(heteroaryl-2-ylmethyl) guanosine 5'-diphosphoroimidazolide disodium salt (3.00 equivalents, 0.2 mmol) and zinc chloride (20.0 equivalents). After stirring at 37 °C for 3 days, a 500 mM EDTA aqueous solution (pH 8.0) (1.30 equivalents per mole of ZnCl₂) was added to terminate the reaction mixture. The mixture was diluted with water and purified using DEAE Sepharose fast-flow resin, followed by further purification by reversedphase HPLC (Shimadzu instrument; YMC-Actus Triart C8 column (preparative, 250 × 20.0 mm I.D.); solvent A, 50 mM TEAA buffer (pH 6.0) containing 0.5% CH₃CN; solvent B, CH₃CN; linear gradient 5-80% B (25 min); flow rate, 10 mL/min; detection wavelength, 254 nm). Fractions containing the desired compound were collected, concentrated, and lyophilized to obtain the desired N7-(heteroaryl-2-ylmethyl) Cap1 analog as a triethylammonium salt. The product was redissolved in methanol (2.00 mL). To the mixture, 190 mM NaClO₄ in acetone (24.0 mL) was added, and the resulting suspension was centrifuged (4,000 rpm, 20 min). The supernatant was discarded, and the precipitate was resuspended in acetone. The suspensioncentrifugation steps were repeated an additional 3-4 times. The precipitate was dried under reduced pressure to obtain the target N7-(heteroaryl-2-ylmethyl) Cap1 analog as a sodium salt.

According to the above method, using N7-(thiophen-2-ylmethyl) guanosine 5'-diphosphoroimidazolide and N7-(pyridin-2-ylmethyl) guanosine 5'-diphosphoroimidazolide, respectively, trinucleotide-based capping materials containing an N7-methylene bridged heterocyclic group (ThioCapl (^{2-mTHP7}GpppAmpG) and PyCapl (^{2-mPy7}GpppAmpG)) were synthesized, and their NMR analytical results are shown below.

### <Guanosine N7-(thiophen-2-ylmethyl) trinucleotide Cap1 analog>

### <^{2-mTHP7}GpppAmpG>

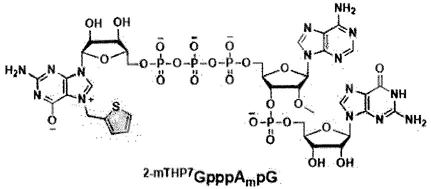

¹H NMR (400 MHz, D₂O) δ = 9.29 (s, 1H), 8.34 (s, 1H), 8.11 (s, 1H), 7.91 (s, 1H), 7.29 (d, J = 5.2 Hz, 1H), 7.21 (d, J = 3.6 Hz, 1H), 6.88 (dd, J1 = 5.2 Hz, J2 = 3.6 Hz, 1H), 5.90 (d, J = 6.0 Hz, 1H), 5.81 (d, J = 4.4 Hz, 1H), 5.78 (d, J = 6.0 Hz, 1H), 5.70 (s, 2H), 4.88 (m, 1H), 4.76 (t, J = 5.2 Hz, 1H), 4.58 (t, J = 4.4 Hz, 1H), 4.48-4.40 (m, 3H), 4.39-4.28 (m, 4H), 4.25-4.12 (m, 5H), 3.36 (s, 3H); ³¹P NMR (162 MHz, D₂O) δ = -0.84 (s, 1P), -11.30 (d, J = 19.6 Hz, 1P), -11.51 (d, J = 18.6 Hz, 1P), -22.93 (t, J = 19.6 Hz, 1P).

### <Guanosine N7-(pyridin-2-ylmethyl) trinucleotide Cap1 analog>

### <^{2-mPy7}GpppAmpG>

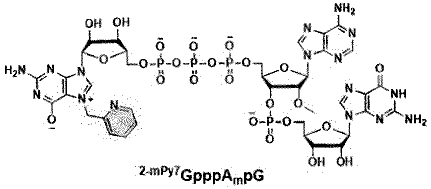

¹H NMR (400 MHz, D₂O) δ = 8.38 (s, 1H), 8.31 (d, J = 4.8 Hz, 1H), 8.13 (s, 1H), 7.90 (s, 1H), 7.77 (t, J = 7.6 Hz, 1H), 7.40 (d, J = 8.0 Hz, 1H), 7.28 (t, J = 5.6 Hz, 1H), 5.95 (d, J = 6.0 Hz, 1H), 5.93 (d, J = 4.0 Hz, 1H), 5.78 (d, J = 6.0 Hz, 1H), 5.72 (s, 2H), 4.88 (m, 1H), 4.80 (t, J = 5.6 Hz, 1H), 4.67 (t, J = 3.6 Hz,1H), 4.50 (t, J = 4.8 Hz,1H), 4.47-4.42 (m, 2H), 4.41-4.28 (m, 4H), 4.27-4.13 (m, 5H), 3.36 (s, 3H); ³¹P NMR (162 MHz, D₂O) δ = -0.82 (s, 1P), - 11.29 (d, J = 19.6 Hz, 1P), -11.50 (d, J = 18.6 Hz, 1P), -22.81 (t, J = 18.6 Hz, 1P).

### <Example 2> Introduction of Trinucleotide Capping into mRNA and Analysis of Capping Efficiency

It was analyzed whether the trinucleotide capping materials prepared in Example 1 can be recognized with high efficiency by T7 RNA polymerase during the transcription process and introduced into mRNA.

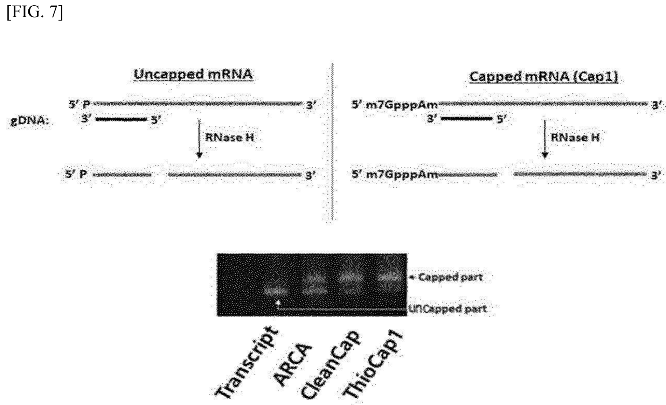

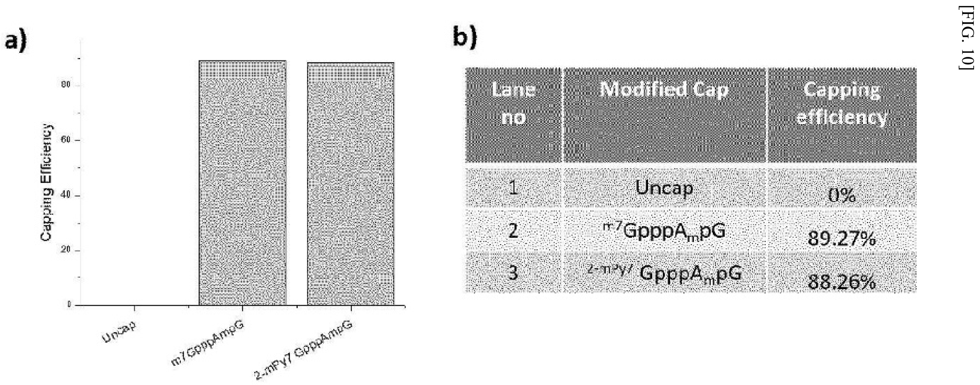

As a result, the transcription efficiency of the capping materials appeared to be similar between CleanCap (cap 1 commercial) and ThioCapl, and it was confirmed that, during the transcription process, the capping efficiency of the developed ThioCapl can be recognized by T7 RNA polymerase with higher efficiency than ARCA or CleanCap (cap 1 commercial) and introduced into RNA. In the case of conventional ARCA, a capping efficiency of about 50% is observed, and in the case of CleanCap (cap 1 commercial), a capping efficiency of about 86% is observed; however, it was confirmed that ThioCap1 exhibits a relatively higher capping efficiency of about 92% or more, and in the case of PyCapl, a capping efficiency of about 88% or more was observed (FIGS. 7 and 10). In the case of ARCA, due to the very low capping efficiency, the expression efficiency of the antigen protein (Luciferase) was not comparatively measured.

### <Example 3> Analysis of Antigen Protein Expression Using Capping Materials Substituted with a Methylene-Bridged Heterocyclic Group

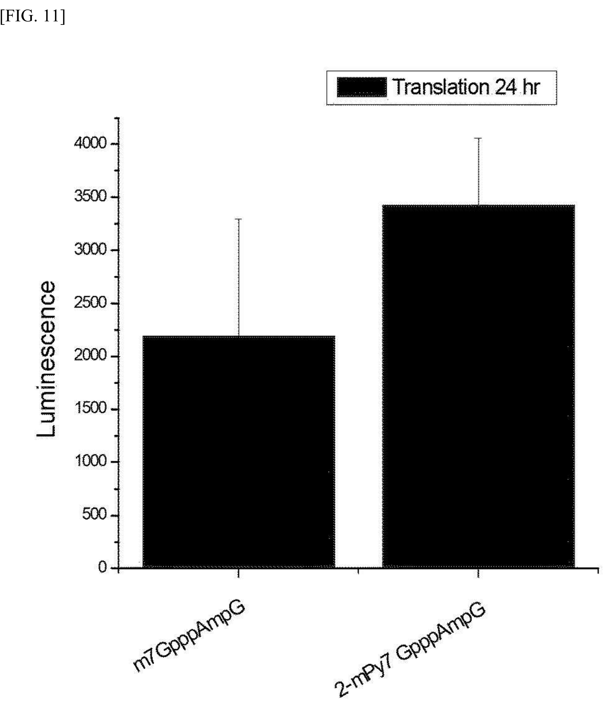

The translation process of mRNA into which a capping material prepared by modifying the N7 position of the guanosine base at the 5' terminus into a methylene-bridged thiophene compound instead of the conventional methyl was introduced was confirmed. To confirm the expression level of the antigen protein, Luciferase mRNA was used, and the expression amount of the antigen protein was analyzed using HEK 293 cells. In the case of Luciferase mRNA, two types were compared: mRNA using natural rNTPs and mRNA into which pseudouridine triphosphate, used to avoid excessive immune activation instead of rUTP, had been introduced. In both cases, the mRNA into which ThioCapl had been introduced (Luc mRNA ThioCAP1) exhibited more efficient antigen protein expression than the mRNA into which CleanCap had been introduced (Luc mRNA CAP1), and the mRNA into which PyCapl had been introduced (Luc mRNA PyCAP1) likewise exhibited more efficient antigen protein expression than the mRNA into which CleanCap had been introduced (Luc mRNA CAP1) (FIGS. 8 and 11).

### ① Preparation of Luciferase DNA Template

A linear dsDNA template including the start codon up to the poly-A tail was prepared from a luciferase plasmid by PCR. During successive PCR steps, the T7 promoter and Kozak sequence were added to the luciferase dsDNA template, and this was ultimately used for the transcription reaction. The primers used were: First forward primer: 5'-ATG GAA GAC GCC AAA AAC ATA AAG-3' (Sequence No. 1), Second forward primer: TAA TAC GAC TCA CTA TAG GGC CAC CAT GGA AGA CGC CAA AAA CAT (Sequence No. 2), Reverse primer: AAT CGC GCC TAG GCG CGC CCG TAC GGC TCT TC-3' (Sequence No. 3).

### ② Preparation of Luciferase mRNA

The concentration of the luciferase template was measured using a nanodrop spectrophotometer. For each transcription reaction, 100 ng of template in 1 µL was placed into a microcentrifuge tube. Then, 1.5 µL of 10X transcription buffer (400 mM Tris-HCl, 60 mM MgCl₂, 10 mM DTT, 20 mM spermidine) and 1.5 µL of 100 mM DTT were added to the tube. rATP, rCTP, and rUTP were each used (1 µL, 10 mM). For cap-dependent transcription of luciferase, rGTP was used (1 µL, 2.5 mM). The ratio of capping material was maintained at four times higher than rGTP so as to provide mRNA capped at ≥80%. Accordingly, 1 µL of capping material (10 mM) was added. RNase inhibitor (0.5 µL, 40 U/µL) and T7 RNA polymerase (1 µL, 50 U/µL) were added. Nuclease-free water (4.5-5.5 µL) was added to bring the final volume of the reaction to 15 µL. In the case of modified rNTPs, 10 mM stock solutions were prepared and the corresponding derivatives were added in place of rCTP and rUTP. The reaction was incubated at 37°C for 1 hour, and after 1 hour, 1.8 µL of 10X DNase I buffer (10 mM Tris-HCl, 2.5 mM MgCl₂, 0.5 mM CaCl₂) and 1 µL of DNase I (2 U/µL) were added to the reaction and incubated for an additional 15 minutes at 37°C to remove all DNA templates.

### ③ Purification of Transcribed mRNA

To remove dsRNA and transcription byproducts that may have been formed, dsRNA was separated through electrophoresis. In the next step, cellulose was added (10 µL, 0.2 g/mL) to the electrophoresis-separated mRNA and gently shaken for 10 minutes. Then, the mixture was transferred to an RNA purification column together with ethanol and the binding buffer supplied with the column, followed by centrifugation at 13,000 rpm for 1 minute. Next, RNA preparation buffer and wash buffer were sequentially added to the column and centrifuged. At the end of this step, all proteins and unincorporated rNTP were removed into the suspension. Finally, a new tube was attached, and nuclease-free water (10 µL) was added to the column, followed by centrifugation at 13,000 rpm for 2 minutes. All dsDNA bound to the cellulose remained in the column, and only the purified single-stranded luciferase mRNA passed through the column. This sample was collected and stored at 70°C for further applications.

### ④ Analysis of Capping Efficiency of Transcribed mRNA

The capped and transcribed 2 µg of mRNA was annealed by heating together with 3 µg of gDNA and RNase H buffer to 70°C and then slowly cooling to 25°C. Next, using thermally stable RNase H, the reaction was carried out at 37°C for 1 hour so as to cleave a short sequence containing the capping material from the long mRNA. The cleaved short sequence containing the capping material was analyzed by electrophoresis using a 16% denaturing PAGE gel. In uncapped mRNA, the cleaved short sequence exhibited a relatively faster gel migration, whereas in capped mRNA, the cleaved short sequence exhibited relatively slower gel migration.

### ⑤ Cell Culture and Preparation for Transfection

HEK 293 cells were cultured in a 60 mL culture plate containing DMEM supplemented with 10% FBS and 1% pen/strep. On the day prior to transfection, the cells were detached using trypsin and resuspended in new medium. The cells were seeded into a 96-well plate. On the next day, when the cells reached 70% confluence, the transfection mixture was prepared and transfection was performed.

### ⑥ Quantification and Transfection of mRNA

The purified mRNA was quantified using a nanodrop spectrophotometer, and the transcription yield for each rNTP combination was compared. For cell transfection, 500 ng of each mRNA was collected and mixed with 5 µL of Opti-MEM. Separately, 0.5 µL of Lipofectamine was mixed with 5 µL of Opti-MEM in another tube and incubated at room temperature for 10 minutes. Then, the mRNA and Lipofectamine mixtures were combined in the same tube and incubated for an additional 5 minutes at room temperature. Finally, the transfection mixture was added to the cells and incubated for 24 hours.

### ⑦ Analysis of Luciferase Expression

After the incubation period, the medium was removed, and the cells were lysed using 50 µL of 1X lysis buffer. The cell lysate was transferred into microcentrifuge tubes. For luciferase luminescence analysis, 100 µL of luciferin substrate was added to each sample in a 96-well white plate. Then, 20 µL of each cell lysate sample was added and mixed by pipetting. Finally, the luminescence of luciferase was recorded using a luminometer instrument.

As a result, it was confirmed that the capping material according to the present invention can be recognized by T7 RNA polymerase during transcription with higher efficiency than ARCA or CleanCap. Whereas conventional ARCA exhibited approximately 50% capping efficiency and CleanCap (cap 1 commercial) exhibited approximately 89% capping efficiency, ThioCapl exhibited capping efficiency of ≥90%, and PyCap1 exhibited capping efficiency of ≥88% (FIGS. 7 and 10).

Furthermore, the translation process of mRNA into which the capping material according to the present invention had been introduced was confirmed. To examine the expression level of antigen protein, Luciferase mRNA was used, and the expression amount of antigen protein was confirmed using HEK 293 cells. For Luciferase mRNA, two types were compared: mRNA using natural rNTPs and mRNA into which pseudouridine triphosphateused to avoid excessive immune activation instead of rUTP-had been introduced.

As a result, the mRNA into which pseudouridine triphosphate was introduced exhibited higher antigen protein expression efficiency than the mRNA using rNTPs.

Furthermore, compared to mRNA into which the conventional CleanCap (cap 1 commercial) was introduced (Luc mRNA Capl), the mRNA into which ThioCapl according to the present invention was introduced (Luc mRNA ThioCapl) and the mRNA into which PyCap1 was introduced (Luc mRNA PyCAP1) exhibited higher antigen protein expression efficiency in both mRNA using rNTPs and mRNA containing pseudouridine instead of rUTP (FIGS. 8 and 11).

### B. N7-Arylmethyl (benzyl (BzCap1))-Substituted CleanCap Analogs

### <Example 1> Synthesis of Trinucleotide Capping Materials

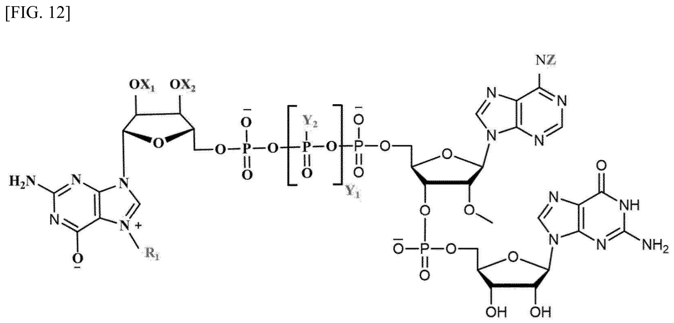

The present example relates to the synthesis of newly modified trinucleotide capping materials and their use as mRNA vaccines, and more specifically, to novel compounds in which the N7 position of the 5'-terminal guanosine base in the trinucleotide structure of CleanCap is modified with an arylmethyl group instead of the conventional methyl group, more specifically benzyl and 4-chlorobenzyl (^{Bn7}GpppAₘpG, ^{Bn7}GppAₘpG, ^{4-ClBn7}GppAₘpG, ^{Bn7}GpppBn6AₘpG, ^{4-ClBn7}GpppAₘpG , ^{Bn7}GppspAₘpG (D1), ^{Bn7}GppspAₘpG (D2), ^{4-ClBn7}GppspAₘpG (D1), ^{4-ClBn7}GppₛpAₘpG, ^{Bn7}IpppAₘpG, and ^{Bn7}LGpppAₘpG), which were synthesized through organic chemical reactions (FIG. 12).

### <1-1> Reagent Information

¹H, ¹³C, and ³¹P NMR spectra were recorded using a Bruker AV-400 spectrometer, with D₂O or DMSO-d₆ used as the solvent and tetramethylsilane used as the internal standard. UV-Vis spectra were recorded at room temperature using a Cary series UV-Vis spectrophotometer (Agilent Technologies) and quartz cuvettes with a path length of 1 cm, and absorbance changes were measured immediately after irradiating the sample solution with ultraviolet light in the cuvette. Fluorescence emission spectra were recorded at room temperature using a PF-65000 fluorescence spectrophotometer.

### <1-2> Method for Synthesizing N7-(arylmethyl) Guanosine 5'-diphosphoroimidazolide Analogs

### ① Synthesis of the Triethylammonium Salt of Guanosine 5'-diphosphate

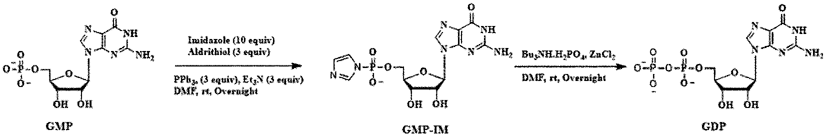

To synthesize the triethylammonium salt of guanosine 5'-diphosphate (GDP·TEA), a solution of triethylamine salt of guanosine 5'-phosphate (GMP·TEA) (930 mg, 2 mmol) in anhydrous dimethyl sulfoxide (4 mL) was stirred, and triethylamine (1.7 mL, 12 mmol), imidazole (817 mg, 12 mmol), and 2,2'-dithiopyridine (1.3 g, 6 mmol) were added. The mixture was stirred for 5 minutes, after which triphenylphosphine (1.56 g, 6 mmol) in anhydrous dimethyl sulfoxide (4 mL) was added, and stirring was continued for 5 hours at room temperature. After completion of the reaction, the mixture was slowly poured into a mixture of acetone (50 mL) and sodium perchlorate (0.5 g). After cooling at 4°C for 30 minutes, the mixture was centrifuged at 5,000 rpm for 8 minutes and the supernatant was discarded. To remove trace amounts of imidazole and triphenylphosphine, the solid was triturated with fresh acetone (20 mL), the mixture was cooled, and centrifuged again. This process was repeated once more, and the precipitate was dried at room temperature in a vacuum oven.

The resulting guanosine 5'-phosphoroimidazolide (NaGMP-IM) was dissolved in dimethylformamide (10 mL) and added dropwise into a mixture prepared by stirring a 1 M solution of tributylammonium orthophosphate in dimethylformamide (5 mL) for 30 minutes. Finally, zinc chloride (200 mg, 1.5 mmol) was added, and the reaction mixture was stirred overnight at room temperature. The reaction mixture was extracted with chloroform (3 × 20 mL) saturated with water (50 mL). The resulting solution was concentrated using a rotary evaporator (bath temperature 37°C). The product was subjected to purification on an anion-exchange resin.

Chromatographic purification was carried out using DEAE (diethylaminoethyl weak anion-exchange) Sepharose fast-flow resin. The desired compound was eluted on a CombiFlash EZ Prep system at a flow rate of 3 mL/min using four bed volumes with a 0-30% gradient of 1 M TEAB (triethylammonium bicarbonate) buffer, pH 7.5. The fractions containing GDP·TEA were combined, concentrated using a rotary evaporator (bath temperature 37°C), and dried in a freeze dryer to obtain GDP·TEA as a fine white powder (951 mg, 64%). The NMR results of GDP-TEA are shown below.

### GDP·TEA (951 mg, 64%)

¹H NMR (400 MHz, D₂O) δ = 7.99 (s, 1H), 5.81 (d, J = 6.0 Hz, 1H), 4.63 (t, J = 5.2 Hz, 1H), 4.51 (t, J = 5.6 Hz, 1H), 4.22 (m, 1H), 4.14 (m, 1H), 4.05 (m, 1H); ³¹P NMR (162 MHz, D₂O) δ = -6.47 (d, J = 23.5 Hz, 1P), -10.99 (d, J = 22.5 Hz, 1P).

### ② Synthesis of the Triethylammonium Salt of Guanosine 5'-diphosphate

According to the method of Example <1-2> above, inosine monophosphate (IMP) and L-guanosine monophosphate (LGMP) were respectively used to synthesize the triethylammonium salt of inosine 5'-diphosphate (IDP·TEA, 830 mg, 57%) and the triethylammonium salt of L-guanosine 5'-diphosphate (LGDP·TEA, 892 mg, 60%), and their NMR results are shown below.

### IDP.TEA. (830 mg, 57 %)

¹H NMR (400 MHz, D₂O) δ = 8.41 (s, 1H), 8.13 (s, 1H), 6.05 (d, J = 5.2 Hz, 1H), 4.68 (overlapped with H2O, m, 1H), 4.54 (t, J = 4.0 Hz, 1H), 4.29 (bs, 1H), 4.21-4.07 (m, 2H); ³¹P NMR (162 MHz, D₂O) δ = -7.47 (d, J = 22.5 Hz, 1P), -11.07 (d, J = 22.3 Hz, 1P).

### LGDP.TEA. (892 mg, 60 %)

¹H NMR (400 MHz, D₂O) δ = 8.08 (s, 1H), 5.89 (d, J = 5.6 Hz, 1H), 4.74 (t, J = 5.2 Hz, 1H), 4.59 (t, J = 4.0 Hz, 1H), 4.29 (m, 1H), 4.20 (m, 1H), 4.13 (m, 1H); ³¹P NMR (162 MHz, D₂O) δ = -6.35 (d, J = 22.5 Hz, 1P), -10.90 (d, J = 22.4 Hz, 1P).

### <1-3> Method for Synthesizing N7-(aryl-2-ylmethyl) Guanosine 5'-mono or Diphosphate Analogs

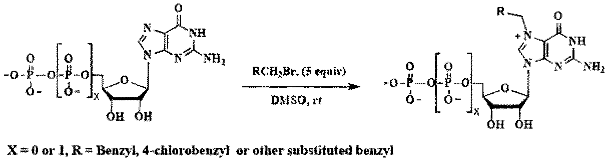

**A** triethylammonium salt of guanosine 5'-diphosphate (0.5 mmol, 1 equiv.) was dispersed in DMSO (3 mL), and an appropriate 2-bromomethyl aryl compound (2.5 mmol, 5 equiv.) was added. The mixture was stirred overnight at room temperature. After completion of the reaction, the mixture was slowly poured into a mixture of acetone (50 mL) and sodium perchlorate (0.5 g). After cooling at 4°C for 30 minutes, the mixture was centrifuged at 5,000 rpm for 8 minutes and the supernatant was discarded. The solid was triturated with fresh acetone (20 mL), the mixture was cooled, and centrifuged again. This process was repeated once more, and the precipitate was vacuum-dried at room temperature. The crude product solution, having a total volume of 15-20 mL, was separated using DEAE Sepharose fast-flow resin as described above. The fractions containing the product were combined, concentrated using a rotary evaporator (bath temperature 37°C), azeotropically evaporated three times with ethanol, and dried using a freeze dryer.

According to the above method, benzyl, 4-chlorobenzyl, and benzylinosine were respectively used to synthesize N7-benzyl guanosine 5'-monophosphate triethylammonium salt, N7-4-chlorobenzyl guanosine 5'-monophosphate triethylammonium salt, N7-benzyl guanosine 5'-diphosphate triethylammonium salt, N7-4-chlorobenzyl guanosine 5'-diphosphate triethylammonium salt, and N7-benzyl inosine 5'-diphosphate triethylammonium salt, and their NMR results are shown below.

### N7-benzylguanosine 5'-monophosphate triethylammonium salt: N7-Bn-GMP.TEA (270 mg, 82 %).

¹H NMR (400 MHz, D₂O) δ = 7.39 (m, 5H), 6.05 (d, J = 4 Hz, 1H), 5.64 (s, 2H), 4.68 (d, J = 4.7 Hz, 1H), 4.46 (t, J = 4.9 Hz, 1H), 4.40 (q, J = 2.3 Hz, 1H), 4.18-4.13 (m, 1H), 4.06-4.01 (m, 1H); ³¹P NMR (162 MHz, D₂O): δ = 2.30 (s, 1P). ESI MS: Calculated m/z for C₁₇H₁₉N₅O₈P ([M]): m/z 452.34; found ([M-1]): 451.2.

### N7-4-cholobenzyl guanosine 5'-monoahosphate triethylammonium salt: N7-ClBn-GMP.TEA (260 mg, 75 %).

¹H NMR (400 MHz, D₂O) δ = 7.28 (q, J =8.1 Hz, 4H), 6.03 (d, J = 3.4 Hz, 1H), 5.58 (s, 1H), 4.63 (t, J = 4.1 Hz, 1H), 4.47 (t, J = 5.0 Hz, 1H), 4.35 (s, 1H), 4.12 (d, J = 12.2 Hz, 1H), 4.01-3.97 (m, 1H); ³¹P NMR (162 MHz, D₂O): δ = 3.70 (s, 1P).

### N7-benzyl guanosine 5'-diphosphate triethylammonium salt: N7-Bn-GDP.TEA (247 mg, 67 %).

¹H NMR (400 MHz, D₂O) δ = 7.23-7.38 (m, 5H), 5.99 (d, J = 3.6 Hz, 1H), 5.64 (d.d, J1 = 21.6 Hz, J2 = 15.2 Hz, 2H), 4.61 (dd, J1 = 5.2 Hz, J2 = 3.2 Hz, 1H), 4.53 (t, J = 5.2 Hz, 1H), 4.30 (m, 1H), 4.18-4.24 (m, 2H); ³¹P NMR (162 MHz, D₂O) δ = -6.48 (d, J = 23.5 Hz, 1P), -10.98 (d, J = 23.5 Hz, 1P).

### N7-4-chlorobenzyl guanosine 5'-diphosphate triethylammonium salt: N7-4-Cl-Bn-GDP.TEA (272 mg, 71 %).

¹H NMR (400 MHz, D₂O) δ = 7.38 (s, 4H), 6.02 (d, J = 4 Hz, 1H), 5.62 (s, 2H), 4.68 (t, J = 8 Hz, 1H), 4.52 (t, J = 8 Hz, 1H), 4.38-4.34 (m, 1H), 4.31-4.25 (m, 1H), 4.24-4.18 (m, 1H); ³¹P NMR (162 MHz, D₂O) δ = -8.88 (d, J = 21 Hz, 1P), -11.08 (d, J = 23 Hz, 1P).

### N7 -benzyl inosine 5'-diphosphate triethylammonium salt: N7-Bn-IDP.TEA

¹H NMR (400 MHz, D₂O) δ = 8.18 (s, 1H), 7.40-7.27 (m, 5H), 6.14 (d, J = 3.2 Hz, 1H), 5.75 (q, J = 15.2 Hz, 2H), 4.61 (t, J = 4.8 Hz, 1H), 4.52 (t, J = 6.0 Hz, 1H), 4.32 (m, 1H), 4.24 (m, 2H); ³¹P NMR (162 MHz, D₂O) δ = -6.35 (d, J = 23.5 Hz, 1P), -11.00 (d, J = 23.5 Hz, 1P).

### <1-4> Method for Synthesizing N7-(aryl-2-ylmethyl) Guanosine 5'-mono or Diphosphoro-imidazolide Analogs

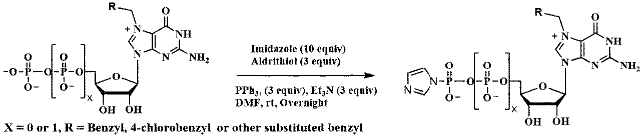

**A** solution prepared by stirring the triethylammonium salt of N7-(aryl-2-ylmethyl) guanosine 5'-diphosphate (0.3 mmol) in anhydrous dimethyl sulfoxide (3 mL) was added with triethylamine (251 µL, 1.8 mmol), imidazole (123 mg, 1.8 mmol), and 2,2'-dithiopyridine (198 mg, 0.9 mmol), and the mixture was stirred for 5 minutes. Then, triphenylphosphine (236 mg, 0.9 mmol) dissolved in anhydrous dimethyl sulfoxide (2 mL) was added, and stirring was continued for 5 hours at room temperature. After completion of the reaction, the mixture was slowly poured into a mixture of acetone (25 mL) and sodium perchlorate (250 mg). After cooling at 4°C for 30 minutes, the mixture was centrifuged and the supernatant was discarded. To remove trace amounts of imidazole and triphenylphosphine, the solid was triturated with fresh acetone (10 mL), the mixture was cooled, and centrifuged again. This process was repeated once more, and the precipitate was dried in a vacuum oven at room temperature using P₂O₅.

According to the above method, using N7-(benzyl-2-ylmethyl) guanosine 5'-diphosphate, N7-(4-chlorobenzyl-2-ylmethyl) guanosine 5'-diphosphate, and N7-(benzylinosine-2-ylmethyl) guanosine 5'-diphosphate, respectively, N7-benzyl guanosine 5'-monophosphoroimidazolide, N7-4-Cl-benzyl guanosine 5'-monophosphoroimidazolide, N7-benzyl guanosine 5'-diphosphoroimidazolide, N7-4-Cl-benzyl guanosine 5'-diphosphoroimidazolide, and N7-benzyl inosine 5'-diphosphoroimidazolide were synthesized, and their NMR results are shown below.

### N7-benzylguanosine 5'-monophosphoroimidazolide: N7-Bn-GMP-IM (378mg, 93%)

¹H NMR (400 MHz, D₂O) = 7.74 (s, 1H), 7.39 (br s, 5H), 7.05 (d, J =1.2 Hz, 1H), 6.86 (s, 1H), 5.93 (d, J = 3.8 Hz, 1H), 5.63 (dd, J1 = 14.8 Hz, J2 = 14.8 Hz, 2H), 4.63 (t, J = 4.1 Hz, 1H), 4.32 (t, J = 5.0 Hz, 1H), 4.28-4.26 (m, 1H), 4.16-4.12 (m, 1H), 4.07-4.01 (m,1H); ³¹P NMR (162 MHz, D₂O) = -8.02 (s, 1P).

Electrospray ionization (ESI) mass spectrometry: Calculated m/z for C₂₀H₂₂N₇O₇P-([M]): m/z 503.41; found([M-2]): 501.2.

### N7-4-Cl-benzyl guanosine 5'-monophosphoroimidazolide: N7-4-Cl-Bn-GMP-IM (392mg, 91%)

¹H NMR (400 MHz, D₂O) = 7.76 (s, 1H), 7.34 (s, 4H), 7.05 (s, 1H), 6.88 (s, 1H), 5.92 (d, J = 3.4 Hz, 1H), 5.58 (dd, J1 = 14.8 Hz, J2 = 15 Hz, 2H), 4.61 (t, J = 4.5Hz, 1H), 4.31-4.28 (m, 2H), 4.13-4.04 (m, 2H); ³¹P NMR (162 MHz, D₂O): = -8.04 (s, 1P).

Electrospray ionization (ESI) mass spectrometry: Calculated m/z for C₂₀H₂₁ClN₇O₇P-([M]): m/z 537.85; found([M-2]): 535.1.

### N7-benzylguanosine 5'-diphosphoroimidazolide: N7-Bn-GDP-IM (153mg, 81%)

¹H NMR (400 MHz, D₂O) δ = 7.80 (s, 1H), 7.24-7.41 (bs, 5H), 7.19 (s, 1H), 6.88 (s, 1H), 5.97 (d, J = 4.0 Hz, 1H), 5.59 (dd, J1 = 21.2 Hz, J2 = 14.4 Hz, 2H), 4.54 (t, J = 4.0 Hz, 1H), 4.25-4.33 (m, 2H), 3.99-4.20 (m, 2H); ³¹P NMR (162 MHz, D₂O) δ = -11.76 (d, J = 21.5 Hz, 1P), -19.88 (d, J = 21.4 Hz, 1P).

### N7-4-Cl-benzyl guanosine 5'-diphosphoroimidazolide: N7-4-Cl-Bn-GDP-IM

¹H NMR (400 MHz, D₂O) δ = 7.82 (s, 1H), 7.32 (bs, 4H), 7.20 (bs, 1H), 6.89 (bs, 1H), 5.97 (d, J = 4.0 Hz, 1H), 5.54 (dd, J1 = 23.6 Hz, J2 = 14.8 Hz, 2H), 4.55 (t, J = 4.0 Hz, 1H), 4.31 (d, J = 4.8 Hz, 2H), 4.20 (m, 1H), 4.06 (m, 1H); ³¹P NMR (162 MHz, D₂O) δ = -11.72 (d, J = 25.6 Hz, 1P), -19.80 (d, J = 20.6 Hz, 1P).

### <N7-benzyl inosine 5'-diphosphoroimidazolide: N7-Bn-IDP-IM>

¹H NMR (400 MHz, D₂O) δ = 8.17 (s, 1H), 7.77 (s, 1H), 7.37-7.26 (m, 5H), 7.17 (s, 1H), 6.86 (s, 1H), 6.10 (d, J = 3.2 Hz, 1H), 5.66 (q, J = 14.8 Hz, 2H), 4.35 (t, J = 4.0 Hz, 1H), 4.31-4.25 (m, 2H), 4.17 (m, 1H), 4.03 (m, 1H); ³¹P NMR (162 MHz, D₂O) δ = -11.76 (d, J = 29.3 Hz, 1P), -19.88 (d, J = 21.4 Hz, 1P).

### <1-5> Method for Synthesizing the Dinucleotide pAmpG

### ① Synthetic Process of 5'-O-DMT-2'-O-methyl Adenosylyl (n-bz)-{3'-OP-[2-cyanoethyl] → 5'}-2',3'-diacetyl Guanosine (n-ibu) 3

DMT-2'-O-methyl adenosine (n-bz) CED phosphoramidite 1 (1.29 g, 1.45 mmol) and 2',3'-diacetyl guanosine (n-ibu) 2 (0.58 g, 1.32 mmol) were dried under high vacuum for 16 hours. To acetonitrile (7.3 mL, 3.30 mmol) were added anhydrous DMF (1.7 mL) and 0.45 M tetrazole, and the mixture was stirred at room temperature for 3 hours under an argon (nitrogen) atmosphere. After confirming consumption of compound 1 by TLC, the mixture was cooled in an ice-salt bath for 30 minutes, and 70% t-butyl hydroperoxide (0.91 mL, 6.60 mmol) in water was added. The reaction mixture was stirred at room temperature for 1 hour. After confirming conversion of the intermediate by TLC, the mixture was extracted with ethyl acetate (2 × 200 mL) and washed with 5% NaHCO₃ solution (2 × 100 mL). The extracted organic layers were combined, dried over sodium sulfate, evaporated, and crude solid 3 (1.77 g) was obtained.

### ② Synthetic Process of 2'-O-Methyl Adenosylyl (n-bz)-{3'-OP-[2-cyanoethyl] → 5'}-2',3'-diacetyl Guanosine (n-ibu) 4

Crude dinucleotide 3 solid (1.77 g) was dissolved in 3% trichloroacetic acid in dichloromethane (31.2 mL, 5.72 mmol). The mixture was stirred at room temperature for 1 hour. After confirming conversion of compound 3 by TLC, 5% NaHCO₃ solution (30 mL) was added and the mixture was stirred for 10 minutes. The mixture was extracted with dichloromethane and evaporated. The resulting residue was purified by silica gel column chromatography to obtain product 4 (0.74 g, 0.79 mmol, 60%).

¹H NMR (DMSO, 400MHz): δ 12.12 (s, 1H), 11.59 (d, 1H), 11.26 (d, 1H), 7.93 (d, d, 2H), 8.28 (d, 1H), 8.06 (d, 2H), 7.66 (t, 1H), 7.57 (d, 2H), 6.19(d, 1H), 6.14 (dd, 1H), 5.84 (q, 1H), 5.54 (dd, 1H), 5. 39 (t, 1H), 5.20 (m, 1H), 4.83 (m, 1H), 4.48 (m, 3H), 4.30 (m, 3H), 3.64 (m, 2H), 3.37 (d, 3H), 2.97 (q, 2H), 2.78 (m, 1H), 2.15 (d, 3H), 2.03 (d, 3H), 1.13 (m, 6H); ³¹p NMR (DMSO, 162 MHz): δ -2.43 (d, 1P);

### ③ Synthetic Process of 5'-O-Phosphoryl-2'-O-methyl Adenosylyl-{3'-OP → 5'} Guanosine (pAmpG) 5

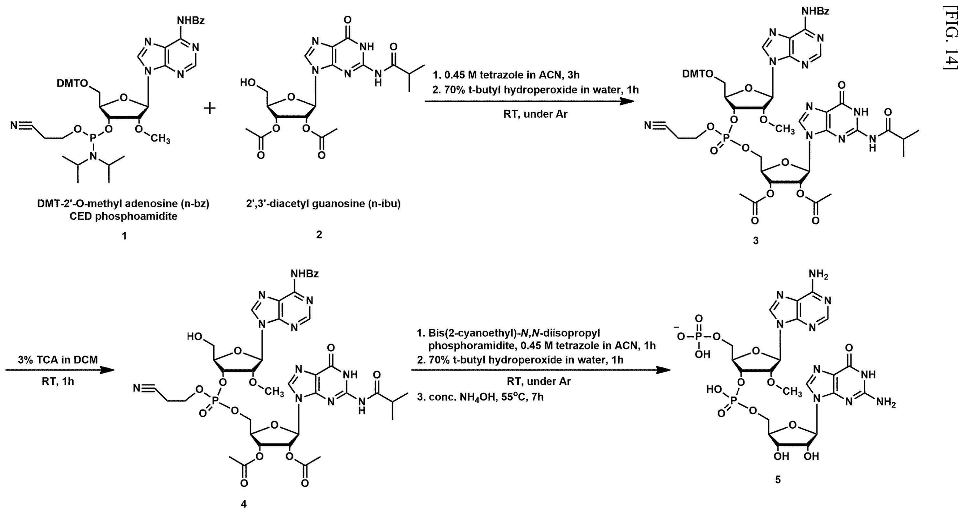

Dinucleotide 4 (0.74 g, 0.7891 mmol) was dried under high vacuum for 16 hours. To the mixture were added 0.45 M tetrazole in acetonitrile (3.51 mL, 1.58 mmol) and bis-(2-cyanoethyl)-N,N-diisopropyl-phosphoramidite (0.41 mL, 1.58 mmol), and the mixture was stirred for 3 hours at room temperature under an argon (nitrogen) atmosphere. After confirming consumption of compound 4, the reaction mixture was cooled in an ice-salt bath for 30 minutes, and 70% t-butyl hydroperoxide in water (17.2 mL, 3.16 mmol) was added. The mixture was stirred at room temperature for 1 hour. After confirming conversion of the intermediate by TLC, the mixture was extracted with ethyl acetate (2 × 200 mL) and washed with 5% NaHCO₃ solution (2 × 100 mL). The extracted organic layers were combined and evaporated. The resulting residue was dissolved in methanol (25 mL) and concentrated ammonia solution (25 mL), and stirred at 55°C for 7 hours. After completion of deprotection, the reaction mixture was evaporated with methanol. The resulting residue was dissolved in water, adjusted to pH 5.5, and loaded onto a DEAE-Sepharose column. Solvent A: 1 M TEAB buffer (pH 7.5-8), Solvent B: water. Fractions containing the product were collected, evaporated, and dried in a freeze dryer to obtain compound 5 as a fine white powder containing the triethylammonium salt (446 mg, 0.4908 mmol, 62%). (FIG. 14)

### Dinucleotide (pAmpG) TEA salt

¹H NMR (D₂O, 400 MHz): δ = 8.53 (s, 1H), 8.10 (s, 1H), 7.89 (s, 1H), 6.06 (d, 1H), 5.77 (d, 1H), 4.88 (m, 1H), 4.50 (t, 1H), 4.42 (m, 2H), 4.27 (m, 1H), 4.12 (t, 2H), 3.92 (m, 2H), 3.37 (s, 3H); ³¹P NMR (D₂O, 162 MHz): δ 3.69 (s, 1P), -0.84 (s, 1P)

### Dinucleotide (pAmpG) imidazole sodium salt: pAmpG-IM.Na (378 mg, 93%)

¹H NMR (400 MHz, D₂O) = 8.10 (d, J = 6.84 Hz, 2H), 7.89 (s, 1H), 7.75 (s, 1H), 7.07 (s, 1H), 6.86 (s, 1H), 5.96 (d, J = 5.4 Hz, 1H), 5.80 (d, J = 5.24 Hz, 1H), 4.85 - 4.81 (m, 1H), 4.45(t, J = 4.68 Hz, 1H), 4.40 (t, J = 5.0 Hz, 1H), 4.31 - 4.29 (m, 2H), 4.14 (s, 2H) 3.98-3.96 (m, 1H), 3.91-3.87 (m, 1H) 3.41 (s, 3H); ³¹P NMR (162 MHz, D₂O) = -0.81 (s, 1P), -8.15 (s, 1P).

### <1-6> General Synthetic Process of N7-(aryl-2-ylmethyl) Guanosine 5'-(2-thiodiphosphate)

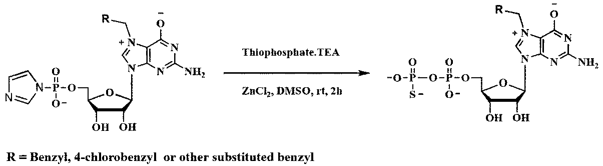

Into DMSO (50 mM) was added fully dried imidazolized N7-modified guanosine monophosphate (^{Bn7}GMP-IM·Na) or ^{4-ClBn7}GMP-IM·Na (0.67 mmol) and dissolved. Then, dried thiophosphate TEA salt [(Et₃NH), PSO₃³⁻] (1 M in DMF) (4.5 equivalents) was added, and the mixture was stirred at room temperature for 10 minutes. Zinc chloride (8 equivalents) and DMSO solution were then added, and stirring was continued for 2 hours. Afterward, the reaction mixture was slowly poured into a cooled solution of sodium perchlorate (400 mg) and acetone (45 mL), followed by centrifugation, and the supernatant was discarded. The precipitate was resuspended in acetone. The suspension-centrifugation process was repeated an additional 3-4 times. The precipitate was dried using P₂O₅ in a vacuum dryer, dissolved in water (15-20 mL), and EDTA·Na₄ (1.5 equivalents) was added, followed by extraction with chloroform (3 × 50 mL). The organic layer was discarded, and the aqueous layer was purified by DEAE Sepharose fast-flow resin as described above. Fractions containing the product were collected, concentrated using a rotary evaporator, and dried using a freeze dryer to obtain a fine powder.

### <^{Bn7}GDPβS.TEA (320mg, 56 %)>

¹H NMR (400 MHz, D₂O) = 7.40 - 7.36 (m, 5H), 6.04 (d, J = 3.7 Hz, 1H), 5.71 (s, 2H), 4.66 (dd, J1 = 2.72 Hz, J2 = 4.96 Hz, 2H), 4.36-4.34 (m, 1H), 4.28-4.27 (m, 2H); ³¹P NMR (162 MHz, D₂O) = 32.86 (d, J = 32.1 Hz, 1P), -11.88 (d, J = 33.0 Hz, 1P).

### <^{4-ClBn7}GDPβS. TEA (280mg, 47%)>

¹H NMR (400 MHz, D₂O) = 7.32 (q, J = 8.5 Hz, 4H), 6.05 (d, J = 3.4 Hz, 1H), 5.65 (dd, J1 = 14.9 Hz, J2 = 14.9 Hz, 2H), 4.74 (t, J = 4.4 Hz, 1H), 4.66 (d, J = 5.1 Hz, 1H), 4.38-4.33 (m, 2H), 4.29-4.24 (m, 1H); ³¹P NMR (162 MHz, D₂O) = 33.33 (d, J = 32.2 Hz, 1P), -11.81 (d, J = 31.9 Hz, 1P).

### <1-7> Method for Synthesizing N7-Arylmethyl Substituted CleanCap Analogs

A solution of dinucleotide pAmpG triethylammonium salt (0.07 mmol, 63 mg) dissolved in DMSO (50 mM) was added with N7-(arylmethyl) guanosine 5'-diphosphoroimidazolide disodium salt (16) (3.00 equiv., 0.2 mmol) and zinc chloride (20.0 equiv.). After stirring at 37°C for 3 days, 500 mM EDTA aqueous solution (pH 8.0) (1.30 equivalents of EDTA per mole of ZnCl₂) was added to the reaction mixture. The mixture was diluted with water and purified through DEAE Sepharose fast-flow resin, followed by additional purification using reversed-phase HPLC (instrument: Shimadzu; column: YMC-Actus Triart C8 (Preparative, 250 × 20.0 mm I.D.)). Fractions containing the target compound were collected, concentrated, and lyophilized to yield the target N7-arylmethyl substituted CleanCap analog as a triethylammonium salt. The product was redissolved in methanol (2.00 mL). To the mixture, 190 mM NaClO₄ in acetone (24.0 mL) was added, and the resulting suspension was centrifuged (4,000 rpm, 20 min). The supernatant was discarded, and the precipitate was resuspended in acetone. The suspension-centrifugation steps were repeated an additional 3-4 times. The precipitate was dried under reduced pressure to obtain the N7-arylmethyl substituted CleanCap analog.

The NMR analytical results of the N7-benzyl CleanCap analog, N7-Bn-GpppAₘpG (26 mg, 29%), are shown below.

¹H NMR (400 MHz, D₂O) δ = 8.19 (s, 1H), 7.93 (s, 1H), 7.77 (s, 1H), 7.01-7.23 (m, 5H), 5.75 (d, J = 5.6 Hz, 1H), 5.69 (d, J = 4.4 Hz, 1H), 5.66(d, J = 6.4 Hz, 1H), 5.44 (dd, J1 = 26.0 Hz, J2 = 14.4 Hz, 2H), 4.71-4.80 (m, 2H), 4.38 (t, J = 4.4 Hz, 1H), 4.34 (t, J = 6.0 Hz, 1H), 4.29 (t, J = 5.2 Hz, 2H), 4.24 (t, J = 4.4 Hz, 1H), 4.14-4.21 (m, 3H), 3.96-4.14 (m, 5H), 3.25 (s, 3H); ³¹P NMR (162 MHz, D₂O) δ = -0.86 (s, 1P), -11.23 (d, J = 19.4 Hz, 1P), -11.43 (d, J = 17.7 Hz, 1P), -22.70 (t, J = 17.7 Hz, 1P).

### <1-8> General Synthetic Process of Guanosine N7-(aryl-2-ylmethyl) Trinucleotide mRNA Cap1 Analogs

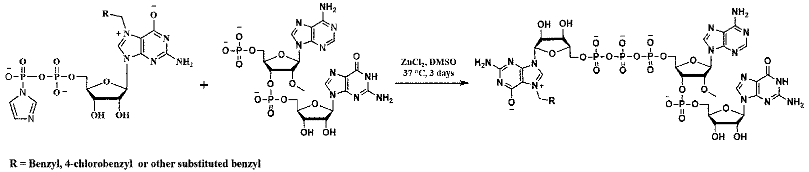

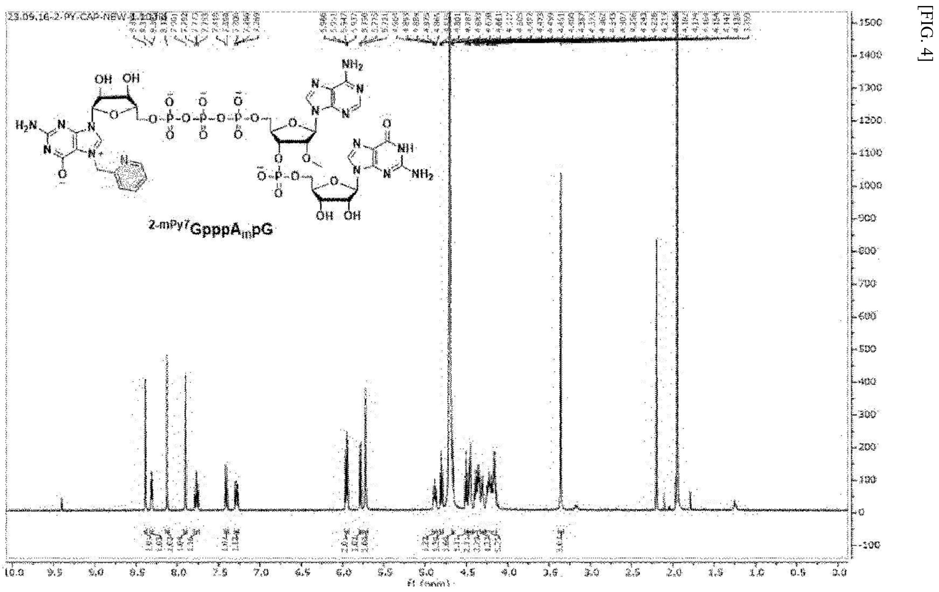

A solution containing pAₘpG triethylammonium salt (0.07 mmol, 63 mg) in 2 mL of DMSO was added with N7-(aryl-2-ylmethyl) guanosine 5'-diphosphoroimidazolide disodium salt (3.00 equivalents, 0.2 mmol) and zinc chloride (20.0 equivalents). After stirring at 37°C for 3 days, a 500 mM EDTA aqueous solution (pH 8.0) (1.30 equivalents of EDTA per mole of ZnCl₂) was added to terminate the reaction mixture. The mixture was diluted with water and purified using DEAE Sepharose fast-flow resin, followed by further purification using reversed-phase HPLC (Shimadzu instrument; YMC-Actus Triart C8 column (preparative, 250 × 20.0 mm I.D.); solvent A, 50 mM TEAA buffer (pH 6.0) containing 0.5% CH₃CN; solvent B, CH₃CN; linear gradient 5-80% B (25 minutes); flow rate, 10 mL/min; detection wavelength, 254 nm). Fractions containing the target compound were collected, concentrated, and lyophilized to obtain the desired N7-(aryl-2-ylmethyl) Cap1 analog as a triethylammonium salt. The product was redissolved in methanol (2.00 mL). To the mixture, 190 mM NaClO₄ in acetone (24.0 mL) was added, and the resulting suspension was centrifuged (4,000 rpm, 20 min). The supernatant was discarded and the precipitate was resuspended in acetone. The suspension-centrifugation procedure was repeated an additional 3-4 times. The precipitate was dried under reduced pressure to obtain the target N7-(aryl-2-ylmethyl) Cap1 analog as a sodium salt (FIG. 4).

According to the above method, using N7-(benzyl-2-ylmethyl) guanosine 5'-diphosphoroimidazolide and N7-(4-Cl-benzyl-2-ylmethyl) guanosine 5'-diphosphoroimidazolide, Guanosine N7-benzyl trinucleotide Cap1 (^{Bn7}GpppAₘpG), Guanosine N7-4-Cl-benzyl trinucleotide Cap1 (^{4-ClBn7}GpppAₘpG), L-Guanosine N7-benzyl trinucleotide Cap1 (^{Bn7}LGpppAₘpG), L-Guanosine N7, adenosine N6-dibenzyl trinucleotide Cap1 (^{Bn7}Gppp^{Bn6}AₘpG), and Inosine N7-benzyl trinucleotide Cap1 (^{Bn7}IpppAₘpG) were synthesized, and their NMR analytical results are shown below.

### <Guanosine N7-benzyl trinucleotide Cap1(^{Bn7}GpppAₘpG)>

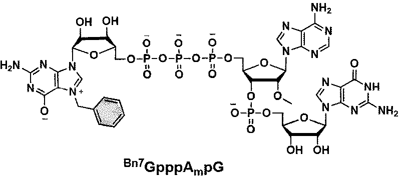

¹H NMR (400 MHz, D₂O) δ = 8.19 (s, 1H), 7.93 (s, 1H), 7.77 (s, 1H), 7.01-7.23 (m, 5H), 5.75 (d, J = 5.6 Hz, 1H), 5.69 (d, J = 4.4 Hz, 1H), 5.66 (d, J = 6.4 Hz, 1H), 5.44 (dd, J1 = 26.0 Hz, J2 = 14.4 Hz, 2H), 4.71-4.80 (m, 2H), 4.38 (t, J = 4.4 Hz, 1H), 4.34 (t, J = 6.0 Hz, 1H), 4.29 (t, J = 5.2 Hz, 2H), 4.24 (t, J = 4.4 Hz, 1H), 4.14-4.21 (m, 3H), 3.96-4.14 (m, 5H), 3.25 (s, 3H); ³¹P NMR (162 MHz, D₂O) δ = -0.86 (s, 1P), -11.23 (d, J = 19.4 Hz, 1P), -11.43 (d, J = 17.7 Hz, 1P), -22.70 (t, J = 17.7 Hz, 1P).

### <Guanosine N7-4-Cl-benzyl trinucleotide Cap1(^{4-ClBn7}GpppAₘpG)>

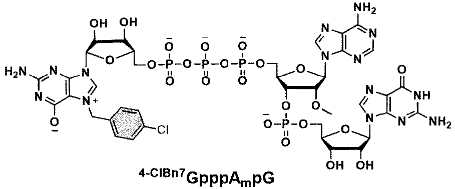

¹H NMR (400 MHz, D₂O) δ = 8.34 (s, 1H), 8.09 (s, 1H), 7.90 (s, 1H), 7.17 (d, J = 8.8 Hz, 2H), 7.05 (d, J = 8.4 Hz, 2H), 5.89 (d, J = 5.2 Hz, 2H), 5.76 (d, J = 5.6 Hz, 1H), 5.45 (dd, J1 = 20.4 Hz, J2 = 14.8 Hz, 2H), 4.89 (m, 1H), 4.75 (t, J = 5.6 Hz, 1H), 4.62 (t, J = 4.4 Hz, 1H), 4.47-4.43 (m, 3H), 4.41-4.32 (m, 3H), 4.32-4.22 (m, 3H), 4.22-4.13 (m, 3H), 3.36 (s, 3H); ³¹P NMR (162 MHz, D₂O) δ = -0.82 (s, 1P), -11.19 (d, J = 19.6 Hz, 1P), -11.47 (d, J = 18.6 Hz, 1P), -22.83 (t, J = 19.4 Hz, 1P).

### <L-Guanosine N7-benzyl trinucleotide Cap1(^{Bn7}LGpppAₘpG)>

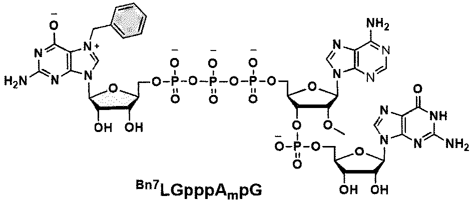

¹H NMR (400 MHz, D₂O) δ = 9.29 (s, 1H), 8.17 (s, 1H), 8.02 (s, 1H), 7.82 (s, 1H), 7.28 (m, 2H), 7.20 (m, 3H), 5.88 (d, J = 5.2 Hz, 1H), 5.82 (d, J = 3.6 Hz, 1H), 5.71 (d, J = 6.0 Hz, 1H), 5.46 (dd, J1 = 22.4 Hz, J2 = 14.8 Hz, 2H), 4.79 (m, 1H), 4.52 (t, J = 4.4 Hz, 1H), 4.41-4.34 (m, 3H), 4.31-4.19 (m, 5H), 4.18-4.03 (m, 5H), 3.24 (s, 3H); ³¹P NMR (162 MHz, D₂O) δ = -0.93 (s, 1P), -11.33 (d, J = 18.6 Hz, 1P), -11.54 (d, J = 17.5 Hz, 1P), -22.80 (t, J = 18.6 Hz, 1P).

### <Guanosine N⁷, adenosine N⁶- dibenzyl trinucleotide Cap1(^{Bn7}Gppp^{Bn6}AₘpG)>

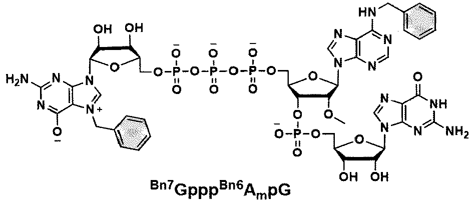

¹H NMR (400 MHz, D₂O) δ = 8.32 (s, 1H), 8.00 (s, 1H), 7.85 (s, 1H), 7.26-7.05 (m, 10H), 5.88 (d, J = 6.0 Hz, 1H), 5.77 (d, J = 4.8 Hz, 1H), 5.73 (d, J = 8.0 Hz, 1H), 5.42 (bs, 2H), 4.85 (m, 2H), 4.59 (m, 2H), 4.44-4.36 (m, 4H), 4.32-4.22 (m, 4H), 4.20-4.08 (m, 5H),3.30 (s, 3H); ³¹P NMR (162 MHz, D₂O) δ = -0.83 (s, 1P), -11.31 (d, J = 18.6 Hz, 1P), - 11.51 (d, J = 17.6 Hz, 1P), -23.00 (t, J = 17.5 Hz, 1P).

### <Inosine N7-benzyl trinucleotide Cap1(^{Bn7}IpppAₘpG)>

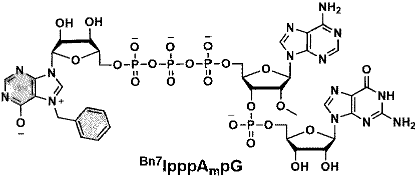

¹H NMR (400 MHz, D₂O) δ = 8.31 (s, 1H), 8.21 (s, 1H), 8.03 (s, 1H), 7.84 (s, 1H), 7.29-7.22 (m, 2H), 7.17-7.08 (m, 3H), 6.07 (d, J = 4.0 Hz, 1H), 5.85 (d, J = 6.0 Hz, 1H), 5.72 (d, J = 6.0 Hz, 1H), 5.62 (bs, 2H), 4.75 (overlapped with H2O, m, 1H), 4.63 (t, J = 4.4 Hz, 1H), 4.44 (t, J = 5.9 Hz, 1H), 4.41 (t, J = 5.0 Hz, 1H), 4.38-4.31 (m, 3H), 4.31-4.22 (m, 2H), 4.22-4.06 (m, 6H), 3.30 (s, 3H); ³¹P NMR (162 MHz, D₂O) δ = -0.87 (s, 1P), -11.27 (d, J = 19.2 Hz, 1P), -11.54 (d, J = 18.7 Hz, 1P), -22.94 (t, J = 19.8 Hz, 1P).

### <1-9> General Synthetic Process of N7-(aryl-2-ylmethyl) Trinucleotide Diphosphate Cap1 Materials (BzCap1)

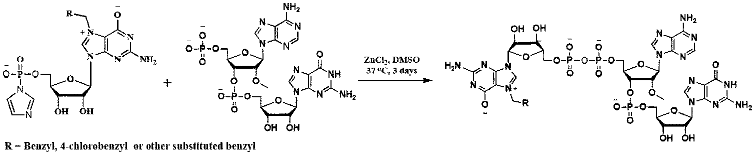

Into a dry DMSO solution under an inert atmosphere, a solution containing the dinucleotide (pAmpG) triethylammonium salt (0.073 mmol, 65 mg) was added with Bn7GMP-IM (0.219 mmol, 142 mg, 3 equivalents) or 4-Cl-Bn7GMP-IM (0.219 mmol, 124 mg, 3 equivalents). Zinc chloride (20.0 equivalents), dissolved in dry DMSO, was added, and the mixture was stirred at 37°C for 3 days. Thereafter, the reaction mixture was quenched by adding an EDTA aqueous solution (pH 7.5) (1.30 equivalents of EDTA per mole of ZnCl₂). The mixture was diluted with water and purified using reversed-phase HPLC (Prep Pure C18 AQ 100 Å, 250 × 20.0 mm I.D.); solvent A, 0.05 M ammonium acetate buffer (pH 5.5); solvent B, 0.05 M ammonium acetate buffer containing 33% CH₃CN (linear gradient 0-80% B for 70 minutes; flow rate 10 mL/min; detection wavelength 254 nm). Fractions containing the target compound were collected, stored in a freeze dryer, and the product was confirmed by NMR analysis.

### <Guanosine N7-benzyl trinucleotide diphosphate Cap1(^{Bn7}GppAₘpG)>

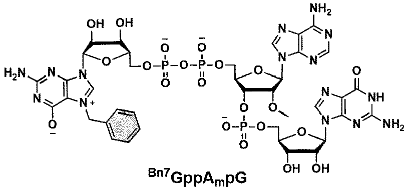

¹H NMR (400 MHz, D₂O) = 9.22 (s, 1H), 8.24 (s, 1H), 8.10 (d, J = 1.1 Hz, 1H), 7.92 (s, 1H), 7.26 (s, 5H), 5.88 (d, J = 5.7 Hz, 1H), 5.82 (d, J = 4.4 Hz, 1H), 5.78 (d, J = 14.8 Hz, 1H), 5.46 (dd, J1 = 12.0 Hz, J2 = 12.0 Hz, 2H), 4.87-4.83 (m, 1H), 4.56 (t, J = 4.8 Hz, 1H), 4.45-4.39 (m, 4H), 4.33-4.30 (m, 5H), 4.16 (br s, 4H), 3.36 (s, 3H); ³¹P NMR (162 MHz, D₂O) = -0.79 (s, 1P), -11.09 (d, J = 21.2 Hz, 1P), -11.43 (d, J = 21.33 Hz, 1P).

### <Guanosine N7-4-Cl-benzyl trinucleotide diphosphate Cap1(^{4-ClBn7}GppAₘpG)>

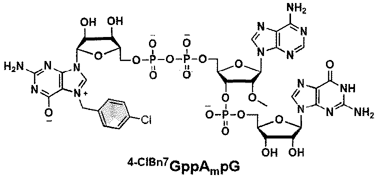

¹H NMR (400 MHz, D₂O) = 9.29 (s, 1H), 8.22 (s, 1H), 8.06 (s, 1H), 7.86 (s, 1H), 7.14 (d, J = 8.3 Hz, 2H), 7.08 (d, J = 8.2 Hz, 2H), 5.83-5.82 (m, 2H), 5.72 (d, J = 5.6 Hz, 1H), 5.37 (dd, J1 = 12.0 Hz, J2 = 16.0 Hz, 2H), 4.53-4.52 (m, 1H), 4.38 (s, 4H), 4.30-4.26 (m, 5H), 4.12 (br s, 5H), 3.32 (s, 3H); ³¹P NMR (162 MHz, D₂O) = -0.83 (s, 1P), -11.07 (d, J = 21.1 Hz, 1P), -11.4341 (d, J = 21.0 Hz, 1P).

### <1-10> Synthesis of N7-(aryl-2-ylmethyl) p-phosphorothioate Trinucleotide Cap1 Analogs

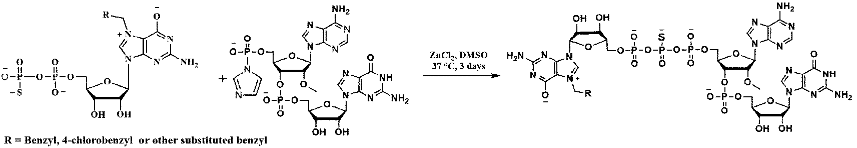

Into a dry DMSO solution under an inert atmosphere, a solution containing the dinucleotide (pAmpG-IM) sodium salt (0.075 mmol, 60 mg, 1 equivalent) was added with Bn7GDPβS·TEA (0.225 mmol, 192.14 mg, 3 equivalents) or 4-Cl-Bn7GDPβS·TEA (0.225 mmol, 200 mg, 3 equivalents). Zinc chloride (20.0 equivalents), dissolved in dry DMSO, was added, and the mixture was stirred at 37°C for 3 days. Thereafter, the reaction mixture was quenched by adding an EDTA aqueous solution (pH 7.5) (1.30 equivalents of EDTA per mole of ZnCl₂). The mixture was diluted with water and purified using reversed-phase HPLC (Prep Pure C18 AQ 100 Å, 250 × 20.0 mm I.D.); solvent A, 0.05 M ammonium acetate buffer (pH 5.5); solvent B, 0.05 M ammonium acetate buffer containing 33% CH₃CN (linear gradient 0-80% B for 110 minutes; flow rate 3 mL/min; detection wavelength 254 nm). The reaction provided two partly separated stereoisomers of the target compound (D1 and D2), and they were separated according to retention time during HPLC purification. The product with the longer retention time was designated D2, and the product with the shorter retention time was designated D1. Fractions containing the target compounds D1 and D2 were collected, stored in a freeze dryer, and the products were confirmed by NMR analysis.

### <Guanosine N7-benzyl β-phosphorothioate trinucleotide Cap1(^{Bn7}GppₛpAₘpG)(D1)>

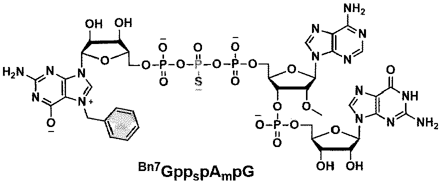

¹H NMR (400 MHz, D₂O) = 9.35 (s, 1H), 8.40 (s, 1H), 8.04 (s, 1H), 7.84 (s, 1H), 7.20-7.11 (m, 5H), 5,86 (t, J = 4.0 Hz, 2H), 5.73 (d, J = 8.0 Hz, 1H), 5.47 (dd, J1 = 16.0 Hz, J2 = 12.0 Hz, 2H), 4.88 (br s, 1H), 4.76 (t, J = 8.0 Hz, 1H), 4.62 (t, J = 4.0 Hz, 5H), 4.46 (t, J = 4.0 Hz, 2H), 4.42-4.41 (m, 3H), 4.31 (br s, 2H), 4.25 (br s, 1H), 4.19 (br s, 3H), 4.11 (br s, 2H), 3.28 (s, 3H); ³¹P NMR (162 MHz, D₂O) = 29.68 (d, J = 25.9 Hz, 1P), -0.81 (s, 1P), -12.30 (t, J = 25.9 Hz, 2P).

### <^{Bn7}GppₛpAₘpG (D2)>

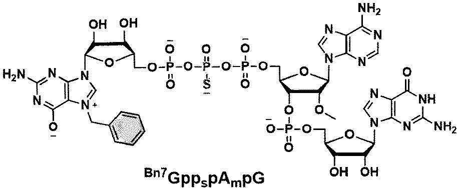

¹H NMR (400 MHz, D₂O) = 8.16 (s, 1H), 8.00 (s, 1H), 7.85 (s, 1H), 7.17 (br s, 5H), 5,81 (d, J = 8.0 Hz, 1H), 5.75 (d, J = 4Hz, 1H), 5.72 (d, J = 4.0 Hz, 1H), 5.37 (dd, J1 = 12.0 Hz, J2 = 16.0 Hz, 2H), 4.82-4.78 (m, 1H), 4.65 (t, J = 8.0 Hz, 1H), 4.49 (t, J = 4.0 Hz, 1H), 4.38 (t, J = 4.0 Hz, 1H), 4.35-4.33 (m, 2H), 4.27-4.26 (m, 4H), 4.11 (br s, 5H), 3.31 (s, 3H); ³¹P NMR (162 MHz, D₂O) = 29.60 (t, J = 24.3 Hz, 1P), -0.93 (s, 1P), -12.11 (d, J = 24.3 Hz, 1P), -12.46 (d, J = 25.9 Hz, 1P).

### <Guanosine N7-4-Cl-benzyl β-phosphorothioate trinucleotide Cap1 (^{4-ClBn7}GppₛpAₘpG)(D1)>

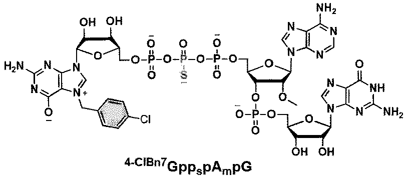

¹H NMR (400 MHz, D₂O) = 9.49 (s, 1H), 8.49 (s, 1H), 8.14 (s, 1H), 7.91 (s, 1H) 7.17 (d, J = 8.4 Hz, 1H), 7.05 (d, J = 8.5 Hz, 1H), 5.95 (d, J = 3.9 Hz, 1H), 5.91 (d, J = 6.0 Hz, 1H), 5.79 (d, J = 5.8 Hz, 1H), 5.51 (dd, J1 = 14.8 Hz, J2 = 14.7 Hz, 2H), 4.96-4.92 (m, 1H), 4.80 (t, J = 5.5 Hz, 1H), 4.53-4.45 (m, 4H), 4.39-4.38 (m, 2H), 4.31-4.18 (m, 7H), 3.34 (s, 3H); ³¹P NMR (162 MHz, D₂O) = 29.54 (t, J = 62.0 Hz, 1P), -0.75 (s, 1P), -12.22 (t, 2P, J = 22.9 Hz, 2P).

### <^{4-ClBn7}GppₛpAₘpG)(D2>

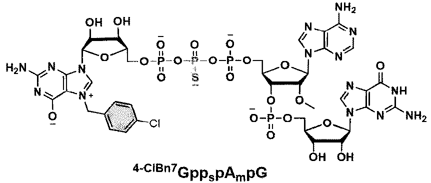

¹H NMR (400 MHz, D₂O) = 9.42 (s, 1H), 8.37 (s, 1H), 8.08 (s, 1H), 7.90 (s, 1H) 7.19 (d, J = 7.6 Hz, 2H), 7.08 (d, J = 7.6 Hz, 2H), 5.90-5.87 (m, 2H), 5.77 (br s, 1H), 5.59 (dd, J1 = 13.4 Hz, J2 = 15.0 Hz, 2H), 4.96 (br s, 1H), 4.48-4.41 (m, 5H), 4.36 (br s, 2H), 4.30 (br s, 1H), 4.24-4.18 (m, 6H), 3.36 (s, 3H); ³¹P NMR (162 MHz, D₂O) = 29.75 (t, J = 25.3 Hz, 1P), -0.86 (s, 1P), -11.92 (d, J = 25.3 Hz, 1P) and -12.29 (d, J = 24.6 Hz, 1P).

### <Example 2> Introduction of Trinucleotide Cappings into mRNA and Analysis of Capping Efficiency

It was analyzed whether the trinucleotide capping materials prepared in Example 1 can be recognized by T7 RNA polymerase with high efficiency during the transcription process and introduced into mRNA.

As a result, it was confirmed that mRNA into which a capping material modified at the N7 position of the guanosine at the 5' terminus with an arylmethyl group instead of the conventional methyl group had been introduced exhibited antigen protein expression levels in the translation process that were similar to or higher than those of the conventional CleanCap (cap 1 commercial) and the previously reported ARCA cap.

In addition, it was confirmed that the capping materials can be recognized by T7 RNA polymerase during the transcription process with higher efficiency than ARCA and can be introduced into RNA. Whereas the conventional ARCA exhibited a capping efficiency of approximately 50% and CleanCap (cap 1 commercial) exhibited a capping efficiency of approximately 85%, it was confirmed that BzCap1 and BzCap analogs exhibited relatively higher capping efficiencies of approximately 90% or more (see FIG. 45).

### <Example 3> Analysis of Antigen Protein Expression Using Capping Materials Substituted with an Arylmethyl Group

The translation process of mRNA into which a capping material prepared by modifying the N7 position of the guanosine base at the 5' terminus into an arylmethyl compound instead of the conventional methyl group had been introduced was confirmed. To examine the expression level of the antigen protein, Luciferase mRNA was used, and the expression level of the antigen protein was analyzed using HEK 293 cells. In the case of Luciferase mRNA, it was confirmed that the antigen protein expression efficiency of BzCap1 and BzCap analogs in mRNA using natural rNTPs was more efficient than that of CleanCap (see FIG. 46).

### ① Preparation of Luciferase DNA Template

A linear dsDNA template including the start codon up to the poly-A tail was prepared from a luciferase plasmid by PCR. During successive PCR steps, the T7 promoter and Kozak sequence were added to the luciferase dsDNA template, and this was ultimately used for the transcription reaction. The primers used were as follows: First forward primer: 5'-ATG GAA GAC GCC AAA AAC ATA AAG-3' (Sequence No. 1), Second forward primer: TAA TAC GAC TCA CTA TAG GGC CAC CAT GGA AGA CGC CAA AAA CAT (Sequence No. 2), Reverse primer: AAT CGC GCC TAG GCG CGC CCG TAC GGC TCT TC-3' (Sequence No. 3).

### ② Preparation of Luciferase mRNA

The concentration of the luciferase template was measured using a nanodrop spectrophotometer. For each transcription reaction, 100 ng of template in 1 µL was placed into a microcentrifuge tube. Then, 1.5 µL of 10X transcription buffer (400 mM Tris-HCl, 60 mM MgCl₂, 10 mM DTT, 20 mM spermidine) and 1.5 µL of 100 mM DTT were added to the tube. rATP, rCTP, and rUTP were each used (1 µL, 10 mM). For cap-dependent transcription of luciferase, rGTP was used (1 µL, 2.5 mM). The ratio of the capping material was maintained at four times higher than that of rGTP in order to provide mRNA capped at ≥80%. Accordingly, 1 µL of capping material (10 mM) was added. RNase inhibitor (0.5 µL, 40 U/µL) and T7 RNA polymerase (1 µL, 50 U/µL) were added. Nuclease-free water (4.5-5.5 µL) was added to bring the final reaction volume to 15 µL. In the case of modified rNTPs, 10 mM stock solutions were prepared, and the corresponding derivatives were added instead of rCTP and rUTP. The reaction was incubated at 37°C for 1 hour, and after 1 hour, 1.8 µL of 10X DNase I buffer (10 mM Tris-HCl, 2.5 mM MgCl₂, 0.5 mM CaCl₂) and 1 µL of DNase I (2 U/µL) were added to the reaction and incubated for an additional 15 minutes at 37°C to remove all DNA templates.

### ③ Purification of Transcribed mRNA

To remove dsRNA and transcription byproducts that may have been formed, cellulose was added to the reaction (10 µL, 0.2 g/mL) and gently shaken for 10 minutes. Next, the mixture was transferred to an RNA purification column together with ethanol and the binding buffer provided with the column, and finally centrifuged at 13,000 rpm for 1 minute. Then, RNA preparation buffer and wash buffer were sequentially added to the column and centrifuged. After this step, all proteins and unincorporated rNTP were removed into the suspension. Finally, a new tube was attached, and nuclease-free water (10 µL) was added to the column and centrifuged at 13,000 rpm for 2 minutes. All dsDNA bound to the cellulose remained in the column, and only the purified single-stranded GFP or luciferase mRNA passed through the column. This sample was collected and stored at 70°C for further application.

### ④ Analysis of Capping Efficiency of Transcribed mRNA

The capped and transcribed 2 µg of mRNA was annealed by heating together with 3 µg of gDNA and RNase H buffer to 70°C and then slowly cooling to 25°C. Next, the reaction was carried out at 37°C for 1 hour using thermally stable RNase H so as to cleave a short sequence containing the capping material from the long mRNA. The cleaved short sequence containing the capping material was analyzed by electrophoresis using a 16% denaturing PAGE gel. In uncapped mRNA, the cleaved short sequence exhibited faster gel migration, whereas in capped mRNA, the cleaved short sequence exhibited relatively slower gel migration.

### ⑤ Cell Culture and Preparation for Transfection

HEK 293 cells were cultured in a 60 mL culture plate containing DMEM supplemented with 10% FBS and 1% pen/strep. On the day prior to transfection, the cells were detached using trypsin and resuspended in fresh medium. The cells were then seeded in a 96-well plate. On the following day, when the cells reached 70% confluence, the transfection mixture was prepared and transfection was performed.

### ⑥ Quantification and Transfection of mRNA

The purified mRNA was quantified using a nanodrop spectrophotometer, and the transcription yield for each rNTP combination was compared. For cell transfection, 500 ng of each mRNA was collected and mixed with 5 µL of Opti-MEM. Separately, 0.5 µL of Lipofectamine was mixed with 5 µL of Opti-MEM in another tube and incubated at room temperature for 10 minutes. Then, the mRNA and Lipofectamine mixtures were combined in the same tube and incubated for an additional 5 minutes at room temperature. Finally, the transfection mixture was added to the cells and incubated for 24 hours.

### ⑦ Analysis of Luciferase Expression

After the incubation period, the medium was removed, and the cells were lysed using 50 µL of 1X lysis buffer. The cell lysate was transferred into microcentrifuge tubes. For luciferase luminescence analysis, 100 µL of luciferin substrate was taken for each sample in a 96-well white plate. Then, 20 µL of each cell lysate sample was added and mixed by pipetting. Finally, the luminescence of luciferase was recorded using a luminometer instrument.

As a result, it was confirmed that various types of aryl methyl-introduced capping materials according to the present invention can be recognized by T7 RNA polymerase during the transcription process with higher efficiency than ARCA or CleanCap, and can be introduced into RNA. Whereas the conventional ARCA exhibited approximately 50% capping efficiency and CleanCap exhibited 89% capping efficiency, it was confirmed that the various trinucleotide capping materials into which different arylmethyl groups had been introduced (^{Bn7}GpppAₘpG, ^{Bn7}GppAₘpG, ^{4-ClBn7}GppAₘpG, ^{Bn7}Gppp^{Bn6}AₘpG, ^{4-ClBn7}GpppAₘpG, ^{Bn7}GppₛpAₘpG (D1), ^{Bn7}GppₛpAₘpG (D2), ^{4-ClBn7}GppₛpAₘpG (D1), ^{4-ClBn7}GppₛpAₘpG, ^{Bn7}IpppAₘpG, and ^{Bn7}LGpppAₘpG) exhibited capping efficiencies of approximately 90% or more (see FIG. 45).

In addition, the translation process of mRNA into which a capping material prepared by modifying the N7 position of the guanosine base at the 5' terminus into an arylmethyl compound instead of the conventional methyl group had been introduced was confirmed.

To examine the expression level of the antigen protein, Luciferase mRNA was used, and the expression amount of the antigen protein was confirmed using HEK 293 cells. In the case of Luciferase mRNA, mRNA using natural rNTPs was used.

Furthermore, it was confirmed that Luciferase mRNA into which the trinucleotide capping materials developed in the present invention (^{Bn7}GpppAₘpG, ^{Bn7}GppAₘpG, ^{4-ClBn7}GppAₘpG, ^{Bn7}Gppp^{Bn6}AₘpG, ^{4-ClBn7}GpppAₘpG, ^{Bn7}GppₛpAₘpG (D1), ^{Bn7}GppₛpAₘpG (D2), ^{4-ClBn7}GppₛpAₘpG (D1), ^{4-ClBn7}GppₛpAₘpG, ^{Bn7}IpppAₘpG, and ^{Bn7}LGpppAₘpG) had been introduced exhibited higher antigen protein expression efficiency than mRNA into which the conventional ARCA cap (cap 0) or CleanCap (cap 1 commercial) had been introduced (see FIG. 46).

### Industrial Applicability

The present invention relates to novel modified trinucleotide capping derivatives and mRNA into which the capping derivatives are introduced, and the derivatives in which methylene-bridged heterocyclic compounds and arylmethyl groups are respectively introduced at the N7 position of the 5' terminal guanosine base of the present invention provide derivatives having superior efficiency compared to the capping materials used in conventional mRNA vaccines. Since the novel modified trinucleotide capping derivatives of the present invention and the mRNA into which the capping derivatives are introduced can be usefully employed in the development of new mRNA vaccines and in various fields of development of mRNA-based therapeutics, they can be variously utilized in industrial processes and the like.

Accordingly, the modified trinucleotide capping derivatives according to an embodiment of the present invention and the mRNA into which the capping derivatives are introduced can be regarded as having industrial applicability.

## Claims

1. A trinucleotide cap analog comprising a compound of Chemical Formula 1: 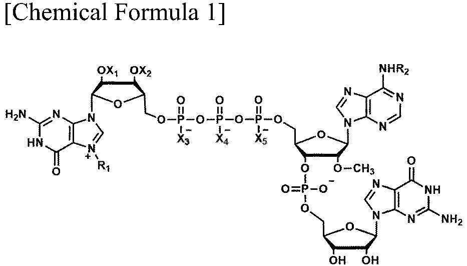
wherein R₁ is a methylene bridged heterocyclic compound linked via a methylene bridge to the N7 position of a guanosine base,
X₁ and X₂ are each H or CH₃,
X₃, X₄, and X₅ are each selected from the group consisting of O, S, and Se,
R₂ is a derivative comprising Benzyl, and
the heterocyclic compound is a substituent selected from heteroaromatic ring compounds having 1 to 2 heteroatoms selected from O, N, and S and being a saturated or partially saturated 5- to 10-membered heteroaromatic ring compound,
wherein the heteroaromatic ring compound is a substituent selected from the group consisting of furan, pyrole, thiophene, imidazole, pyrazole, oxazole, isoxazole, thiazole, pyridine, pyrazine, primidine, pyridazine, and triazine.

2. The trinucleotide cap analog according to Claim 1,
wherein the trinucleotide cap analog is the trinucleotide cap analog of Chemical Formula 2 or Chemical Formula 3:

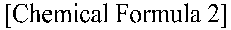

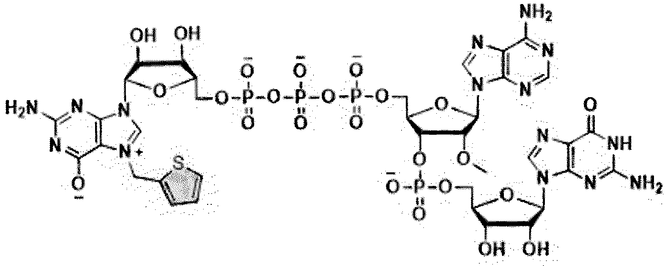

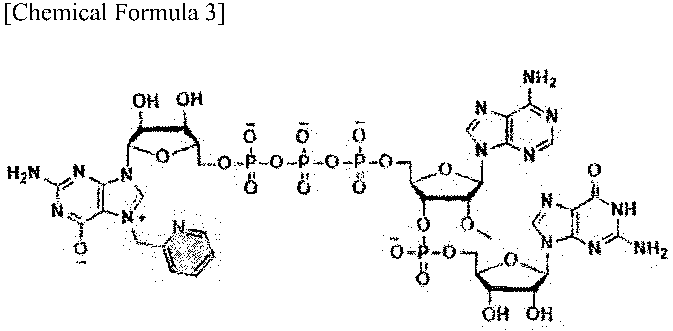

3. An mRNA capped at the 5' end with the trinucleotide cap analog according to Claim 1.

4. A composition for producing an mRNA capped at the 5' end, comprising the trinucleotide cap analog according to Claim 1.

5. A pharmaceutical composition for expression of a desired peptide or protein, comprising the mRNA capped at the 5' end with the trinucleotide cap analog according to Claim 1 and a pharmaceutically acceptable carrier.

6. A trinucleotide cap analog comprising a compound of Chemical Formula [4-1], [4-2], or [4-3]: 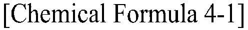 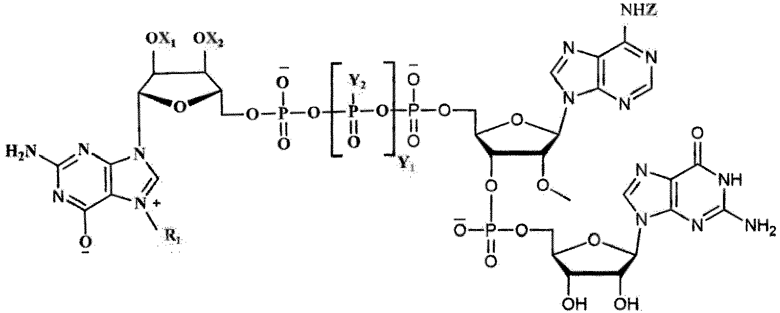 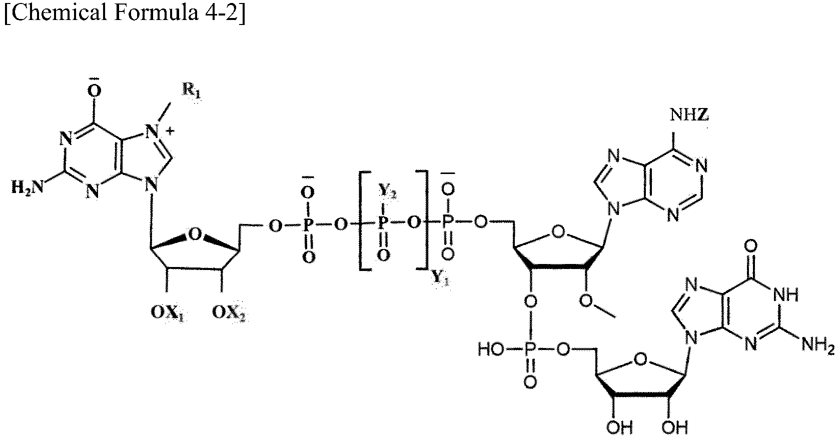 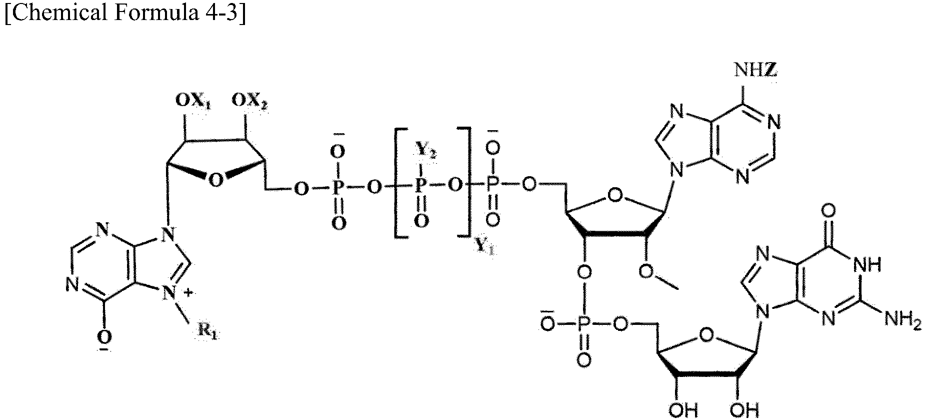 wherein
R₁ is a compound comprising a compound of Chemical Formula 5 linked via methyl (CH₂) to the N7 position,
X₁ and X₂ are each H or CH₃,
Y₁ is 0 or 1,
Y₂ is O or S, and
Z is H or C₁-C₃ alkylbenzyl; and 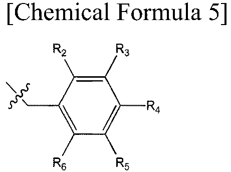
in Chemical Formula 5,
R₂, R₃, R₄, R₅, and R₆ are each independently a substituent selected from the group consisting of H, OH, Halo, methyl, alkyl, alkoxy, nitro, carboxylic, azide, amino, and cyano.

7. The trinucleotide cap analog according to Claim 6,
wherein R₁ is benzyl or chlorobenzyl.

8. The trinucleotide cap analog according to Claim 6,
wherein Z is methylbenzyl.

9. An mRNA capped at the 5' end with the trinucleotide cap analog according to Claim 6.

10. A composition for producing an mRNA capped at the 5' end, comprising the trinucleotide cap analog according to Claim 6.

11. A pharmaceutical composition for expression of a desired peptide or protein, comprising the mRNA capped at the 5' end with the trinucleotide cap analog according to Claim 6 and a pharmaceutically acceptable carrier.
